# EUROPEAN PATENT APPLICATION

(11) **EP 2 769 990 A2**
(43) Date of publication of application: **27.08.2014**
(21) Application number: 13163377.8
(22) Date of filing: 30.11.2005
(51) Int. Cl.: C07K 16/28, C07K 16/44, A61K 47/48, A61P 11/00, A61K 38/00, C07K 14/705, C07K 14/715, C12N 15/62, A61K 39/395

(54) **Bispecific domain antibodies targeting serum albumin and GLP-1 or PYY**

(30) Priority: 02.12.2004 US 632361 P; 31.05.2005 GB 0511019
(62) Divisional of application: 05810713.7
(71) Applicant: Domantis Limited, Brentford, Middlesex TW8 9GS (GB)
(72) Inventor: Holmes, Steve, Cambridge, Cambridgeshire CB4 0WG (GB); Holt, Lucy J, Cambridge, Cambridgeshire CB4 0WG (GB); Jespers, Laurent S, Cambridge, Cambridgeshire CB4 0WG (GB); Tomlinson, Ian M, Cambridge, Cambridgeshire CB4 0WG (GB)
(74) Representative: Wilson, Lynn Margaret

(57) **Abstract**

Drug fusions and conjugates that contain an incretin therapeutic or diagnostic agent that is fused or conjugated to an antigen-binding fragment of an antibody that binds serum albumin. The conjugates and fusion have a longer in vivo half life in comparison with the unconjugated or unfused therapeutic or diagnostic agent.

## Description

### RELATED APPLICATIONS

This application claims the benefit of U.S. Provisional Patent Application No. 60/632,361, filed on December 2, 2004 and the benefit of GB Patent Application No. 0511019.2. The entire teachings of the above applications are incorporated herein by reference.

### BACKGROUND OF THE INVENTION

Many drugs that possess activities that could be useful for therapeutic and/or diagnostic purposes have limited value because they are rapidly eliminated from the body when administered. For example, many polypeptides that have therapeutically useful activities are rapidly cleared from the circulation via the kidney. Accordingly, a large dose must be administered in order to achieve a desired therapeutic effect. A need exists for improved therapeutic and diagnostic agents that have improved pharmacokinetic properties. Polypeptides that bind serum albumin are known in the art. (See, *e.g.,* EP 0486525 B 1 (Cemu Bioteknik AB); US 6,267,964 B1 (Nygren et al.); WO 04/001064 A2 (Dyax, Corp.); WO 02/076489 A1(Dyax, Corp.); WO 01/45746 (Genentech, Inc.).)

One such class of drugs that have a short half life in the body or systemic circulation is the incretin hormones such as Glucagon-like peptide 1, or Peptide YY.

Glucagon-like peptide (GLP)-1 is an incretin hormone with potent glucose-dependent insulinotropic and glucagonostatic actions, trophic effects on the pancreatic *β* cells, and inhibitory effects on gastrointestinal secretion and motility, which combine to lower plasma glucose and reduce glycemic excursions. Furthermore, via its ability to enhance satiety, GLP-1 reduces food intake, thereby limiting weight gain, and may even cause weight loss. Taken together, these actions give GLP-1 a unique profile, considered highly desirable for an antidiabetic agent, particularly since the glucose dependency of its antihyperglycemic effects should minimize any risk of severe hypoglycemia. However, its pharmacokinetic/pharmacodynamic profile is such that native GLP-1 is not therapeutically useful. Thus, while GLP-1 is most effective when administered continuously, single subcutaneous injections have short-lasting effects. GLP-1 is highly susceptible to enzymatic degradation in vivo, and cleavage by dipeptidyl peptidase IV (DPP-IV) is probably the most relevant, since this occurs rapidly and generates a noninsulinotropic metabolite. Strategies for harnessing GLP-1's therapeutic potential, based on an understanding of factors influencing its metabolic stability and pharmacokinetic/pharmacodynamic profile, have therefore been the focus of intense research.

Extensive work has been done to attempt to inhibit the peptidase or to modify GLP-1 in such a way that its degradation is slowed down while still maintaining biological activity. WO05/027978 discloses GLP-1 derivatives having a protracted profile of action (and incorporated herein by reference as examples of GLP-1 derivatives and analogues that can be used in the present invention). WO 02/46227 discloses heterologous fusion proteins comprising a polypeptide (for example, albumin) fused to GLP-1 or analogues (the disclosure of these analogues is incorporated herein by reference as examples of GLP-1 analogues that can be used in the present invention). WO05/003296, WO03/060071, WO03/059934 disclose amino fusion protein wherein GLP-1 has fused with albumin to attempt to increase the half-life of the hormone.

However, despite these efforts a long lasting active GLP-1 has not been produced.

As such, particularly in the fields of diabetes and obesity, there is a tremendous need for improved GLP-1 peptides or other agents that similarly have an insulinotropic effect amenable to treatment for diabetes and obesity in particular. There is thus a need to modify GLP-1 and other insulinotropic peptides to provide longer duration of action *in vivo* while maintaining their low toxicity and therapeutic advantages.

### SUMMARY OF THE INVENTION

The invention relates to drug fusions and drug conjugates that have improved serum half lives. In one aspect, the drug fusion is a continuous polypeptide chain having the formula:
a-(X)ₙ₁-b-(Y)ₙ₂-c-(Z)ₙ₃-d or a-(Z)ₙ₃-b-(Y)ₙ₂-c-(X)ₙ₁-d,
wherein
X is a polypeptide drug that has binding specificity for a first target;
Y is an immunoglobulin heavy chain variable domain (V_{H}) that has binding specificity for serum albumin, or an immunoglobulin light chain variable domain (V_{L}) that has binding specificity for serum albumin;
Z is a polypeptide drug that has binding specificity for a second target;
a, b, c and d are each independently absent or one to about 100 amino acid residues;
n1 is one to about 10;
n2 is one to about 10; and
n3 is zero to about 10,
with the proviso that when n1 and n2 are both one and n3 is zero, X does not comprise an antibody chain or a fragment of an antibody chain.

In some embodiments, Y comprises an amino acid sequence selected from the group consisting of SEQ ID NO:10, SEQ ID NO:11, SEQ ID NO:12, SEQ ID NO:13, SEQ ID NO:14, SEQ ID NO:15, SEQ ID NO:24, SEQ ID NO:25 and SEQ ID NO:26, or an amino acid sequence selected from the group consisting of SEQ ID NO:16, SEQ ID NO:17, SEQ ID NO:18, SEQ ID NO:19, SEQ ID NO:20, SEQ ID NO:21, SEQ ID NO:22 and SEQ ID NO:23. In particular embodiments, X is GLP-1 or a GLP-1 analogue.

In another aspect, the drug fusion comprises a continuous polypeptide chain, said chain comprising moieties X' and Y', wherein

X' is a polypeptide drug, with the proviso that X' does not comprise an antibody chain or a fragment of an antibody chain; and

Y' is an immunoglobulin heavy chain variable domain (V_{H}) that has binding specificity for serum albumin, or an immunoglobulin light chain variable domain (V_{L}) that has binding specificity for serum albumin. In some embodiments, Y' comprises an amino acid sequence selected from the group consisting of SEQ ID NO:10, SEQ ID NO:11, SEQ ID NO:12, SEQ ID NO:13, SEQ ID NO:14, SEQ ID NO:15, SEQ ID NO:24, SEQ ID NO:25 and SEQ ID NO:26, or an amino acid sequence selected from the group consisting of SEQ ID NO:16, SEQ ID NO:17, SEQ ID NO:18, SEQ ID NO:19, SEQ ID NO:20, SEQ ID NO:21, SEQ ID NO:22 and SEQ ID NO:23. In particular embodiments, X' is GLP-1 or a GLP-1 analogue.

In another aspect, the invention is a drug conjugate comprising an immunoglobulin heavy chain variable domain (V_{H}) that has binding specificity for serum albumin, or an immunoglobulin light chain variable domain (V_{L}) that has binding specificity for serum albumin; and a drug that is covalently bonded to said V_{H} or V_{L}. In some embodiments, the immunoglobulin heavy chain variable domain comprises an amino acid sequence selected from the group consisting of SEQ ID NO:10, SEQ ID NO:11, SEQ ID NO:12, SEQ ID NO:13, SEQ ID NO:14, SEQ ID NO:15, SEQ ID NO:24, SEQ ID NO:25 and SEQ ID NO:26, or an amino acid sequence selected from the group consisting of SEQ ID NO:16, SEQ ID NO:17, SEQ ID NO:18, SEQ ID NO:19, SEQ ID NO:20, SEQ ID NO:21, SEQ ID NO:22 and SEQ ID NO:23. In particular embodiments, the drug is GLP-1 or a GLP-1 analogue.

The invention also provides recombinant nucleic acids and constructs that encode the drug fusions described herein, and host cells that comprise the recombinant nucleic acids and/or constructs. The invention further provides a method for producing a drug fusion comprising maintaining a host cell that comprises a recombinant nucleic acid and/or construct that encodes a drug fusion described herein under conditions suitable for expression of said recombinant nucleic acid, whereby a drug fusion is produced.

The invention also provides compositions (e.g., pharmaceutical compositions) comprising a drug fusion or drug conjugate of the invention. The invention also provides a method for treating an individual having a disease or disorder, such as those described herein, comprising administering to said individual a therapeutically effective amount of a drug conjugate or drug fusion of the invention. In some embodiments, the disease or disorder is an inflammatory disease, such as arthritis (*e.g.,* rheumatoid arthritis). In a further embodiment, the disease or disorder is a metabolic disease such as diabetes or obesity. The invention also provides for use of a drug conjugate or drug fusion of the invention for the manufacture of a medicament for treatment of a disease or disorder, such as an inflammatory disease (*e.g.,* arthritis (*e.g.,* rheumatoid arthritis)), or diabetes or obesity. The invention also relates to use of a drug fusion or drug conjugate as described herein for use in therapy, diagnosis or prophylaxis.

In another aspect, the invention is a noncovalent drug conjugate comprising an immunoglobulin heavy chain variable domain (VH) that has binding specificity for serum albumin, or an immunoglobulin light chain variable domain (VL) that has binding specificity for serum albumin, and a drug that is noncovalently bonded to said VH or VL. In some embodiments, the immunoglobulin heavy chain variable domain comprises an amino acid sequence selected from the group consisting of SEQ ID NO:10, SEQ ID NO:11, SEQ ID NO:12, SEQ ID NO:13, SEQ ID NO:14, SEQ ID NO:15, SEQ ID NO:24, SEQ ID NO:25 and SEQ ID NO:26, or an amino acid sequence selected from the group consisting of SEQ ID NO:16, SEQ ID NO:17, SEQ ID NO:18, SEQ ID NO:19, SEQ ID NO:20, SEQ ID NO:21, SEQ ID NO:22 and SEQ ID NO:23.

In a further embodiment, the invention provides an inactivated version of Dom7h-8, iDom7h-8, which does not bind to serum albumin which is used as a research tool and is predictive of the active serum albumin binding Dom7h-8.

### BRIEF DESCRIPTION OF THE DRAWINGS

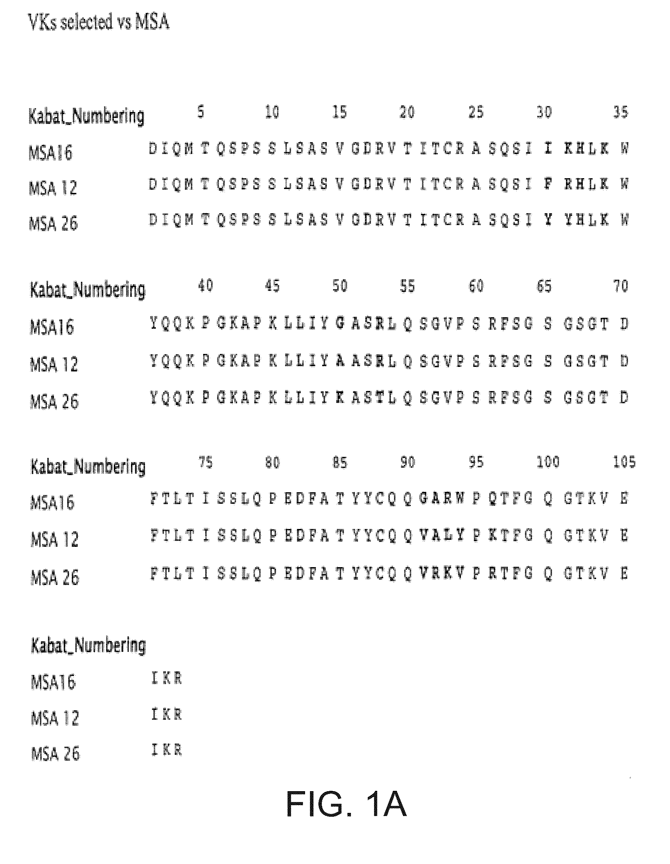

FIG. 1A is an alignment of the amino acid sequences of three V*κ*s selected by binding to mouse serum albumin (MSA). The aligned amino acid sequences are from V*κ*s designated MSA16, which is also referred to as DOM7m-16 (SEQ ID NO:1), MSA 12, which is also referred to as DOM7m-12 (SEQ ID NO:2), and MSA 26, which is also referred to as DOM7m-26 (SEQ ID NO:3).

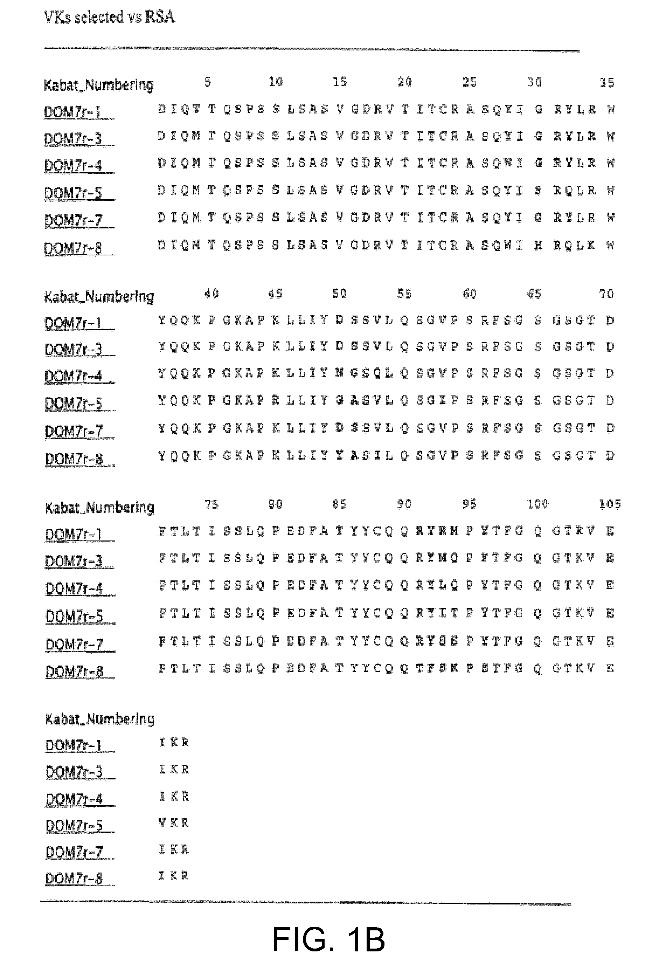

FIG. 1B is an alignment of the amino acid sequences of six V*κ*s selected by binding to rat serum albumin (RSA). The aligned amino acid sequences are from V*κ*s designated DOM7r-1 (SEQ ID NO:4), DOM7r-3 (SEQ ID NO:5), DOM7r-4 (SEQ ID NO:6), DOM7r-5 (SEQ ID NO:7), DOM7r-7 (SEQ ID NO:8), and DOM7r-8 (SEQ ID NO:9).

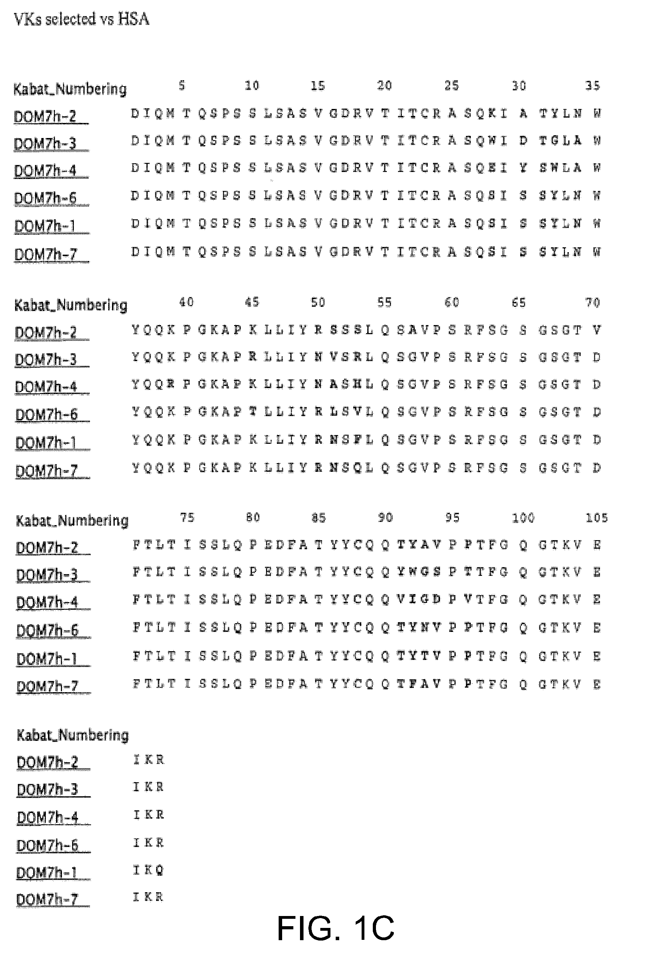

FIG. 1C is an alignment of the amino acid sequences of six V*κ*s selected by binding to human serum albumin (HSA). The aligned amino acid sequences are from V*κ*s designated DOM7h-2 (SEQ ID NO:10), DOM7h-3 (SEQ ID NO:11), DOM7h-4 (SEQ ID NO:12), DOM7h-6 (SEQ ID NO:13), DOM7h-1 (SEQ ID NO:14), DOM7h-7 (SEQ ID NO:15).

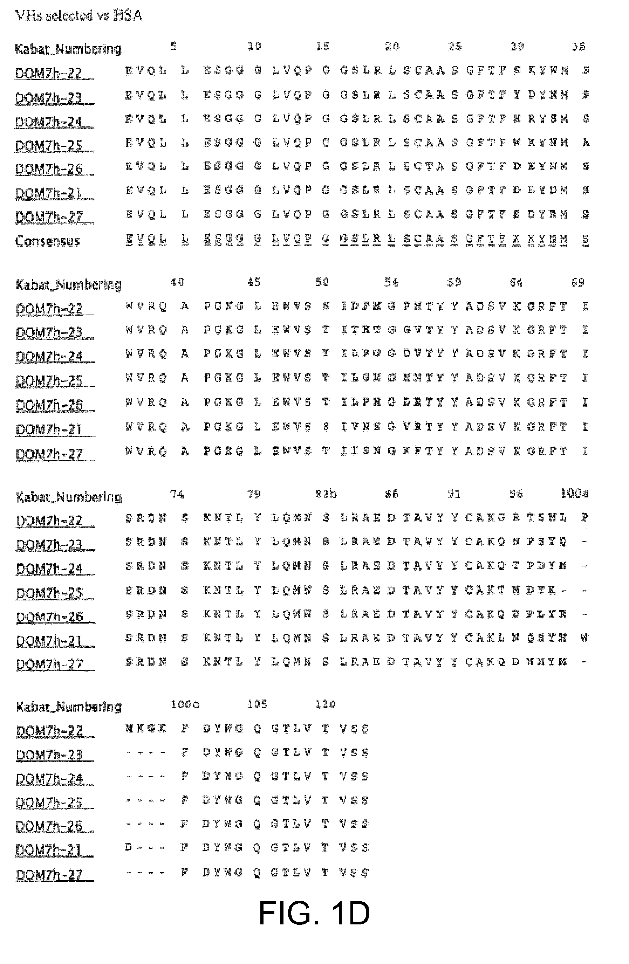

FIG. 1D is an alignment of the amino acid sequences of seven V_{H}s selected by binding to human serum albumin and a consensus sequence (SEQ ID NO:23). The aligned sequences are from V*κ*s designated DOM7h-22 (SEQ ID NO:16), DOM7h-23 (SEQ ID NO:17), DOM7h-24 (SEQ ID NO:18), DOM7h-25 (SEQ ID NO:19), DOM7h-26 (SEQ ID NO:20), DOM7h-21 (SEQ ID NO:21), and DOM7h-27 (SEQ ID NO:22).

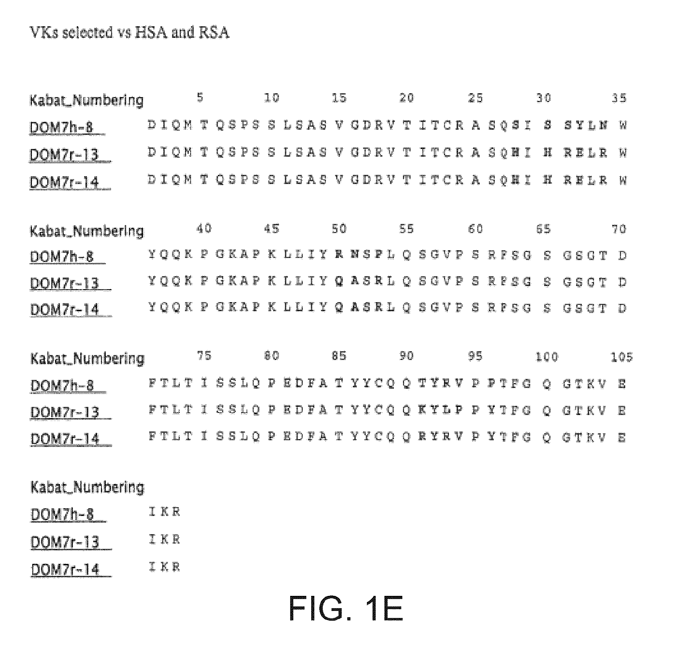

FIG. 1E is an alignment of the amino acid sequences of three V*κ*s selected by binding to human serum albumin and rat serum albumin. The aligned amino acid sequences are from V*κ*s designated DOM7h-8 (SEQ ID NO:24), DOM7r-13 (SEQ ID NO:25), and DOM7r-14 (SEQ ID NO:26).
FIG. 2A and 2B are schematics maps of the vectors used to express the MSA16IL-1ra (also referred to as DOM7m-16/IL-1ra) and IL-1raMSA16 (also referred to as IL-1ra/DOM7m-16) fusions, respectively.
FIG. 2C-2D is an illustration of the nucleotide sequence (SEQ ID NO:27) encoding the IL-1raMSA16 fusion (also referred to as IL-1ra/DOM7m-16)_and of the amino acid sequence (SEQ ID NO:28) of the fusion.
FIG. 2E-2F is an illustration of the nucleotide sequence (SEQ ID NO:29) encoding the MSA16IL-1ra fusion (also referred to as DOM7m-16/IL-1ra)_and of the amino acid sequence (SEQ ID NO:30) of the fusion.
FIG. 2G-2H is an illustration of the nucleotide sequence (SEQ ID NO:31) encoding the DummyIL-1ra fusion that did not bind serum albumin, and of the amino acid sequence (SEQ ID NO:32) of the fusion.

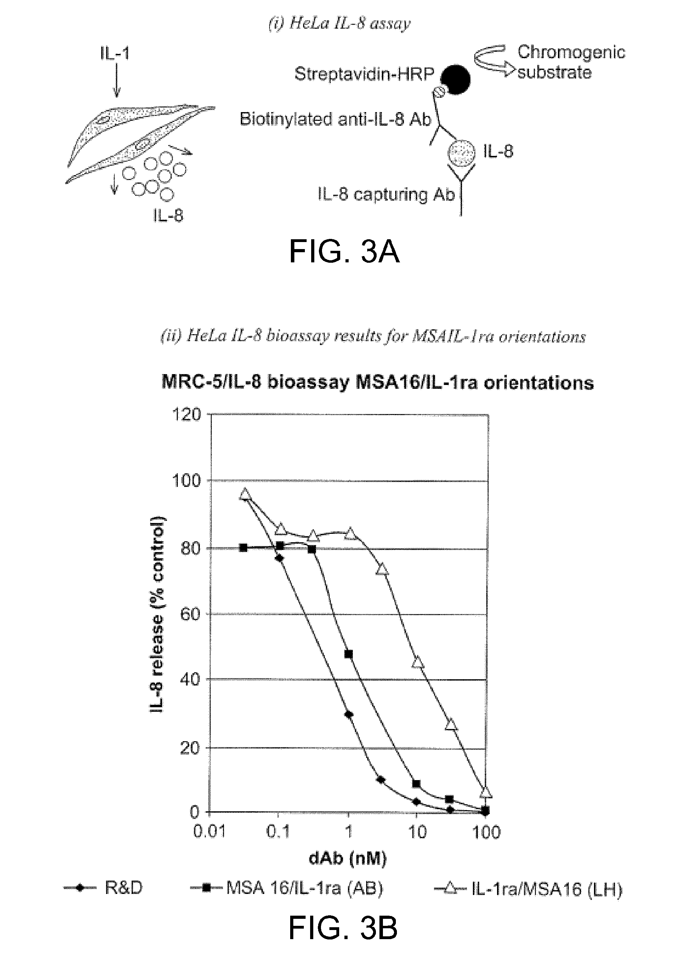

FIG. 3A is an illustration showing that IL-1 induces the production of IL-8 by HeLa cells, and showing the mechanism by which IL-8 is detected in an ELISA assay.
FIG. 3B is a graph showing that IL-1ra (◆), MSA16IL-1ra (■) and IL-1raMSA16 (▲) each inhibited IL-1-induced secretion of IL-8 by cultured MRC-5 cells. The observed inhibition was dose dependant for IL-1ra, MSA16IL-1ra and IL-1raMSA16.

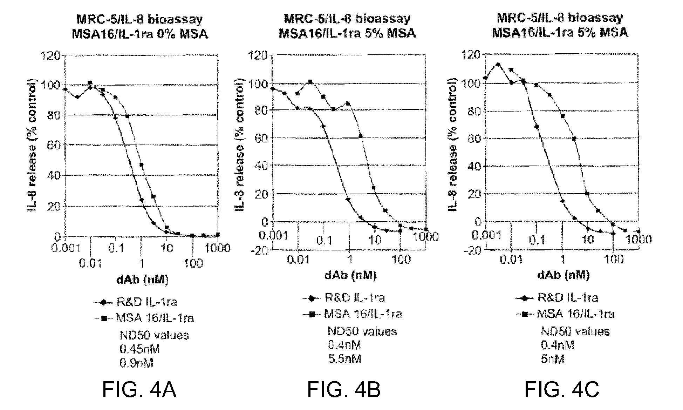

FIGS. 4A-4C are graphs showing that IL-1ra (◆) MSA16IL-1ra (■) both inhibited IL-1-induced secretion of IL-8 by cultured MRC-5 cells in assays that included no mouse serum albumin (4A), 5% mouse serum albumin (4B) or 10% mouse serum albumin (4C). The observed inhibition was dose dependant for IL-1ra and MSA16IL-1ra under all conditions tested.

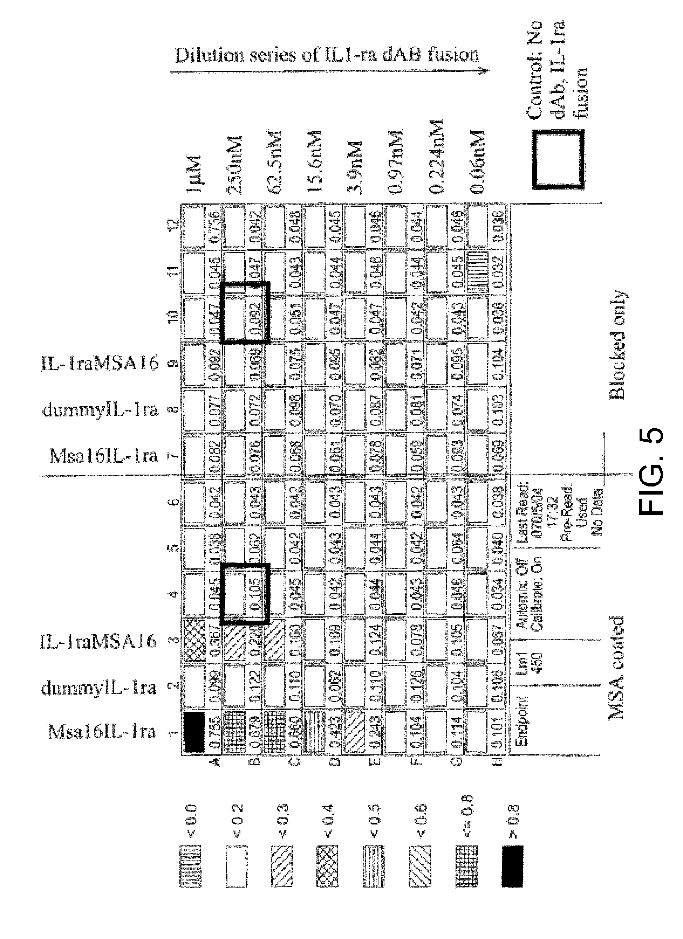

FIG. 5 is a schematic presentation of the results of an ELISA demonstrating that the MSA16IL1-ra fusion and the IL-1raMSA16 fusion both bound serum albumin, but the dummyIL1-ra fusion did not.
FIGS. 6A-6C are sensograms and tables showing BIACORE affinity data for clone DOM7h-1 binding to human serum albumin (HSA) (6A), DOM7h-7 binding to HSA (6B) and DOM7r-1 binding to rat serum albumin (RSA).
FIG. 7 is a table showing the affinities of DOM7h-1, DOM7r-1, DOM7h-2, DOM7r-3, DOM7h-7, DOM7h-8, DOM7r-8, DOM7r-13, DOM7r-14, DOM7m-16, DOM7h-22, DOM7h-23, DOM7h-26, DOM7r-16, DOM7m-26, DOM7r-27 and DOM7R-31 for the serum albumins that they bind.

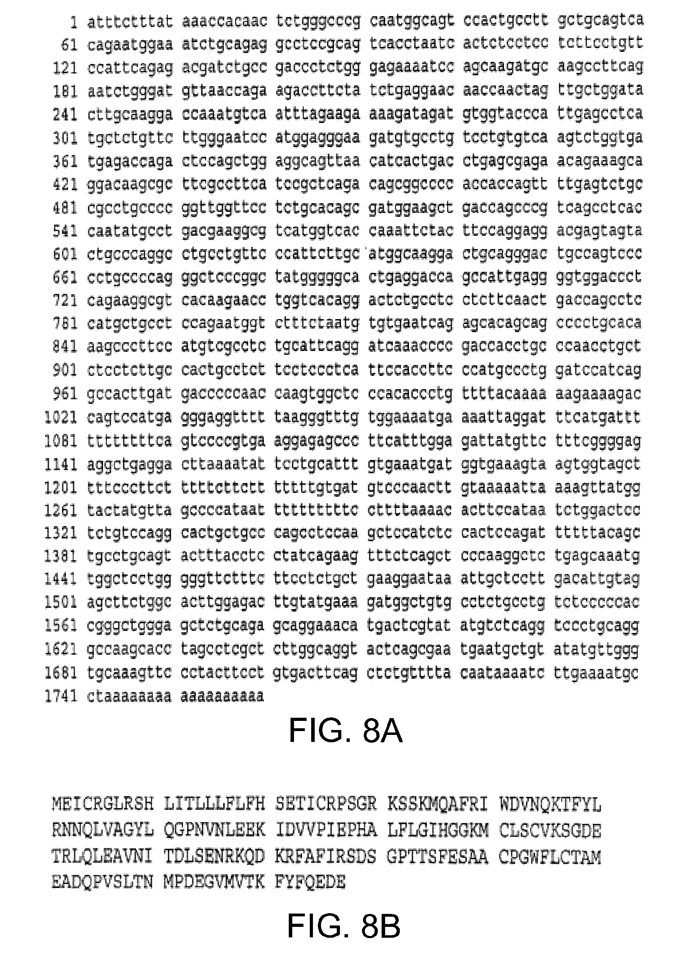

FIG. 8A is an illustration of the nucleotide sequence (SEQ ID NO:33) of a nucleic acid encoding human interleukin 1 receptor antagonist (IL-1ra) deposited in GenBank under accession number NM_173842. The nucleic acid has an open reading frame starting at position 65.
FIG. 8B is an illustration of the amino acid sequence of human IL-1ra (SEQ ID NO:34) encoded by the nucleic acid shown in FIG. 8A (SEQ ID NO:33). The mature protein consists of 152 amino acid residues (amino acid residues 26-177 of SEQ ID NO:34).

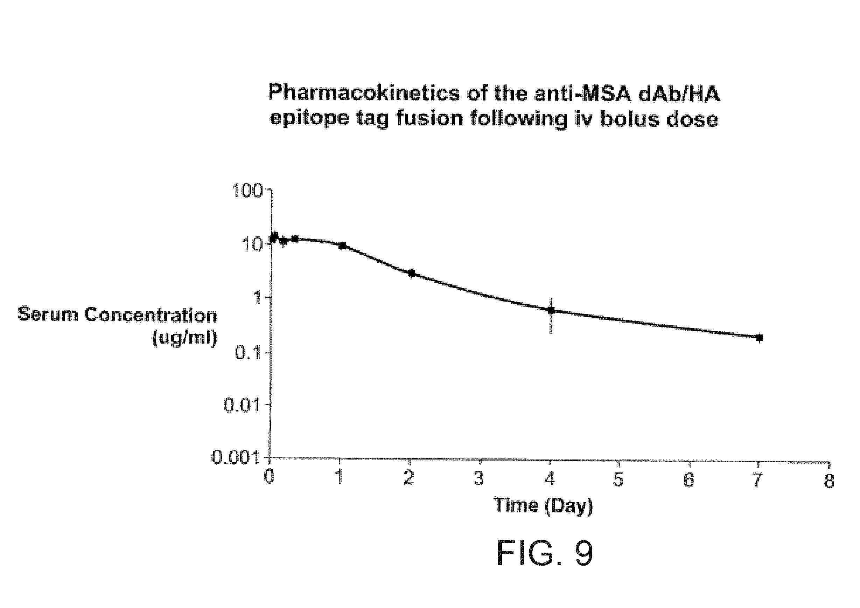

FIG. 9 is a graph showing the concentration (µg/mL) of MSA binding dAb/HA epitope tag fusion protein in mouse serum following a single intravenous (i.v.) injection (dose was about 1.5 mg/kg) into CD1 strain male animals over time (days). Serum concentration was determined by ELISA using goat anti-HA (Abcam, UK) capture and protein L-HRP (Invitrogen, USA) detection reagents. Standard curves of known concentrations of MSA binding dAb/HA fusion were set up in the presence of 1x mouse serum to ensure comparability with the test samples. Modelling with a 1 compartment model (WinNonlin Software, Pharsight Corp., USA) showed the MSA binding dAb/HA epitope tag fusion protein had a terminal phase t1/2 of 29.1 hours and an area under the curve of 559 hr·µg/mL.

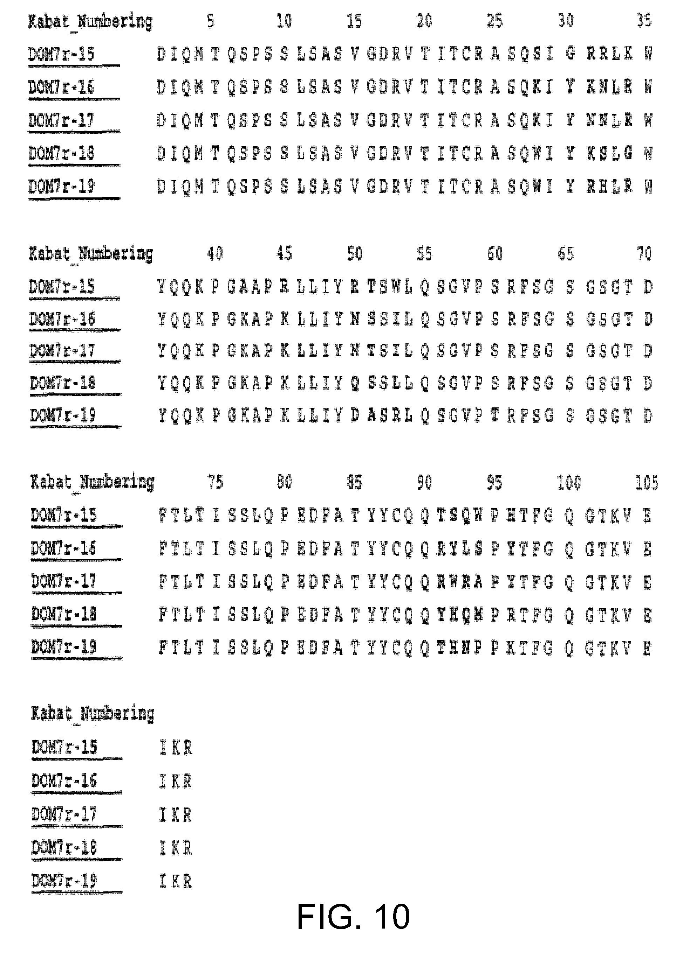

FIG. 10 is an illustration of the amino acid sequences of the amino acid sequences of V*κ*s selected by binding to rat serum albumin (RSA). The illustrated sequences are from V*κ*s designated DOM7r-15 (SEQ ID NO:37), DOM7r-16 (SEQ ID NO:38), DOM7r-17 (SEQ ID NO:39), DOM7r-18 (SEQ ID NO:40), DOM7r-19 (SEQ ID NO:41).

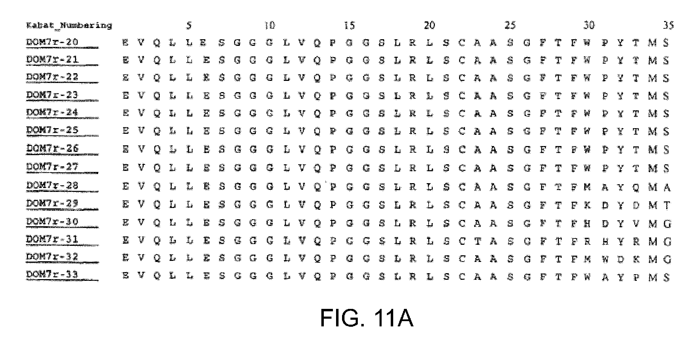

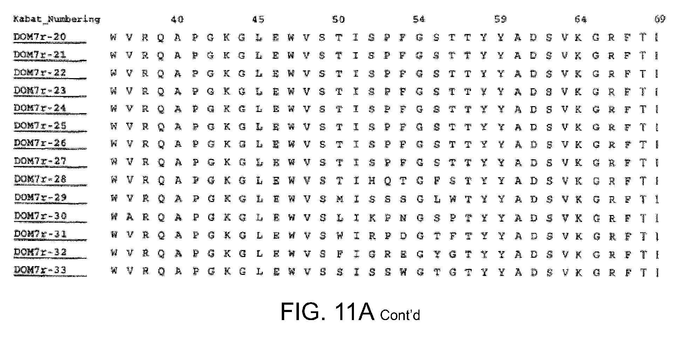

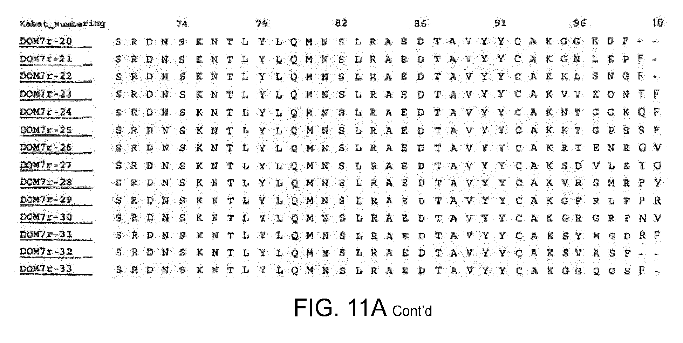

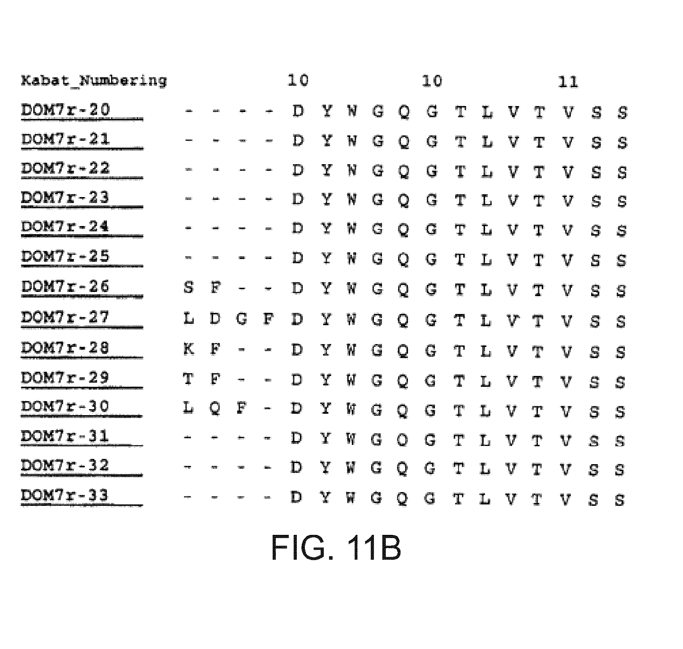

FIG. 11A-11B is an illustration of the amino acid sequences of the amino acid sequences of V*κ*s that bind rat serum albumin (RSA). The illustrated sequences are from V*κ*s designated DOM7r-20 (SEQ ID NO:42), DOM7r-21 (SEQ ID NO:43), DOM7r-22 (SEQ ID NO:44), DOM7r-23 (SEQ ID NO:45), DOM7r-24 (SEQ ID NO:46), DOM7r-25 (SEQ ID NO:47), DOM7r-26 (SEQ ID NO:48), DOM7r-27 (SEQ ID NO:49), DOM7r-28 (SEQ ID NO:50), DOM7r-29 (SEQ ID NO:51), DOM7r-30 (SEQ ID NO:52), DOM7r-31 (SEQ ID NO:53), DOM7r-32 (SEQ ID NO:54), DOM7r-33 (SEQ ID NO:55).

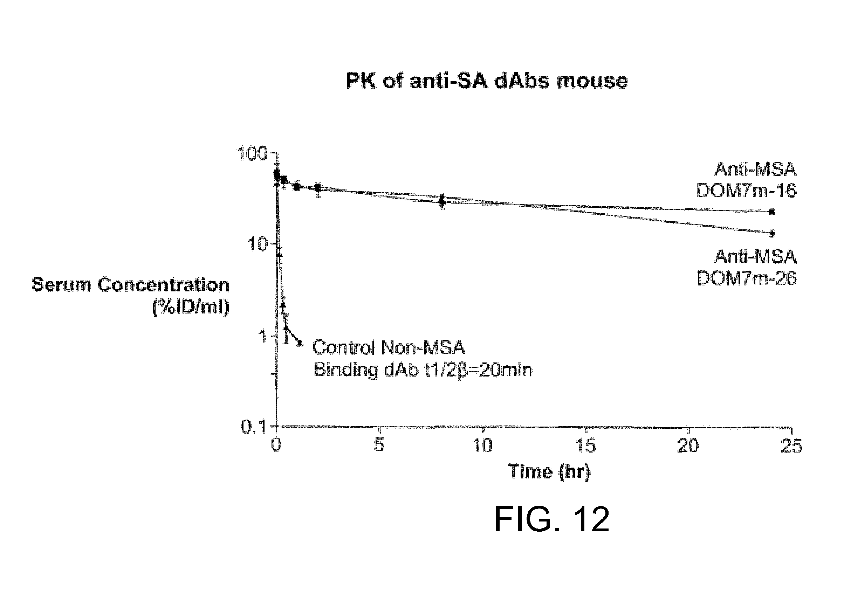

FIG. 12 is a graph showing the concentration (% initial dose) of DOM7m-16, DOM7m-26 or a control dAb that does not bind MSA, each of which contained an HA epitope tag, in mouse serum following a single intravenous (i.v.) injection (dose was about 1.5 mg/kg) into CD1 strain male animals over time. Serum concentration was determined by ELISA using goat anti-HA (Abcam, UK) capture and protein L-HRP (Invitrogen, USA) detection reagents. Standard curves of known concentrations of MSA binding dAb/HA fusion were set up in the presence of 1x mouse serum to ensure comparability with the test samples. Modelling with a 1 compartment model (WinNonlin Software, Pharsight Corp., USA) showed control dAb had a terminal phase t1/2α of 20 minutes, while DOM7m-16, DOM7m-26 persisted in serum significantly longer.

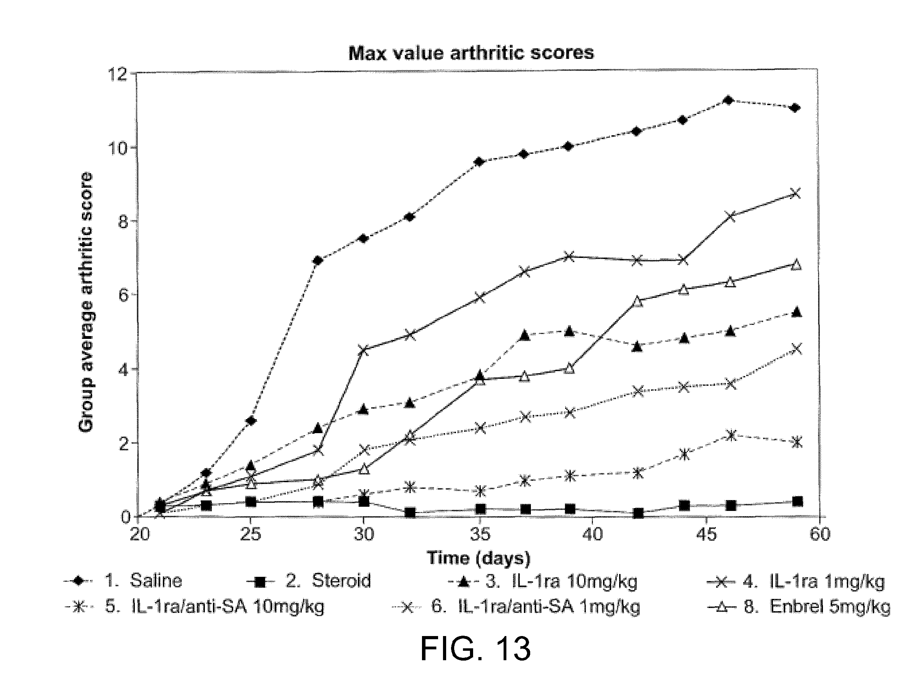

FIG. 13 is a graph showing that DOM7m-16/IL-1ra was more effective than IL-1ra or ENBREL® (entarecept; Immunex Corporation) in treating arthritis in a mouse collagen-induced arthritis (CIA) model. Arthritis was induced and, beginning on day 21, mice were treated with Dexamethasone at 0.4 mg/Kg (Steroid), DOM7m-16/IL-1ra at 1 mg/Kg (IL-1ra/anti-SA 1mg/kg) or 10 mg/Kg (IL-1ra/anti-SA 10 mg/kg), IL-1ra at 1 mg/Kg or 10 mg/Kg, ENBREL® (entarecept; Immunex Corporation) at 5 mg/Kg, or saline. The results show that DOM7m-16/IL-1ra was more effective than IL-1ra or ENBREL® (entarecept; Immunex Corporation) in this study. The response to IL-1ra was dose dependent, as expected, and that the response to DOM7m-16/IL-1ra was also dose dependent. The average scores for treatment with DOM7m-16/IL-1ra at 1 mg/Kg were consistently lower than the average scores obtained by treatment with IL-1ra at 10 mg/kg. The results indicate that treatment with DOM7m-16/IL-1ra was 10 times more effective than IL-1ra in this study.

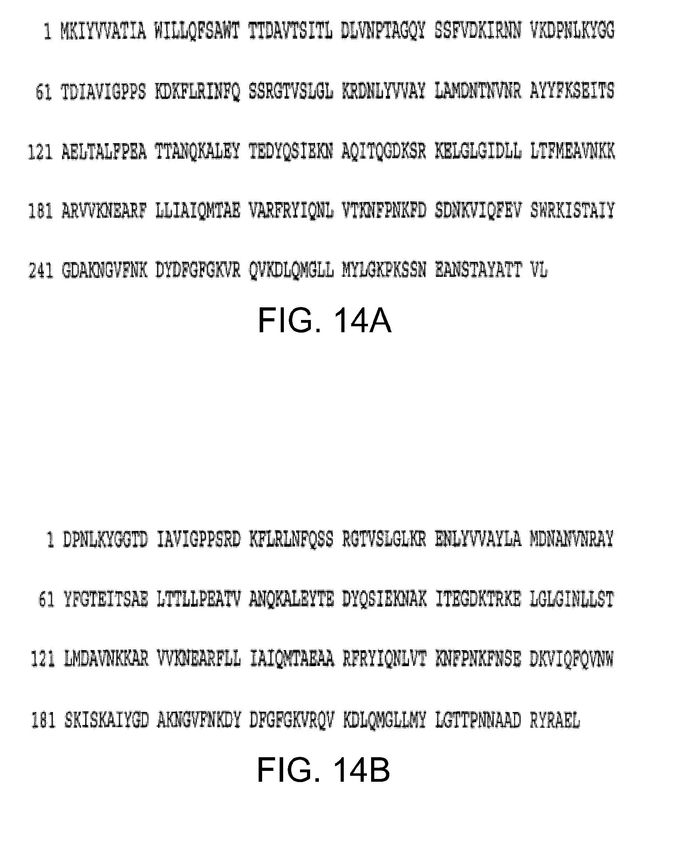

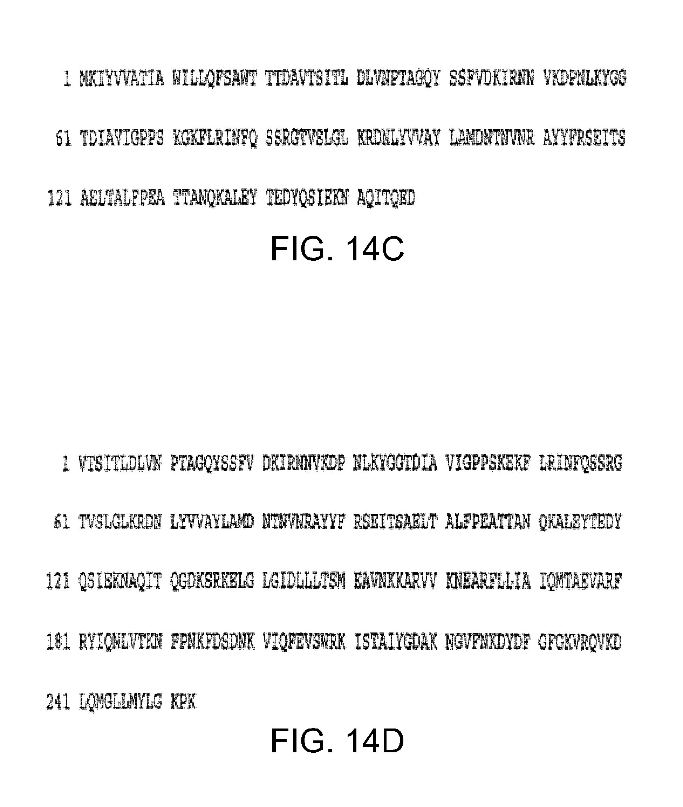

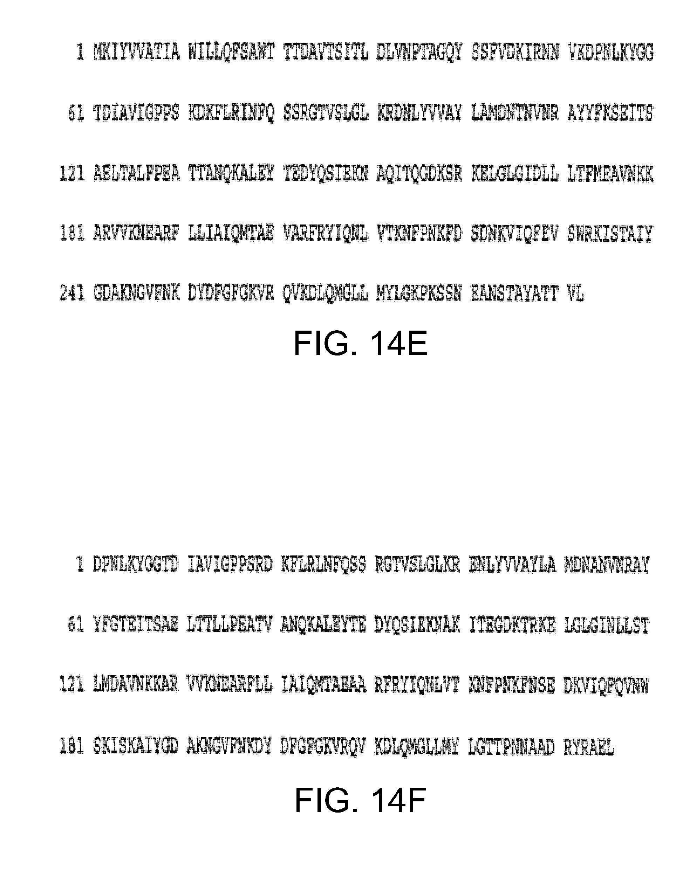

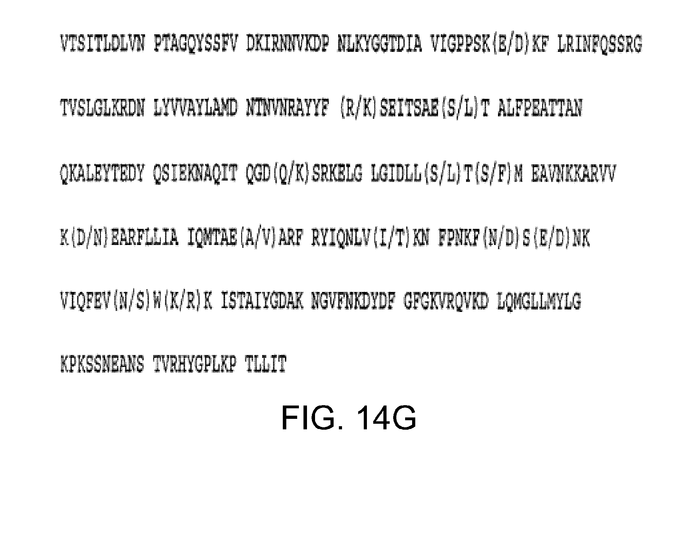

FIGS. 14A-14G illustrate the amino acid sequences of saporin polypeptides. FIG. 14A illustrates the amino acid sequence of saporin-2 precursor deposited as Swissprot Accession Number P27559 (SEQ ID NO:60). The signal peptide is amino acids 1-24 of SEQ ID NO:60. FIG. 14B illustrates the amino acid sequence of saporin-3 deposited as Swissprot Accession Number P27560 (SEQ ID NO:61). FIG. 14C illustrates the amino acid sequence of saporin-4 precursor deposited as Swissprot Accession Number P27561 (SEQ ID NO:62). The signal peptide is amino acids 1-24 of SEQ ID NO:62. FIG. 14D illustrates the amino acid sequence of saporin-5 deposited as Swissprot Accession Number Q41389 (SEQ ID NO:63). FIG. 14E illustrates the amino acid sequence of saporin-6 precursor deposited as Swissprot Accession Number P20656 (SEQ ID NO:64). The signal peptide is amino acids 1-24 of SEQ ID NO:64, and a potential propeptide is amino acids 278-299 of SEQ ID NO:64. The mature polypeptide is amino acids 25-277 of SEQ ID NO:64 (SEQ ID NO:65). FIG. 14F illustrates the amino acid sequence of saporin-7 deposited as Swissprot Accession Number Q41391 (SEQ ID NO:66). FIG. 14G illustrates a consensus amino acid sequence encompassing several variants and isoforms of saporin-6 (SEQ ID NO:67).

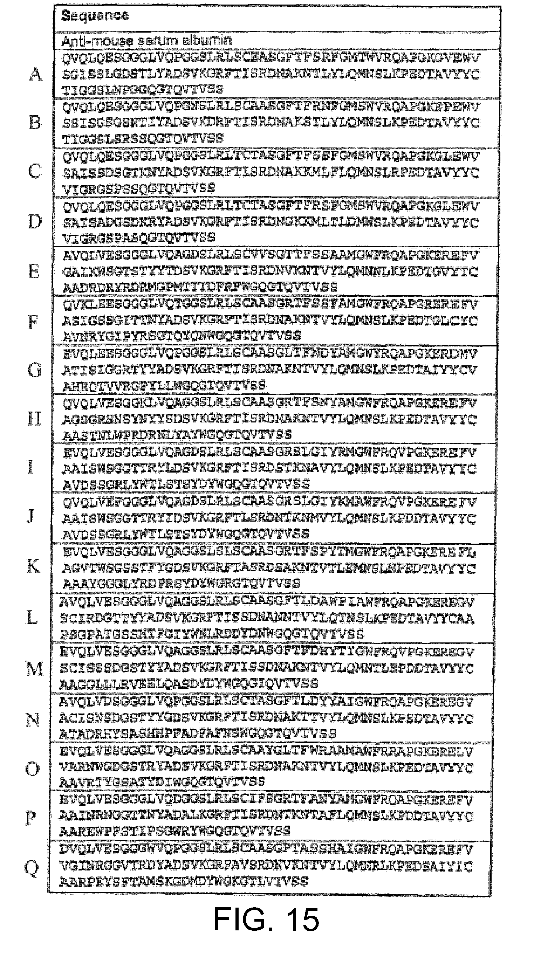

FIG. 15 illustrates the amino acid sequences of several *Camelid* V_{HH}s that bind mouse serum albumin that are disclosed in WO 2004/041862. Sequence A (SEQ ID NO:72), Sequence B (SEQ ID NO:73), Sequence C (SEQ ID NO:74), Sequence D (SEQ ID NO:75), Sequence E (SEQ ID NO:76), Sequence F (SEQ ID NO:77), Sequence G (SEQ ID NO:78), Sequence H (SEQ ID NO:79), Sequence I (SEQ ID NO:80), Sequence J (SEQ ID NO:81), Sequence K (SEQ ID NO:82), Sequence L (SEQ ID NO:83), Sequence M (SEQ ID NO:84), Sequence N (SEQ ID NO:85), Sequence O (SEQ ID NO:86), Sequence P (SEQ ID NO:87), Sequence Q (SEQ ID NO:88).
FIG 16A is an illustration of the nucleotide sequence encoding the [Pro⁹]GLP-1-Dom7h8 fusion (SEQ ID NO:175) and of the amino acid sequence of the fusion (SEQ ID NO:176).
FIG 16B is an illustration of the nucleotide sequence encoding the [Pro⁹]GLP-1-PSS-Dom7h8 fusion (SEQ ID NO:177) and of the amino acid sequence of the fusion (SEQ ID NO:178).
FIG 16C is an illustration of the nucleotide sequence encoding the [Pro⁹]GLP-1-PSSGAP-Dom7h8 fusion (SEQ ID NO:179) and of the amino acid sequence of the fusion (SEQ ID NO:180).
FIG 17 is a graph showing that [Pro⁹]GLP-1-PSSGAP-Dom7h8 fusion (□) had an equivalent dose dependent cell proliferation activity to GLP-1 control, (A), Exendin-4 (▼). Basal zero control is shown (◆).
FIG 18 is a graph showing that that [Pro⁹]GLP-1-PSSGAP-Dom7h8 fusion (□) had an equivalent dose dependent insulin release to GLP-1 control, (▲), Exendin-4 (▼). Basal zero control is shown (◆).
FIG 19A-19C illustrates the amino acid sequence of Dom7h-8 PYY (3-36) (SEQ ID NO:181), PYY (3-36) DOM7h-8 (SEQ ID NO:182) and [Pro9]GLP-1(3-37)-DOM 7h-8 PYY (3-36) (SEQ ID NO:183) fusions respectively.

### DETAILED DESCRIPTION OF THE INVENTION

Within this specification embodiments have been described in a way which enables a clear and concise specification to be written, but it is intended and will be appreciated that embodiments may be variously combined or separated without parting from the invention.

As used herein, "drug" refers to any compound (e.g., small organic molecule, nucleic acid, polypeptide) that can be administered to an individual to produce a beneficial therapeutic or diagnostic effect though binding to and/or altering the function of a biological target molecule in the individual. The target molecule can be an endogenous target molecule encoded by the individual's genome (e.g., an enzyme, receptor, growth factor, cytokine encoded by the individual's genome) or an exogenous target molecule encoded by the genome of a pathogen (e.g., an enzyme encoded by the genome of a virus, bacterium, fungus, nematode or other pathogen).

As used herein the term "drug basis" refers to activities of drug compositions and drugs that are normalized based on the amount of drug (or drug moiety) used to assess, measure or determine activity. Generally, the drug compositions of the invention (*e.g.,* drug conjugate, noncovalent drug conjugate, drug fusion) have a larger molecular weight than the drug they contain. Thus, equivalent amounts of drug composition and drug, by weight, will contain different amounts of drug on a molecular or molar basis. For example, if a drug composition of the invention has a molecular weight that is twice the molecular weight of the drug it comprises, activities can be determined on a "drug basis" using 2 µg of drug composition and 1 µg of drug, because these quantities would contain the same amount of drug (as free drug or as part of the drug composition). Activities can be normalized and expressed on a "drug basis" using appropriate calculations, for example, by expressing activity on a per target binding site basis or, for enzyme drugs, on a per active site basis.

As used herein, "drug composition" refers to a composition comprising a drug that is covalently or noncovalently bonded to a polypeptide binding moiety, wherein the polypeptide binding moiety contains a binding site (e.g., an antigen-binding site) that has binding specificity for a polypeptide that enhances.serum half-life in vivo. The drug composition can be a conjugate wherein the drug is covalently or noncovalently bonded to the polypeptide binding moiety. The drug can be covalently or noncovalently bonded to the polypeptide binding moiety directly or indirectly (e.g., through a suitable linker and/or noncovalent binding of complementary binding partners (e.g., biotin and avidin)). When complementary binding partners are employed, one of the binding partners can be covalently bonded to the drug directly or through a suitable linker moiety, and the complementary binding partner can be covalently bonded to the polypeptide binding moiety directly or through a suitable linker moiety. When the drug is a polypeptide or peptide, the drug composition can be a fusion protein, wherein the polypeptide or peptide drug and the polypeptide binding moiety are discrete parts (moieties) of a continuous polypeptide chain.

As used herein "conjugate" refers to a composition comprising an antigen-binding fragment of an antibody that binds serum albumin that is bonded to a drug. Such conjugates include "drug conjugates," which comprise an antigen-binding fragment of an antibody that binds serum albumin to which a drug is covalently bonded, and "noncovlaent drug conjugates," which comprise an antigen-binding fragment of an antibody that binds serum albumin to which a drug is noncovalently bonded.

As used herein, "drug conjugate" refers to a composition comprising an antigen-binding fragment of an antibody that binds serum albumin to which a drug is covalently bonded. The drug can be covalently bonded to the antigen-binding fragment directly or indirectly through a suitable linker moiety. The drug can be bonded to the antigen-binding fragment at any suitable position, such as the amino-terminus, the carboxyl-terminus or through suitable amino acid side chains (e.g., the *ε* amino group of lysine, or thiol group of cysteine).

As used herein, "noncovalent drug conjugate" refers to a composition comprising an antigen-binding fragment of an antibody that binds serum albumin to which a drug is noncovalently bonded. The drug can be noncovalently bonded to the antigen-binding fragment directly (e.g., electrostatic interaction, hydrophobic interaction) or indirectly (e.g., through noncovalent binding of complementary binding partners (e.g., biotin and avidin), wherein one partner is covalently bonded to drug and the complementary binding partner is covalently bonded to the antigen-binding fragment). When complementary binding partners are employed, one of the binding partners can be covalently bonded to the drug directly or through a suitable linker moiety, and the complementary binding partner can be covalently bonded to the antigen-binding fragment of an antibody that binds serum albumin directly or through a suitable linker moiety.

As used herein, "drug fusion" refers to a fusion protein that comprises an antigen-binding fragment of an antibody that binds serum albumin and a polypeptide drug. The antigen-binding fragment of an antibody that binds serum albumin and the polypeptide drug are present as discrete parts (moieties) of a single continuous polypeptide chain.

The term "albumin binding residue" as used herein means a residue which binds non-covalently to human serum albumin. The albumin binding residue attached to the therapeutic polypeptide typically has an affinity below 10 µM to human serum albumin and preferably below 1 pM. In on embodiment, a range of albumin binding residues are known among linear and branched lipohophillic moieties containing 4-40 carbon atoms, compounds with a cyclopentanophenanthrene skeleton, peptides having 10-30 amino acid residues etc.

As used herein "interleukin 1 receptor antagonist" (IL-1ra) refers to naturally occurring or endogenous mammalian IL-1ra proteins and to proteins having an amino acid sequence which is the same as that of a naturally occurring or endogenous corresponding mammalian IL-1ra protein (e.g., recombinant proteins, synthetic proteins (i.e., produced using the methods of synthetic organic chemistry)). Accordingly, as defined herein, the term includes mature protein, polymorphic or allelic variants, and other isoforms of a IL-1ra (e.g., produced by alternative splicing or other cellular processes), and modified or unmodified forms of the foregoing (e.g., lipidated, glycosylated, PEGylated). Naturally occurring or endogenous IL-1ra include wild type proteins such as mature IL-1ra, polymorphic or allelic variants and other isoforms which occur naturally in mammals (e.g., humans, non-human primates). Such proteins can be recovered or isolated from a source which naturally produces IL-1ra, for example. These proteins and IL-1ra proteins having the same amino acid sequence as a naturally occurring or endogenous corresponding IL-1ra, are referred to by the name of the corresponding mammal. For example, where the corresponding mammal is a human, the protein is designated as a human IL-1ra.

"Functional variants" of IL-1ra include functional fragments, functional mutant proteins, and/or functional fusion proteins which can be produce using suitable methods (e.g., mutagenesis (e.g., chemical mutagenesis, radiation mutagenesis), recombinant DNA techniques). A "functional variant" antagonizes interleukin-1 type 1 receptor. Generally, fragments or portions of IL-1ra include those having a deletion and/or addition (i.e., one or more amino acid deletions and/or additions) of an amino acid (i.e., one or more amino acids) relative to the mature IL-1ra (such as N-terminal, C-terminal or internal deletions). Fragments or portions in which only contiguous amino acids have been deleted or in which non-contiguous amino acids have been deleted relative to mature IL-1ra are also envisioned. A functional variant of human IL-1ra can have at least about 80%, or at least about 85%, or at least about 90%, or at least about 95%, or at least about 96%, or at least about 97%, or at least about 98%, or at least about 99% amino acid sequence identity with the mature 152 amino acid form of human IL-1ra and antagonize human Interleukin-1 type 1 receptor. (See, Eisenberg et al., Nature 343:341-346 1990). The variant can comprise one or more additional amino acids (e.g., comprise 153 or 154 or more amino acids). For example, the variant IL-1ra can have an amino acid sequence that consists of an amino-terminal methionine residue followed by residues 26 to 177 of SEQ ID NO:33. (KINERET^{®} (anakinra), Amgen).

As referred to herein, the term "about" is optional, but is preferably interpreted to mean plus or minus 20%, more preferably plus or minus 10%, even more preferably plus or minus 5%, even more preferably plus or minus 2%, most preferably plus or minus 1%.

The term "analogue" as used herein referring to a polypeptide means a modified peptide wherein one or more amino acid residues of the peptide have been substituted by other amino acid residues and/or wherein one or more amino acid residues have been deleted from the peptide and/or wherein one or more amino acid residues have been deleted from the peptide and or wherein one or more amino acid residues have been added to the peptide. Such addition or deletion of amino acid residues can take place at the N-terminal of the peptide and/or at the C-terminal of the peptide or they can be within the peptide. A simple system is used to describe analogues of GLP-1: For example [Arg³⁴] GLP-1 (7-37) Lys designates a GLP-1 analogue wherein the naturally occurring lysine at position 34 has been substituted with arginine and a lysine residue has been added to the C-terminal (position 38). Formulae of peptide analogs and derivatives thereof are drawn using standard single letter abbreviation for amino acids used according to IUPAC-IUB nomenclature.

The term "GLP-1 peptide" as used herein means GLP-1 (7-37) (SEQ ID No. 158) or GLP-1 (7-36) (SEQ ID No. 159), a GLP-1 analogue, a GLP-1 derivative or a derivative of a GLP-1 analogue. Such peptides, analogues and derivatives are insulinotropic agents.

The term "insulinotropic agent" as used herein means a compound which is able to stimulate, or cause the stimulation of, the synthesis or expression of, or the activity of the hormone insulin. Known examples of insulinotropic agents include but are not limited to glucose, GIP, GLP, Exendin, and OXM.

The term "incretin" as used herein means a type of gastrointestinal hormone that causes an increase in the amount of insulin released when glucose levels are normal or particularly when they are elevated. By way of example they include GLP-1, GIP, and OXM.

The term "exendin-4 peptide" as used herein means exendin-4 (1-39), an exendin-4 analogue, an exendin-4 derivative or a derivative of an exendin-4 analogue. In one embodiment the exendin-4 peptide is an insulinotropic agent. Such peptides, analogues and derivatives are insulinotropic agents.

The term "DPP-IV protected" as used herein referring to a polypeptide means a polypeptide which has been modified (eg, chemically modified) in order to render said compound resistant to the plasma peptidase dipeptidyl aminopeptidase-4 (DPP-IV). The DPP-IV enzyme in plasma is known to be involved in the degradation of several peptide hormones, e. g. GLP-1, GLP-2, etc. Thus, a considerable effort is being made to develop analogues and derivatives of the polypeptides susceptible to DPP-IV mediated hydrolysis in order to reduce the rate and/or extent of degradation by DPP-IV.

As used herein "saporin" refers to a family of single-chain ribosome-inactivating polypeptides produced by the plant Saponaria officinalis. (Stirpe, F., et al., Biochem. J. 216:617-625 (1983), Bagga, S. et al., J. Biol. Chem. 278:4813-4820 (2003).) Saporin polypeptides exist is several forms that differ in length and/or amino acid sequence. (See, e.g., Id. and Barthelemy, I. et al., J. Biol. Chem. 268:6541-6548 (1993).) Saporin-6 is the most active form of saporin. (Bagga, S. et al., J. Biol. Chem. 278:4813-4820 (2003).) At least four naturally occurring isoforms of saporin-6 in which the amino acid at position 48 of the mature polypeptide (SEQ ID NO:65) is Asp or Glu, and the amino acid a position 91 of the mature polypeptide (SEQ ID NO:65) is Arg of Lys have been described. (Barthelemy, I. et al., J. Biol. Chem. 268:6541-6548 (1993).) Additional forms of saporin-6 include polypeptide in which the amino acid at position 99 of the mature polypeptide (SEQ ID NO:65) is Ser of Leu, the amino acid at position 134 of the mature polypeptide (SEQ ID NO:65) is Gln or Lys, the amino acid at position 147 of the mature polypeptide (SEQ ID NO:65) is Ser or Leu, the amino acid at position 149 of the mature polypeptide (SEQ ID NO:65) is Ser or Phe, the amino acid at position 162 of the mature polypeptide (SEQ ID NO:65) is Asp or Asn, the amino acid at position 177 of the mature polypeptide (SEQ ID NO:65) is Ala or Val, the amino acid at position 188 of the mature polypeptide (SEQ ID NO:65) is Ile or Thr, the amino acid at position 196 of the mature polypeptide (SEQ ID NO:65) is Asn or Asp, the amino acid at position 198 of the mature polypeptide (SEQ ID NO:65) is Glu or Asp, the amino acid at position 231 of the mature polypeptide (SEQ ID NO:65) is Asn or Ser, and polypeptides in which the amino acid at position 233 of the mature polypeptide (SEQ ID NO:65) is Lys or Arg. (Id.) A consensus sequence encompassing these isoforms and variants is presented in FIG. 14G (SEQ ID NO:67).

Accordingly, the term "saporin" includes precursor protein, mature polypeptide, native protein, polymorphic or allelic variants, and other isoforms (e.g., produced by alternative splicing or other cellular processes), and modified or unmodified forms of the foregoing (e.g., lipidated, glycosylated, PEGylated). Naturally occurring or endogenous saporin include wild type proteins such as mature saporin (e.g., mature saporin-6), polymorphic or allelic variants and other isoforms which occur naturally in Saponaria officinalis. Such proteins can be recovered or isolated from Saponaria officinalis using any suitable methods. "Functional variants" of saporin include functional fragments, functional mutant proteins, and/or functional fusion proteins which can be produce using suitable methods (e.g., mutagenesis (e.g., chemical mutagenesis, radiation mutagenesis), recombinant DNA techniques). Generally, fragments or portions of saporin (e.g., saporin-6) include those having a deletion and/or addition (i.e., one or more amino acid deletions and/or additions) of an amino acid (i.e., one or more amino acids) relative to mature saporin (such as N-terminal, C-terminal or internal deletions). Fragments or portions in which only contiguous amino acids have been deleted or in which non-contiguous amino acids have been deleted relative to mature saporin are also envisioned. A variety of active variants of saporin can be prepared. For example, fusion proteins of saporin-6 that contain amino-terminal extensions have been prepared and shown to retain full ribosome-inhibiting activity in rabbit reticulocyte lysate assays. (Barthelemy, I. et al., J. Biol. Chem. 268:6541-6548 (1993).) Variants or saporin-6 is which an active site residue, Tyr72, Tyr120, Glu176, Arg 179 or Trp208 (amino acids 72, 120, 176, 179 or 208 of SEQ ID NO:65), was replaced with alanine had reduced cytotoxic activity in *in vitro* assays. (Bagga, S. et al., J. Biol. Chem. 278:4813-4820 (2003).) Accordingly, if preparing additional functional variants of saporin is desired, mutation, substitution, replacement, deletion or modification of the active site residues should be avoided. Preferably, a functional variant of saporin that contains fewer amino acids than naturally occurring mature polypeptide includes at least the active site. For example, a variant of saporin-6 that contains fewer amino acids than naturally occurring mature saporin-6 can include the active site residues of mature saporin-6 (Tyr72, Tyr120, Glu176, Arg 179 and Trp208 (amino acids 72, 120, 176, 179 and 208 of SEQ ID NO:65)), and be at least about 137 amino acids in length, at least about 150 amino acids in length, at least about 175 amino acids in length, at least about 200 amino acids in length, at least about 225 amino acids in length or at least about 250 amino acids in length.

A "functional variant" of saporin has ribosome-inactivating activity (e.g., rRNA N-Glycosidase activity) and/or cytotoxic activity. Such activity can readily be assessed using any suitable method, such as inhibition of protein synthesis using the well-known rabbit reticulocyte lysate assay or any of the well-known cytotoxicity assays that employ tumor cell lines. (See, e.g., Bagga, S. et al., J. Biol. Chem. 278:4813-4820 (2003) and Barthelemy, I. et al., J. Biol. Chem. 268:6541-6548 (1993).)

In some embodiments, a functional variant of saporin has at least about 80%, or at least about 85%, or at least about 90%, or at least about 91%, or at least about 92%, or at least about 93%, or at least about 94%, or at least about 95%, or at least about 96%, or at least about 97%, or at least about 98%, or at least about 99% amino acid sequence identity with mature saporin-6 (SEQ ID NO:65).

The invention relates to compositions that comprise a drug and a polypeptide binding moiety that contains an antigen-binding site that has binding specificity for a polypeptide that enhances serum half-live in vivo. As described herein in detail with respect to compositions that comprise and antigen-binding fragment of an antibody that has binding specificity for serum albumin, the drug and the binding polypeptide can be conjugated covalently or noncovalently. In some embodiments, the composition is a fusion protein that comprises a polypeptide drug and a polypeptide binding moiety that contains an antigen-binding site that has binding specificity for a polypeptide that enhances serum half-live *in vivo.* In other embodiments, the composition comprises a drug that is covalently or noncovalently bonded to a polypeptide binding moiety that contains an antigen-binding site that has binding specificity for a polypeptide that enhances serum half-live in vivo.

The invention relates to drug compositions that comprise a drug and a polypeptide binding moiety that contains a binding site (*e.g.,* an antigen-binding site) that has binding specificity for a polypeptide that enhances serum half-life *in vivo.* As described herein in detail with respect to drug compositions that comprise an antigen-binding fragment of an antibody that has binding specificity for serum albumin, the drug and the polypeptide binding moiety can be bonded to each other covalently or noncovalently. In some embodiments, the drug composition is a fusion protein that comprises a polypeptide drug and a polypeptide binding moiety that contains an antigen-binding site that has binding specificity for a polypeptide that enhances serum half-life *in vivo.* In other embodiments, the drug composition comprises a drug that is covalently or noncovalently bonded to a polypeptide binding moiety that contains an antigen-binding site that has binding specificity for a polypeptide that enhances serum half-life *in vivo.*

Typically, a polypeptide that enhances serum half-life *in vivo* is a polypeptide which occurs naturally *in vivo* and which resists degradation or removal by endogenous mechanisms which remove unwanted material from the organism (*e.g.,* human). For example, a polypeptide that enhances serum half-life *in vivo* can be selected from proteins from the extracellular matrix, proteins found in blood, proteins found at the blood brain barrier or in neural tissue, proteins localized to the kidney, liver, lung, heart, skin or bone, stress proteins, disease-specific proteins, or proteins involved in Fc transport.

Suitable polypeptides that enhance serum half-life *in vivo* include, for example, transferrin receptor specific ligand-neuropharmaceutical agent fusion proteins (see U.S. Patent No. 5,977,307, the teachings of which are incorporated herein by reference), brain capillary endothelial cell receptor, transferrin, transferrin receptor (*e.g.,* soluble transferrin receptor), insulin, insulin-like growth factor 1 (IGF 1) receptor, insulin-like growth factor 2 (IGF 2) receptor, insulin receptor, blood coagulation factor X, αl-antitrypsin and TNF 1α. Suitable polypeptides that enhance serum half-life also include alpha-1 glycoprotein (orosomucoid; AAG), alpha-1 antichymotrypsin (ACT), alpha-1 microglobulin (protein HC; AIM), antithrombin III (AT III), apolipoprotein A-1 (Apo A-1), apolipoprotein B (Apo B), ceruloplasmin (Cp), complement component C3 (C3), complement component C4 (C4), C1 esterase inhibitor (C1 INH), C-reactive protein (CRP), ferritin (FER), hemopexin (HPX), lipoprotein(a) (Lp(a)), mannose-binding protein (MBP), myoglobin (Myo), prealbumin (transthyretin; PAL), retinol-binding protein (RBP), and rheumatoid factor (RF).

Suitable proteins from the extracellular matrix include, for example, collagens, laminins, integrins and fibronectin. Collagens are the major proteins of the extracellular matrix. About 15 types of collagen molecules are currently known, found in different parts of the body, e.g. type I collagen (accounting for 90% of body collagen) found in bone, skin, tendon, ligaments, cornea, internal organs or type II collagen found in cartilage, vertebral disc, notochord, and vitreous humor of the eye.

Suitable proteins from the blood include, for example, plasma proteins (*e.g.,* fibrin, α-2 macroglobulin, serum albumin, fibrinogen (*e.g.,* fibrinogen A, fibrinogen B), serum amyloid protein A, haptoglobin, profilin, ubiquitin, uteroglobulin and α-2-microglobulin), enzymes and enzyme inhibitors (*e.g.,* plasminogen, lysozyme, cystatin C, alpha-1-antitrypsin and pancreatic trypsin inhibitor), proteins of the immune system, such as immunoglobulin proteins (*e.g.,* IgA, IgD, IgE, IgG, IgM, immunoglobulin light chains (kappa/lambda)), transport proteins *(e.g.,* retinol binding protein, α-1 microglobulin), defensins (*e.g.,* beta-defensin 1, neutrophil defensin 1, neutrophil defensin 2 and neutrophil defensin 3) and the like.

Suitable proteins found at the blood brain barrier or in neural tissue include, for example, melanocortin receptor, myelin, ascorbate transporter and the like.

Suitable polypeptides that enhances serum half-life *in vivo* also include proteins localized to the kidney (*e.g.,* polycystin, type IV collagen, organic anion transporter Kl, Heymann's antigen), proteins localized to the liver (*e.g.,* alcohol dehydrogenase, G250), proteins localized to the lung (*e.g.,* secretory component, which binds IgA), proteins localized to the heart (*e.g.,* HSP 27, which is associated with dilated cardiomyopathy), proteins localized to the skin (*e.g.,* keratin), bone specific proteins such as morphogenic proteins (BMPs), which are a subset of the transforming growth factor ß superfamily of proteins that demonstrate osteogenic activity (*e.g.,* BMP-2, BMP-4, BMP-5, BMP-6, BMP-7, BMP-8), tumor specific proteins (*e.g.,* trophoblast antigen, herceptin receptor, oestrogen receptor, cathepsins (*e.g.,* cathepsin B, which can be found in liver and spleen)).

Suitable disease-specific proteins include, for example, antigens expressed only on activated T-cells, including LAG-3 (lymphocyte activation gene), osteoprotegerin ligand (OPGL; see Nature 402, 304-309 (1999)), OX40 (a member of the TNF receptor family, expressed on activated T cells and specifically up-regulated in human T cell leukemia virus type-I (HTLV-I)-producing cells; see Immunol. 165 (1):263-70 (2000)). Suitable disease-specific proteins also include, for example, metalloproteases (associated with arthritis/cancers) including CG6512 Drosophila, human paraplegin, human FtsH, human AFG3L2, murine ftsH; and angiogenic growth factors, including acidic fibroblast growth factor (FGF-1), basic fibroblast growth factor (FGF-2), vascular endothelial growth factor/vascular permeability factor (VEGF/VPF), transforming growth factor-α (TGF-α), tumor necrosis factor-alpha (TNF-α), angiogenin, interleukin-3 (IL-3), interleukin-8 (IL-8), platelet-derived endothelial growth factor (PD-ECGF), placental growth factor (P1GF), midkine platelet-derived growth factor-BB (PDGF), and fractalkine.

Suitable polypeptides that enhance serum half-life *in vivo* also include stress proteins such as heat shock proteins (HSPs). HSPs are normally found intracellularly. When they are found extracellularly, it is an indicator that a cell has died and spilled out its contents. This unprogrammed cell death (necrosis) occurs when as a result of trauma, disease or injury, extracellular HSPs trigger a response from the immune system. Binding to extracellular HSP can result in localizing the compositions of the invention to a disease site.

Suitable proteins involved in Fc transport include, for example, Brambell receptor (also known as FcRB). This Fc receptor has two functions, both of which are potentially useful for delivery. The functions are (1) transport of IgG from mother to child across the placenta (2) protection of IgG from degradation thereby prolonging its serum half-life. It is thought that the receptor recycles IgG from endosomes. (See, Holliger et al., Nat Biotechnol 15(7):632-6 (1997).)

The drug compositions of the invention can comprise any polypeptide binding moiety that contains a binding site (*e.g.,* an antigen-binding site) that has binding specificity for a polypeptide that enhances serum half-life *in vivo.* Preferably, the polypeptide binding moiety comprises at least 31, at least about 40, at least about 50, at least about 60, at least about 70, at least about 80 amino acids, at least about 90 amino acids, at least about 100 amino acids or at lease about 110 amino acids as a separate molecular entity. Preferably, the polypeptide binding moiety binds a polypeptide that enhances serum half-life *in vivo* with a KD of at least about 5 mM KD (KD=K_{off} (kd)/Kₒₙ (ka)). In some embodiments, the polypeptide binding moiety binds a polypeptide that enhances serum half-life *in vivo* with a KD of about 10 to about 100 nM, or about 100 nM to about 500 nM, or about 500 nM to about 5 mM, as determined by surface plasmon resonance (*e.g.,* using a BIACORE instrument). In particular embodiments, the polypeptide binding moiety binds a polypeptide that enhances serum half-life *in vivo* with a KD of about 50 nM, or about 70 nM, or about 100 nM, or about 150 nM or about 200 nM.

Preferably, the polypeptide binding moiety that contains a binding site (*e.g.,* an antigen-binding site) that has binding specificity for a polypeptide that enhances serum half-life *in vivo* is not a prokaryotic or bacterial polypeptide or peptide. Preferably, the polypeptide binding moiety is a eukaryotic, mammalian or human polypeptide or peptide.

In certain embodiments, the polypeptide binding moiety that contains a binding site *(e.g.,* an antigen-binding site) that has binding specificity for a polypeptide that enhances serum half-life *in vivo* is a folded protein domain. In other embodiments, the polypeptide binding moiety has a molecular weight of at least about 4 KDa, at least about 4.5 KDa, at least about 5 KDa, at least about 5.5 KDa, at least about 6 KDa, at least about 6.5 KDa, at least about 7 KDa, at least about 7.5 KDa or at least about 8 KDa as a separate molecular entity.

Suitable polypeptide binding moieties that contain a binding site (*e.g.,* an antigen-binding site) that has binding specificity for a polypeptide that enhances serum half-life *in vivo* can be identified using any suitable method, such as by screening naturally occurring or non-naturally occurring polypeptides in a suitable adhesion assay. As described herein, preferred polypeptide binding moieties that have an antigen-binding site for a polypeptide that enhances serum half-life *in vivo* are antigen-binding fragments of antibodies that have binding specificity for serum albumin. However, antigen-binding fragments of antibodies that have binding specificity for other polypeptides that enhance serum half-life *in vivo* can be used in the invention.

If desired, one or more of the complementarity determining regions (CDRs) of an antibody or antigen-binding fragment thereof that binds a polypeptide that enhances serum half-life *in vivo* can be formatted into a non-immunoglobulin structure that retains the antigen-binding specificity of the antibody or antigen-binding fragment. The drug compositions of the invention can comprise such a non-immunoglobulin binding moiety. Such non-immunoglobulin binding moieties can be prepared using any suitable method, for example natural bacterial receptors such as SpA have been used as scaffolds for the grafting of CDRs to generate polypeptide binding moieties which specifically bind an epitope. Details of this procedure are described in U.S. Patent Application No. 5,831,012, the teachings of which are incorporated herein by reference. Other suitable scaffolds include those based on fibronectin and affibodies. Details of suitable procedures are described in WO 98/58965. Other suitable scaffolds include lipocallin and CTLA4, as described in van den Beuken et al., J. Mol. Biol. 310:591-601 (2001), and scaffolds such as those described in WO 00/69907 (Medical Research Council), which are based for example on the ring structure of bacterial GroEL or other chaperone polypeptides.

In some embodiments, the drug composition of the invention comprises a non-immunoglobulin binding moiety that has binding specificity for serum albumin, wherein the non-immunoglobulin binding moiety comprises one, two or three of the CDRs of a V_{H}, V*_{K}* or V_{HH} described herein and a suitable scaffold. In certain embodiments, the non-immunoglobulin binding moiety comprises CDR3 but not CDR1 or CDR2 of a V_{H}, V*ₖ* or V_{HH} described herein and a suitable scaffold. In other embodiments, the non-immunoglobulin binding moiety comprises CDR1 and CDR2, but not CDR3 of a V_{H}, V*ₖ* or V_{HH} described herein and a suitable scaffold. In other embodiments, the non-immunoglobulin binding moiety comprises CDR1, CDR2 and CDR3 of a V_{H}, V*ₖ* or V_{HH} described herein and a suitable scaffold. In other embodiments, the drug composition comprises only CDR3 of a V_{H}, V*ₖ* or V_{HH} described herein and a drug.

The drug compositions of the invention can be prepared using suitable methods, such as the methods described herein for preparation of drug fusions, drug conjugates and noncovalent drug conjugates. Additionally, the drug compositions of the invention have the advantages and the utilities that are described in detail herein with respect to drug fusions, drug conjugates and noncovalent drug conjugates.

The invention provides drug compositions (*e.g.,* drug conjugates, noncovalent drug conjugates, drug fusions) that have improved pharmacokinetic properties (*e.g.,* increase serum half-life) and other advantages in comparison to the drug alone (unconjugated drug, unfused drug). The drug conjugates, noncovalent drug conjugates and drug fusions comprise an antigen-binding fragment of an antibody that has binding specificity for serum albumin and one or more desired drugs.

As described herein, drug compositions (*e.g.,* drug conjugates, noncovalent drug conjugates, drug fusions) of the invention can have dramatically prolonged *in vivo* serum half-life and/or increased AUC, as compared to drug alone. In addition, the activity of the drug is generally not substantially altered in the drug composition (*e.g.,* drug conjugate, noncovalent drug conjugate, drug fusion). However, some change in the activity of a drug composition compared to drug alone is acceptable and is generally compensated for by the improved pharmacokinetic properties of the drug composition (*e.g.,* drug conjugate, noncovalent drug conjugate, drug fusion). For example, drug compositions (*e.g.,* drug conjugates, noncovalent drug conjugates, drug fusions) may bind the drug target with lower affinity than drug alone, but have about equivalent or superior efficacy in comparison to drug alone due to the improved pharmacokinetic properties (*e.g.,* prolonged *in vivo* serum half-life, larger AUC) of the drug composition. In addition, lower amounts of drug compositions (*e.g.,* drug conjugates, noncovalent drug conjugates and drug fusions) can be administered to achieve the desired therapeutic or diagnostic effect. Preferably the activity of the drug composition (e.g., drug conjugate, noncovalent drug conjugate, drug fusion) differs from that of the drug alone by a factor of no more than about 100, or no more than about 50, or no more than about 10, or no more than about 5, or no more than about 4, or no more than about 3, or no more than about 2. For example, a drug can have a KD, Ki or neutralizing dose 50 (ND50) of 1 nM, and a drug composition (*e.g.,* drug conjugate, noncovalent drug conjugate, drug fusion) can have a KD, Ki or ND50 of about 2 nM, or about 3 nM, or about 4 nM, or about 5 nM, or about 10 nM.

Preferably, the activity of the drug composition (*e.g.,* drug conjugate, noncovalent drug conjugate, drug fusion) is not substantially reduced as compared to the activity of the drug. In certain embodiments, the activity of the drug composition is reduced, relative to the activity of drug, by no more than about 10%, no more than about 9%, no more than about 8%, no more than about 7%, no more than about 6%, no more than about 5%, no more than about 4%, no more than about 3%, no more than about 2%, no more than about 1% or is substantially unchanged. Alternatively stated, the drug composition (*e.g.,* drug conjugate, noncovalent drug conjugate, drug fusion) retains at least about 90%, at least about 91%, at least about 92%, at least about 93%, at least about 94%, at least about 95%, at least about 96%, at least about 97%, at least about 98%, at least about 99% of the activity of the drug, or substantially the same activity as the drug. Preferably, the activity of drug compositions (*e.g.,* drug conjugate, noncovalent drug conjugate, drug fusion) and drugs are determined and/or compared on a "drug basis."

As described and shown herein, the drug compositions (*e.g.,* drug conjugate, noncovalent drug conjugate, drug fusion) of the invention can have greater activity (*e.g., in vivo* activity) than drug alone. For example, as shown in Example 6, DOM7m-16/IL-Ira was more effective in treating arthritis in a mouse model than IL-1ra when these agents were administered at the same dose by weight (10 mg/Kg or 1 mg/Kg). DOM7m-16/IL-1ra was more effective even though its molecular weight is approximately twice the molecular weight of IL-1ra. Thus, mice that received DOM7m-16/IL-1ra received only about half of the IL-1ra (as a moiety in DOM7m-16/IL1-ra) as mice that received IL-1ra.

In certain embodiments, the drug composition (*e.g*., drug conjugate, noncovalent drug conjugate, drug fusion) has greater activity (*e.g., in vivo* activity) than drug, for example, the drug composition can have at least about 100%, at least about 150%, at least about 200%, at least about 250%, at least about 300%, at least about 350%, at least about 400%, at least about 450%, or at least about 500% of the activity of drug. Preferably, the activity of drug compositions (*e.g.,* drug conjugate, noncovalent drug conjugate, drug fusion) and drugs are determined and/or compared on a "drug basis." The activity of drug compositions (*e.g.,* drug conjugate, noncovalent drug conjugate, drug fusion) and drugs can be determined using a suitable *in vitro* or *in vivo* system. In certain embodiments, a drug composition (*e.g.,* drug conjugate, noncovalent drug conjugate, drug fusion) has greater activity than the drug it comprises, as determined *in vivo.* In other embodiments, a drug composition (*e.g.,* drug conjugate, noncovalent drug conjugate, drug fusion) has greater activity than the drug it comprises, as determined *in vitro.*

Drug compositions (*e.g.,* drug conjugates, noncovalent drug conjugates, drug fusions) that comprise a domain antibody (dAb) that has binding specificity for serum albumin provide further advantages. Domain antibodies are very stable, are small relative to antibodies and other antigen-binding fragments of antibodies, can be produced in high yields by expression in *E*. *coli* or yeast (*e.g., Pichia pastoris*)*,* and as described herein antigen-binding fragments of antibodies that bind serum albumin can be easily selected from libraries of human origin or from any desired species. Accordingly, drug compositions (*e.g.,* drug conjugates, noncovalent drug conjugates, drug fusions) that comprise a dAb that binds serum albumin can be produced more easily than therapeutics that are generally produced in mammalian cells (*e.g.,* human, humanized or chimeric antibodies) and dAbs that are not immunogenic can be used (*e.g.,* a human dAb can be used for a drug fusion or drug conjugate for treating or diagnosing disease in humans.)

The iminunogenicity of a drug can be reduced when the drug is part of a drug composition (*e.g.,* drug conjugate, noncovalent drug conjugate, drug fusion) that contains a polypeptide binding moiety that binds serum albumin (*e.g.,* an antigen-binding fragment of an antibody that binds serum albumin). Accordingly, a drug can be less immunogenic (than drug alone) or be substantially non-immunogenic in the context of a drug composition that contains a polypeptide binding moiety that binds serum albumin (*e.g.,* drug conjugate, noncovalent drug conjugate, drug fusion). Thus, such drug compositions (*e.g.,* drug conjugates, noncovalent drug conjugates, drug fusions) can be administered to a subject repeatedly over time with minimal loss of efficacy due to the elaboration of anti-drug antibodies by the subject's immune system.

Additionally, the drug compositions (*e.g.,* drug conjugates, noncovalent drug conjugates, drug fusions) described herein can have an enhanced safety profile and fewer side effects than drug alone. For example, as a result of the serum albumin-binding activity of the antigen-binding fragment of an antibody that has binding specificity for serum albumin, the drug fusions and conjugates (drug conjugate, noncovalent drug conjugate) have enhanced residence time in the vascular circulation. Additionally, the conjugates and drug fusions are substantially unable to cross the blood brain barrier and to accumulate in the central nervous system following systemic administration (*e.g.,* intravascular administration). Accordingly, conjugates (drug conjugate, noncovalent drug conjugate) and drug fusions that contain a drug that has neurological toxicity or undesirable psychotropic effects can be administered with greater safety and reduced side effects in comparison to the drug alone. Similarly, the conjugates (drug conjugate, noncovalent drug conjugate) and drug fusions can have reduced toxicity toward particular organs (*e.g.,* kidney or liver) than drug alone. The conjugates and drug fusions described herein can also be used to sequester a drug or a target that binds a drug (*e.g,* a toxin) in the vascular circulation, thereby decreasing the effects of the drug or target on tissues (*e.g.,* inhibiting the effects of a toxin).

Suitable methods for pharmacokinetic analysis and determination of *in vivo* half-life are well known in the art. Such methods are described, for example, in Kenneth, A et al: Chemical Stability of Pharmaceuticals: A Handbook for Pharmacists, and in Peters et al, Pharmacokinetc analysis: A Practical Approach (1996). Reference is also made to "Pharmacokinetics", M Gibaldi & D Perron, published by Marcel Dekker, 2nd Rev. edition (1982), which describes pharmacokinetic parameters such as t alpha and t beta half-lives (t½ alpha, t½ beta) and area under curve (AUC).

Half-lives (t½ alpha and t½ beta) and AUC can be determined from a curve of serum concentration of conjugate or fusion against time. The WinNonlin analysis package (available from Pharsight Corp., Mountain View, CA 94040, USA) can be used, for example, to model the curve. In a first phase (the alpha phase) the drug composition (*e.g.,* drug conjugate, noncovalent drug conjugate, drug fusion) is undergoing mainly distribution in the patient, with some elimination. A second phase (beta phase) is the terminal phase when the drug composition (*e.g.,* drug conjugate, noncovalent drug conjugate, drug fusion) has been distributed and the serum concentration is decreasing as the drug composition is cleared from the patient. The t alpha half-life is the half-life of the first phase and the t beta half-life is the half-life of the second phase. Thus, the present invention provides a drug composition (*e.g.,* drug conjugate, noncovalent drug conjugate, drug fusion) or a composition comprising a drug composition (*e.g.,* drug conjugate, noncovalent drug conjugate, drug fusion) according to the invention having a tα half-life in the range of 15 minutes or more. In one embodiment, the lower end of the range is 30 minutes, 45 minutes, 1 hour, 2 hours, 3 hours, 4 hours, 5 hours, 6 hours, 7 hours, 8 hours, 9 hours, 10 hours, 11 hours or 12 hours. In addition, or alternatively, a drug composition (*e.g.,* drug conjugate, noncovalent drug conjugate, drug fusion) or composition according to the invention will have a tα half-life in the range of up to and including 12 hours. In one embodiment, the upper end of the range is 11, 10, 9, 8, 7, 6 or 5 hours. An example of a suitable range is 1 to 6 hours, 2 to 5 hours or 3 to 4 hours.

Advantageously, the present invention provides drug compositions (*e.g.,* drug conjugates, noncovalent drug conjugates, drug fusions) having a t*β* half-life in the range of 2.5 hours or more. In one embodiment, the lower end of the range is 3 hours, 4 hours, 5 hours, 6 hours, 7 hours, 8 hours, 9 hours, 10 hours, 11 hours, or 12 hours. In some embodiments, the drug compositions (*e.g.,* drug conjugates, noncovalent drug conjugates, drug fusions) have a t*β* half-life in the range of up to and including 21 days. In one embodiment, the upper end of the range is 12 hours, 24 hours, 2 days, 3 days, 5 days, 10 days, 15 days or 20 days. In particular embodiments, a drug composition (*e.g.,* drug conjugate, noncovalent drug conjugate, drug fusion) according to the invention will have a tβ half-life in the range 12 to 60 hours. In a further embodiment, it will be in the range 12 to 48 hours. In a further embodiment still, it will be in the range 12 to 26 hours.

In addition, or alternatively to the above criteria, the present invention provides drug compositions (*e.g.,* drug conjugates, noncovalent drug conjugates, drug fusions) having an AUC value(area under the curve) in the range of 0.01 mg.min/mL or more, or 1 mg.min/mL or more. In one embodiment, the lower end of the range is 0.01, 0.1, 1, 5, 10, 15, 20, 30, 100, 200 or 300 mg.min/mL. In particular embodiments, the drug composition (*e.g.,* drug conjugate, noncovalent drug conjugate, drug fusion) has an AUC in the range of up to 600 mg.min/mL. In one embodiment, the upper end of the range is 500, 400, 300, 200, 150, 100, 75 or 50 mg.min/mL. In other embodiments, the drug composition (*e.g.,* drug conjugate, noncovalent drug conjugate, drug fusion) has an AUC in the range selected from the group consisting of the following: 15 to 150 mg.min/mL, 15 to 100 mg.min/mL, 15 to 75 mg.min/mL, 15 to 50 mg.min/mL, 0.01 to 50 mg.min/mL, 0.1 to 50 mg.min/mL, 1 to 50 mg.min/mL, 5 to 50 mg.min/mL, and 10 to 50 mg.min/mL.

The invention relates to drug compositions (*e.g.,* drug conjugates, noncovalent drug conjugates, drug fusions) that comprise a drug and a polypeptide binding moiety that contains a binding site (*e.g.,* an antigen-binding site) that has binding specificity for a polypeptide that enhances serum half-life *in vivo.* In preferred embodiments of drug compositions, the polypeptide binding moiety that contains a binding site (*e.g.,* an antigen-binding site) that has binding specificity for a polypeptide that enhances serum half-life *in vivo,* has binding specificity for serum albumin.

In some embodiments, the drug composition comprises a drug that is covalently bonded to a polypeptide binding moiety that contains a binding site (e.g., an antigen-binding site) that has binding specificity for a polypeptide that enhances serum half-life *in vivo.* In these embodiments, the drug can be covalently bonded to the polypeptide binding domain at any suitable position, such as the amino-terminus, the carboxyl-terminus or through suitable amino acid side chains (*e.g.,* the ε amino group of lysine or thiol group of cysteine).

In other embodiments, the drug composition comprises a drug that is noncovalently bonded to a polypeptide binding moiety that contains a binding site (e.g., an antigen-binding site) that has binding specificity for a polypeptide that enhances serum half-life *in vivo.* In such embodiments, the drug can be noncovalently bonded to the antigen-binding fragment directly (*e.g.,* through electrostatic interaction, hydrophobic interaction) or indirectly (*e.g.,* through noncovalent binding of complementary binding partners (*e.g.,* biotin and avidin), wherein one partner is covalently bonded to drug and the complementary binding partner is covalently bonded to the antigen-binding fragment). When complementary binding partners are employed, one of the binding partners can be covalently bonded to the drug directly or through a suitable linker moiety, and the complementary binding partner can be covalently bonded to the polypeptide binding domain directly or through a suitable linker moiety.

In other embodiments, the drug composition is a fusion protein that comprises a polypeptide binding moiety that contains a binding site (*e.g.,* an antigen-binding site) that has binding specificity for a polypeptide that enhances serum half-life *in vivo* and a polypeptide drug. The fusion proteins comprise a continuous polypeptide chain, said chain comprising a polypeptide binding moiety that contains a binding site (*e.g.,* an antigen-binding site) that has binding specificity for a polypeptide that enhances serum half-life *in vivo* as a first moiety, and a polypeptide drug as a second moiety, which are present as discrete parts (moieties) of the polypeptide chain. The first and second moieties can be directly bonded to each other through a peptide bond, or linked through a suitable amino acid, or peptide or polypeptide linker. Additional moieties (*e.g.,* third, fourth) and/or linker sequences can be present as appropriate. The first moiety can be in an N-terminal location, C-terminal location or internal relative to the second moiety *(i.e.,* the polypeptide drug): In certain embodiments, the fusion protein comprises one or more one or more polypeptide binding moieties that contain a binding site that has binding specificity for a polypeptide that enhances serum half-life *in vivo* and one or more polypeptide drug moieties. In these embodiments, the fusion protein can comprise one to about ten (*e.g.,* 1, 2, 3, 4, 5, 6, 7, 8, 9 or 10) polypeptide drug moieties that can be the same or different, and one to about twenty (e.g., 1, 2, 3, 4, 5, 6, 7, 8, 9, 10, 11, 12, 13, 14, 15, 16, 17, 18 19 or 20) polypeptide binding moieties that contain a binding site that has binding specificity for a polypeptide that enhances serum half-life *in vivo* that can be the same or different.

The polypeptide binding moieties that contain a binding site that has binding specificity for a polypeptide that enhances serum half-life *in* vivo and polypeptide drug moieties can be present in any desired location. For example, proceeding from the amino terminus to the carboxyl terminus, the moieties can be present in the following order: one or more polypeptide binding moieties, one or more polypeptide drug moieties, one or more polypeptide binding moieties. In another example, proceeding from the amino terminus to the carboxyl terminus, the moieties can be present in the following order: one or more polypeptide binding moieties, one or more polypeptide drug moieties, one or more polypeptide binding moieties, one or more polypeptide drug moieties, one or more polypeptide binding moieties. As described herein, the polypeptide binding moieties and polypeptide drug moieties can be directly bonded to each other through a peptide bond, or linked through a suitable amino acid, or peptide or polypeptide linker.

In certain embodiments, the fusion protein is a continuous polypeptide chain that has the formula (amino-terminal to carboxy-terminal):
a-(P)n2-b-(X)n1-c-(Q)n3-d or a-(Q)n3-b-(X)n1-c-(P)n2-d
wherein X is a polypeptide drug;
P and Q are each independently a polypeptide binding moiety that contains a binding site that has binding specificity for a polypeptide that enhances serum half-life *in vivo;*
a, b, c and d are each independently absent or one to about 100 amino acid residues;
n1, n2 and n3 represent the number of X, P or Q moieties present, respectively;
n1 is one to about 10;
n2 is zero to about 10; and
n3 is zero to about 10,
with the proviso that both n2 and n3 are not zero; and
with the proviso that when n1 and n2 are both one and n3 is zero, X does not comprise an antibody chain or a fragment of an antibody chain.

In some embodiments, n2 is one, two, three, four, five or six, and n3 is zero. In other embodiments, n3 is one, two, three, four, five or six, and n2 is zero. In other embodiments, n1, n2 and n3 are each one.

In certain embodiments, X does not comprises an antibody chain or a fragment of an antibody chain.

In preferred embodiments, P and Q are each independently a polypeptide binding moiety that has binding specificity for serum albumin.

In particularly preferred embodiments, the drug composition (e.g., drug conjugate, noncovalent drug conjugate, drug fusion) comprises a polypeptide binding moiety that contains a binding site (e.g., an antigen-binding site) that has binding specificity for a polypeptide that enhances serum half-life *in vivo,* wherein the polypeptide binding domain is an antigen-binding fragment of an antibody that has binding specificity for serum albumin.

### Antigen-binding Fragment of an Antibody that Binds Serum Albumin

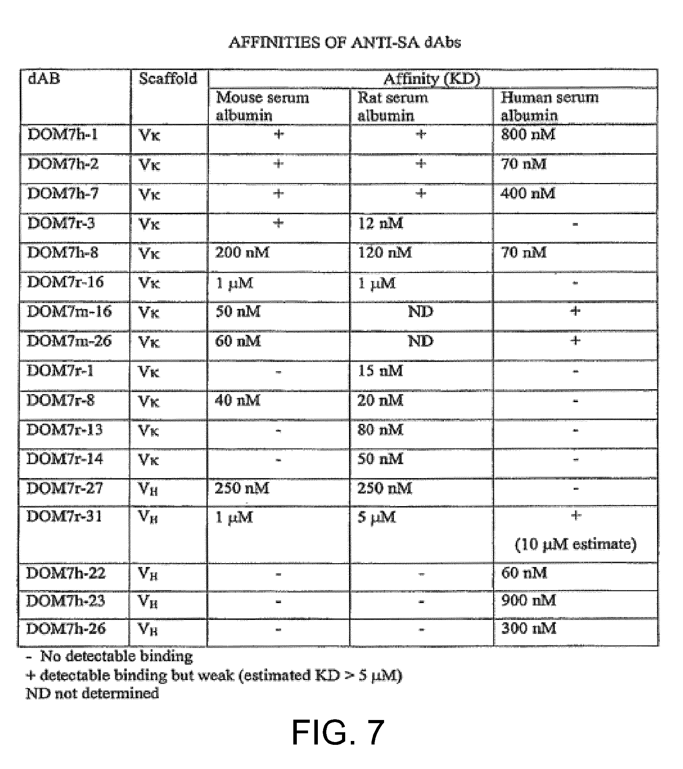

The drug conjugates, noncovalent drug conjugates and drug fusions of the invention comprise an (i. e., one or more) antigen-binding fragment of an antibody that binds serum albumin. The antigen-binding fragment can have binding specificity for serum albumin of an animal to which the drug conjugate or drug fusion will be administered. Preferably, the antigen-binding fragment has binding specificity for human serum albumin. However, veterinary applications are contemplated and the antigen-binding fragment can have binding specificity for serum albumin from a desired animal, for example serum albumin from dog, cat, horse, cow, chicken, sheep, pig, goat, deer, mink, and the like. In some embodiments the antigen-binding fragment has binding specificity for serum albumin from more than one species. For example, as described herein, human dAbs that have binding specificity for rat serum albumin and mouse serum albumin, and a dAb that has binding specificity for rat, mouse and human serum albumin have been produced. (Table 1 and FIG. 7) Such dAbs provide the advantage of allowing preclinical and clinical studies using the same drug conjugate or drug fusion and obviate the need to conduct preclinical studies with a suitable surrogate drug fusion or drug conjugate.

Antigen-binding fragments suitable for use in the invention include, for example, Fab fragments, Fab' fragments, F(ab')₂ fragments, Fv fragments (including single chain Fv (scFv) and disulfide bonded Fv), a single variable domain, and dAbs (V_{H}, V_{L}). Such antigen-binding fragments can be produced using any suitable method, such as by proteolysis of an antibody using pepsin, papain or other protease having the requisite cleavage specificity, or using recombinant techniques. For example, Fv fragments can be prepared by digesting an antibody with a suitable protease or using recombinant DNA technology. For example, a nucleic acid can be prepared that encodes a light chain variable region and heavy chain variable region that are connected by a suitable peptide linker, such as a chain of two to about twenty Glycyl residues. The nucleic acid can be introduced into a suitable host (*e.g., E. coli*) using any suitable technique (*e.g*., transfection, transformation, infection), and the host can be maintained under conditions suitable for expression of a single chain Fv fragment. A variety of antigen-binding fragments of antibodies can be prepared using antibody genes in which one or more stop codons have been introduced upstream of the natural stop site. For example, an expression construct encoding a F(ab')₂ portion of an immunoglobulin heavy chain can be designed by introducing a translation stop codon at the 3' end of the sequence encoding the hinge region of the heavy chain. The drug conjugates, noncovalent drug conjugates and drug fusions of the invention can comprise the individual heavy and light chains of antibodies that bind serum albumin or portions of the individual chains that bind serum albumin (*e.g.*, a single V_{H}, V*_{κ}* or V_{λ}).

Antibodies and antigen-binding fragments thereof which bind a desired serum albumin (*e.g*., human serum albumin) can be selected from a suitable collection of natural or artificial antibodies or raised against an appropriate immunogen in a suitable host. For example, antibodies can be raised by immunizing a suitable host (*e.g.*, mouse, human antibody-transgenic mouse, rat, rabbit, chicken, goat, non-human primate (*e.g.*, monkey)) with serum albumin (*e.g.*, isolated or purified human serum albumin) or a peptide of serum albumin (*e.g.*, a peptide comprising at least about 8, 9, 10, 11, 12, 15, 20, 25, 30, 33, 35, 37, or 40 amino acid residues). Antibodies and antigen-binding fragments that bind serum albumin can also be selected from a library of recombinant antibodies or antigen-binding fragments, such as a phage display library. Such libraries can contain antibodies or antigen-binding fragments of antibodies that contain natural or artificial amino acid sequences. For example, the library can contain Fab fragments which contain artificial CDRs (*e.g.*, random amino acid sequences) and human framework regions. (See, for example, U.S. Patent No. 6,300,064 (Knappik, et al.).) In other examples, the library contains scFv fragments or dAbs (single V_{H}, single V*_{κ}* or single V_{λ}) with sequence diversity in one or more CDRs. (*See, e.g.*, WO 99/20749 (Tomlinson and Winter), WO 03/002609 A2 (Winter et al.), WO 2004/003019A2 (Winter et al.).)

Suitable antibodies and antigen-binding fragments thereof that bind serum albumin include, for example, human antibodies and antigen-binding fragments thereof, humanized antibodies and antigen-binding fragments thereof, chimeric antibodies and antigen-binding fragments thereof, rodent (*e.g.*, mouse, rat) antibodies and antigen-binding fragments thereof, and *Camelid* antibodies and antigen-binding fragments thereof. In certain embodiments, the drug conjugates, noncovalent drug conjugates and drug fusions comprises a *Camelid* V_{HH} that binds serum albumin. *Camelid* V_{HH}s are immunoglobulin single variable domain polypeptides which are derived from heavy chain antibodies that are naturally devoid of light chains. Such antibodies occur in *Camelid* species including camel, llama, alpaca, dromedary, and guanaco. V_{HH} molecules are about ten times smaller than IgG molecules, and as single polypeptides, are very stable and resistant to extreme pH and temperature conditions. Suitable *Camelid* V_{HH} that bind serum albumin include those disclosed in WO 2004/041862 (Ablynx N.V.) and herein (FIG. 15 and SEQ ID NOS:77-88). In certain embodiments, the *Camelid* V_{HH} binds human serum albumin and comprises an amino acid sequence that has at least about 80%, or at least about 85%, or at least about 90%, or at least about 95%, or at least about 96%, or at least about 97%, or at least about 98%, or at least about 99% amino acid sequence identity with SEQ ID NO: 72, SEQ ID NO:73, SEQ ID NO:74, SEQ ID NO:75, SEQ ID NO:76, SEQ ID NO:77, SEQ ID NO:78, SEQ ID NO:79, SEQ ID NO:80, SEQ ID NO:81, SEQ ID NO:82, SEQ ID NO:83, SEQ ID NO:84, SEQ ID NO:85, SEQ ID NO:86, SEQ ID NO:87, or SEQ ID NO:88. Amino acid sequence identity is preferably determined using a suitable sequence alignment algorithm and default parameters, such as BLAST P (Karlin and Altschul, Proc. Natl. Acad. Sci. USA 87(6):2264-2268 (1990)).

Preparation of the immunizing antigen, and polyclonal and monoclonal antibody production can be performed using any suitable technique. A variety of methods have been described. (See, *e.g.,* Kohler et al., Nature, 256: 495-497 (1975) and Eur. J. Immunol. 6: 511-519 (1976); Milstein et al., Nature 266: 550-552 (1977*);* Koprowski et al., U.S. Patent No. 4,172,124; Harlow, E. and D. Lane, 1988, Antibodies: A Laboratory Manual, (Cold Spring Harbor Laboratory: Cold Spring Harbor, NY); Current Protocols In Molecular Biology, Vol. 2 (Supplement 27, Summer '94), Ausubel, F.M. et al., Eds., (John Wiley & Sons: New York, NY), Chapter 11, (1991).) Generally, where a monoclonal antibody is desired, a hybridoma is produced by fusing suitable cells from an immortal cell line (*e.g.*, a myeloma cell line such as SP2/0, P3X63Ag8.653 or a heteromyeloma) with antibody-producing cells. Antibody-producing cells can be obtained from the peripheral blood or, preferably the spleen or lymph nodes, of humans, human-antibody transgenic animals or other suitable animals immunized with the antigen of interest. Cells that produce antibodies of human origin (e.g., a human antibody) can be produced using suitable methods, for example, fusion of a human antibody-producing cell and a heteromyeloma or trioma, or immortalization of an activated human B cell via infection with Epstein Barr virus. (See, *e.g.*, U.S. Patent No. 6,197,582 (Trakht); Niedbala et al., Hybridoma, 17:299-304 (1998); Zanella et al., J Immunol Methods, 156:205-215 (1992); Gustafsson et al., Hum Antibodies Hybridomas, 2:26-32 (1991).) The fused or immortalized antibody-producing cells (hybridomas) can be isolated using selective culture conditions, and cloned by limiting dilution. Cells which produce antibodies with the desired specificity can be identified using a suitable assay (*e.g.*, ELISA).

Antibodies also can be prepared directly (*e.g.*, synthesized or cloned) from an isolated antigen-specific antibody producing cell (*e.g*., a cell from the peripheral blood or, preferably the spleen or lymph nodes determined to produce an antibody with desired specificity), of humans, human-antibody transgenic animals or other suitable animals immunized with the antigen of interest (see, *e.g.*, U.S. Patent No. 5,627,052 (Schrader)).

When the drug conjugate, noncovalent drug conjugate or drug fusion is for administration to a human, the antibody or antigen-binding fragment thereof that binds serum albumin (e.g., human serum albumin) can be a human, humanized or chimeric antibody or an antigen-binding fragment of such an antibody. These types of antibodies and antigen-binding fragments are less immunogenic or non-immunogenic in humans and provide well-known advantages. For example, drug conjugates, noncovalent drug conjugates or drug fusions that contain an antigen-binding fragment of a human, humanized or chimeric antibody can be administered repeatedly to a human with less or no loss of efficacy (compared with other fully immunogenic antibodies) due to elaboration of human antibodies that bind to the drug conjugate or drug fusion. When the drug conjugate, noncovalent drug conjugate or drug fusion is intended for veterinary administration, analogous antibodies or antigen-binding fragments can be used. For example, CDRs from a murine or human antibody can be grafted onto framework regions from a desired animal, such as a horse or cow.

Human antibodies and nucleic acids encoding same can be obtained, for example, from a human or from human-antibody transgenic animals. Human-antibody transgenic animals (e.g., mice) are animals that are capable of producing a repertoire of human antibodies, such as XENOMOUSE (Abgenix, Fremont, CA), HUMAB-MOUSE, KIRIN TC MOUSE or KM-MOUSE (MEDAREX, Princeton, NJ). Generally, the genome of human-antibody transgenic animals has been altered to include a transgene comprising DNA from a human immunoglobulin locus that can undergo functional rearrangement. An endogenous immunoglobulin locus in a human-antibody transgenic animal can be disrupted or deleted to eliminate the capacity of the animal to produce antibodies encoded by an endogenous gene. Suitable methods for producing human-antibody transgenic animals are well known in the art. (See, for example, U.S. Pat. Nos. 5,939,598 and 6,075,181 (Kucherlapati et al.), U.S. Pat. Nos. 5,569,825, 5,545,806, 5,625,126, 5,633,425, 5,661,016, and 5,789,650 (Lonberg et al.*),* Jakobovits et al., Proc. Natl. Acad. Sci. USA, 90: 2551-2555 (1993), Jakobovits et al., Nature, 362: 255-258 (1993), Jakobovits et al. WO 98/50433, Jakobovits et al. WO 98/24893, Lonberg et al. WO 98/24884, Lonberg et al. WO 97/13852, Lonberg et al. WO 94/25585, Lonberg et al. EP 0 814 259 A2, Lonberg et al. GB 2 272 440 A, Lonberg et al., Nature 368:856-859 (1994), Lonberg et al., Int Rev Immunol 13(1):65-93 (1995), Kucherlapati et al. WO 96/34096, Kucherlapati et al. EP 0 463 151 B1, Kucherlapati et al. EP 0 710 719 A1, Surani et al. US. Pat. No. 5,545,807, Bruggemann et al. WO 90/04036, Bruggemann et al. EP 0 438 474 B1, Taylor et al., Int. Immunol. 6(4)579-591 (1994), Taylor et al., Nucleic Acids Research 20(23):6287-6295 (1992), Green et al., Nature Genetics 7:13-21 (1994), Mendez et al., Nature Genetics 15:146-156 (1997), Tuaillon et al., Proc Natl Acad Sci USA 90(8):3720-3724 (1993) and Fishwild et al., Nat Biotechnol 14(7):845-851 (1996), the teachings of each of the foregoing are incorporated herein by reference in their entirety.)

Human-antibody transgenic animals can be immunized with a suitable antigen (*e.g.*, human serum albumin), and antibody producing cells can be isolated and fused to form hybridomas using conventional methods. Hybridomas that produce human antibodies having the desired characteristics (*e.g*., specificity, affinity) can be identified using any suitable assay (*e.g*., ELISA) and, if desired, selected and subcloned using suitable culture techniques.

Humanized antibodies and other CDR-grafted antibodies can be prepared using any suitable method. The CDRs of a CDR-grafted antibody can be derived from a suitable antibody which binds a serum albumin (referred to as a donor antibody). Other sources of suitable CDRs include natural and artificial serum albumin-specific antibodies obtained from human or nonhuman sources, such as rodent (*e.g.*, mouse, rat, rabbit), chicken, pig, goat, non-human primate (*e.g*., monkey) or a library.

The framework regions of a humanized antibody are preferably of human origin, and can be derived from any human antibody variable region having sequence similarity to the analogous or equivalent region (*e.g.*, heavy chain variable region or light chain variable region) of the antigen-binding region of the donor antibody. Other sources of framework regions of human origin include human variable region consensus sequences. (See, *e.g.,* Kettleborough, C.A. et al., Protein Engineering 4:773-783 (1991); Carter et al., WO 94/04679; Kabat, E.A., et al., Sequences of Proteins of Immunological Interest, Fifth Edition, U.S. Department of Health and Human Services, U.S. Government Printing Office (1991)). Other types of CDR grafted antibodies can contain framework regions of suitable origin, such as framework regions encoded by germline antibody gene segments from horse, cow, dog, cat and the like.

Framework regions of human origin can include amino acid substitutions or replacements, such as "back mutations" which replace an amino acid residue in the framework region of human or animal origin with a residue from the corresponding position of the donor antibody. One or more mutations in the framework region can be made, including deletions, insertions and substitutions of one or more amino acids. Variants can be produced by a variety of suitable methods, including mutagenesis of nonhuman donor or acceptor human chains. (See, *e.g.*, U.S. Patent Nos. 5,693,762 (Queen et al.) and 5,859,205 (Adair et al.), the entire teachings of which are incorporated herein by reference.)

Constant regions of antibodies, antibody chains (*e.g.*, heavy chain, light chain) or fragments or portions thereof, if present, can be derived from any suitable source. For example, constant regions of human, humanized and certain chimeric antibodies, antibody chains (*e.g.*, heavy chain, light chain) or fragments or portions thereof, if present can be of human origin and can be derived from any suitable human antibody or antibody chain. For example, a constant region of human origin or portion thereof can be derived from a human *K* or X light chain, and/or a human γ (*e.g*., γ1, γ2, γ3, γ4), *µ*, *α* (*e.g*., *α*1, *α*2), *δ* or *ε* heavy chain, including allelic variants. In certain embodiments, the antibody or antigen-binding fragment (*e.g*., antibody of human origin, human antibody) can include amino acid substitutions or replacements that alter or tailor function (*e.g*., effector function). For example, a constant region of human origin (*e.g.*, γ1 constant region, γ2 constant region) can be designed to reduce complement activation and/or Fc receptor binding. (See, for example, U.S. Patent Nos. 5,648,260 (Winter et al.), 5,624,821 (Winter et al.) and 5,834,597 (Tso et al.), the entire teachings of which are incorporated herein by reference.) Preferably, the amino acid sequence of a constant region of human origin that contains such amino acid substitutions or replacements is at least about 95% identical over the full length to the amino acid sequence of the unaltered constant region of human origin, more preferably at least about 99% identical over the full length to the amino acid sequence of the unaltered constant region of human origin.

Humanized antibodies, CDR grafted antibodies or antigen-binding fragments of a humanized or CDR grafted antibody can be prepared using any suitable method. Several such methods are well-known in the art. (See, *e.g.,* U.S. Patent No. 5,225,539 (Winter), U.S. Patent No. 5,530,101 (Queen et al.).) The portions of a humanized or CDR grafted antibody (*e.g.,* CDRs, framework, constant region) can be obtained or derived directly from suitable antibodies (*e.g*., by *de novo* synthesis of a portion), or nucleic acids encoding an antibody or chain thereof having the desired property (*e.g.*, binds serum albumin) can be produced and expressed. To prepare a portion of a chain, one or more stop codons can be introduced at the desired position. For example, nucleic acid (*e.g.*, DNA) sequences coding for humanized or CDR grafted variable regions can be constructed using PCR mutagenesis methods to alter existing DNA sequences. (See, *e.g.,* Kamman, M., et al., Nucl. Acids Res. 17:5404 (1989).) PCR primers coding for the new CDRs can be hybridized to a DNA template of a previously humanized variable region which is based on the same, or a very similar, human variable region (Sato, K., et al., Cancer Research 53:851-856 (1993)). If a similar DNA sequence is not available for use as a template, a nucleic acid comprising a sequence encoding a variable region sequence can be constructed from synthetic oligonucleotides (see *e.g.,* Kolbinger, F., Protein Engineering 8:971-980 (1993)). A sequence encoding a signal peptide can also be incorporated into the nucleic acid (*e.g.,* on synthesis, upon insertion into a vector). The natural signal peptide sequence from the acceptor antibody, a signal peptide sequence from another antibody or other suitable sequence can be used (see, *e.g.,* Kettleborough, C.A., Protein Engineering 4:773-783 (1991)). Using these methods or other suitable methods, variants can be readily produced. In one embodiment, cloned variable regions can be mutated, and sequences encoding variants with the desired specificity can be selected (*e.g.*, from a phage library; see, *e.g.,* U.S. Patent No. 5,514,548 (Krebber et al.) and WO 93/06213 (Hoogenboom et al.)).

The antibody or antigen-binding fragment that binds serum albumin can be a chimeric antibody or an antigen-binding fragment of a chimeric antibody. The chimeric antibody or antigen-binding fragment thereof comprises a variable region from one species *(e.g.,* mouse) and at least a portion of a constant region from another species *(e.g.,* human). Chimeric antibodies and antigen-binding fragments of chimeric antibodies can be prepared using any suitable method. Several suitable methods are well-known in the art. (See, *e.g.,* U.S. Patent No. 4,816,567 (Cabilly et al.), U.S. Patent No. 5,116,946 (Capon et al.).)

A preferred method for obtaining antigen-binding fragments of antibodies that bind serum albumin comprises selecting an antigen-binding fragment *(e.g.,* scFvs, dAbs) that has binding specificity for a desired serum albumin from a repertoire of antigen-binding fragments. For example, as described herein dAbs that bind serum albumin can be selected from a suitable phage display library. A number of suitable bacteriophage display libraries and selection methods (*e.g*., monovalent display and multivalent display systems) have been described. (See, *e.g.,* Griffiths et al., U.S. Patent No. 6,555,313 B1 (incorporated herein by reference); Johnson et al., U.S. Patent No. 5,733,743 (incorporated herein by reference); McCafferty et al., U.S. Patent No. 5,969,108 (incorporated herein by reference); Mulligan-Kehoe, U.S. Patent No. 5,702,892 (incorporated herein by reference); Winter, G. et al., Annu. Rev. Immunol. 12:433-455 (1994); Soumillion, P. et al., Appl. Biochem. Biotechnol. 47(2-3):175-189 (1994); Castagnoli, L. et al., Comb. Chem. High Throughput Screen, 4(2):121-133 (2001); WO 99/20749 (Tomlinson and Winter); WO 03/002609 A2 (Winter et al.); WO 2004/003019A2 (Winter et al.).) The polypeptides displayed in a bacteriophage library can be displayed on any suitable bacteriophage, such as a filamentous phage *(e.g.,* fd, M13, F1), a lytic phage *(e.g.,* T4, T7, lambda), or an RNA phage *(e.g.,* MS2), for example, and selected for binding to serum albumin *(e.g.,* human serum albumin).

Generally, a library of phage that displays a repertoire of polypeptides as fusion proteins with a suitable phage coat protein is used. Such a library can be produced using any suitable methods, such as introducing a library of phage vectors or phagemid vectors encoding the displayed antibodies or antigen-binding fragments thereof into suitable host bacteria, and culturing the resulting bacteria to produce phage (*e.g*., using a suitable helper phage or complementing plasmid if desired). The library of phage can be recovered from such a culture using any suitable method, such as precipitation and centrifugation.

The library can comprise a repertoire of antibodies or antigen-binding fragments thereof that contains any desired amount of amino acid sequence diversity. For example, the repertoire can contain antibodies or antigen-binding fragments thereof that have amino acid sequences that correspond to naturally occurring antibodies from a desired organism, and/or can contain one or more regions of random or randomized amino acid sequences *(e.g.,* CDR sequences). The antibodies or antigen-binding fragments thereof in such a repertoire or library can comprise defined regions of random or randomized amino acid sequence and regions of common amino acid sequence. In certain embodiments, all or substantially all polypeptides in a repertoire are a desired type of antigen-binding fragment of an antibody *(e.g.,* human V_{H} or human V_{L}). For example, each polypeptide in the repertoire can contain a V_{H}, a V_{L} or an Fv *(e.g.,* a single chain Fv).

Amino acid sequence diversity can be introduced into any desired region of antibodies or antigen-binding fragments thereof using any suitable method. For example, amino acid sequence diversity can be introduced into a target region, such as a complementarity determining region of an antibody variable domain, by preparing a library of nucleic acids that encode the diversified antibodies or antigen-binding fragments thereof using any suitable mutagenesis methods *(e.g.,* low fidelity PCR, oligonucleotide-mediated or site directed mutagenesis, diversification using NNK codons) or any other suitable method. If desired, a region of the antibodies or antigen-binding fragments thereof to be diversified can be randomized.

A suitable phage display library can be used to selected antibodies or antigen-binding fragments of antibodies that bind serum albumin and have other beneficial properties. For example, antibodies or antigen-binding fragments that resist aggregation when unfolded can be selected. Aggregation is influenced by polypeptide concentration and is thought to arise in many cases from partially folded or unfolded intermediates. Factors and conditions that favour partially folded intermediates, such as elevated temperature and high polypeptide concentration, promote irreversible aggregation. (Fink, A.L., Folding & Design 3:R1-R23 (1998).) For example, storing purified polypeptides in concentrated form, such as a lyophilized preparation, frequently results in irreversible aggregation of at least a portion of the polypeptides. Also, production of a polypeptide by expression in biological systems, such as *E. coli,* often results in the formation of inclusion bodies which contain aggregated polypeptides. Recovering active polypeptides from inclusion bodies can be very difficult and require adding additional steps, such as a refolding step, to a biological production system.

Antibodies and antigen-binding fragments that resist aggregation and unfold reversibly when heated can be selected from a suitable phage display library. Generally, a phage display library comprising a repertoire of displayed antibodies or antigen-binding fragments thereof is heated to a temperature (Ts) at which at least a portion of the displayed antibodies or antigen-binding fragments thereof are unfolded, then cooled to a temperature (Tc) wherein Ts>Tc, whereby at least a portion of the antibodies or antigen-binding fragments thereof have refolded and a portion of the polypeptides have aggregated. Then, antibodies or antigen-binding fragments thereof that unfold reversibly and bind serum albumin are recovered at a temperature (Tr). The recovered antibody or antigen-binding fragment thereof that unfolds reversibly has a melting temperature (Tm), and preferably, the repertoire was heated to Ts, cooled to Tc and the antibody or antigen-binding fragment thereof that unfolds reversibly was isolated at Tr, such that Ts>Tm>Tc, and Ts>Tm>Tr. Generally, the phage display library is heated to about 80°C and cooled to about room temperature or about 4°C before selection. Antibodies or antigen-binding fragment thereof that unfold reversibly and resist aggregation can also be designed or engineered by replacing certain amino acid residue with residues that confer the ability to unfold reversibly. (See, WO 2004/101790 (Jespers et al.), and U.S. Provisional Patent Application Nos: 60/470,340 (filed on May 14, 2003) and 60/554,021 (filed on March 17, 2004) for detailed discussion of methods for selecting and for designing or engineering antibodies or antigen-binding fragments thereof that unfold reversibly. The teachings of WO 2004/101790 and both of the foregoing U.S. Provisional Patent Applications are incorporated herein by reference.).

Antibodies or antigen-binding fragments thereof that unfold reversibly and resist aggregation provide several advantages. For example, due to their resistance to aggregation, antibodies or antigen-binding fragments thereof that unfold reversibly can readily be produced in high yield as soluble proteins by expression using a suitable biological production system, such as *E. coli.* In addition, antibodies or antigen-binding fragments thereof that unfold reversibly can be formulated and/or stored at higher concentrations than conventional polypeptides, and with less aggregation and loss of activity. DOM7h-26 (SEQ ID NO:20) is a human V_{H} that unfolds reversibly.

Preferably, the antibody or antigen-binding fragment thereof that binds serum albumin comprises a variable domain (V_{H}, Vκ, V_{λ}) in which one or more of the framework regions (FR) comprise (a) the amino acid sequence of a human framework region, (b) at least 8 contiguous amino acids of the amino acid sequence of a human framework region, or (c) an amino acid sequence encoded by a human germline antibody gene segment, wherein said framework regions are as defined by Kabat. In certain embodiments, the amino acid sequence of one or more of the framework regions is the same as the amino acid sequence of a corresponding framework region encoded by a human germline antibody gene segment, or the amino acid sequences of one or more of said framework regions collectively comprise up to 5 amino acid differences relative to the amino acid sequence of said corresponding framework region encoded by a human germline antibody gene segment.

In other embodiments, the amino acid sequences of FR1, FR2, FR3 and FR4 are the same as the amino acid sequences of corresponding framework regions encoded by a human germline antibody gene segment, or the amino acid sequences of FR1, FR2, FR3 and FR4 collectively contain up to 10 amino acid differences relative to the amino acid sequences of corresponding framework regions encoded by said human germline antibody gene segments. In other embodiments, the amino acid sequence of said FR1, FR2 and FR3 are the same as the amino acid sequences of corresponding framework regions encoded by said human germline antibody gene segment.

In particular embodiments, the antigen binding fragment of an antibody that binds serum albumin comprises an immunoglobulin variable domain *(e.g.,* V_{H}, V_{L}) based on a human germline sequence, and if desired can have one or more diversified regions, such as the complementarity determining regions. Suitable human germline sequence for V_{H} include, for example, sequences encoded by the V_{H} gene segments DP4, DP7, DP8, DP9, DP10, DP31, DP33, DP45, DP46, DP47, DP49, DP50, DP51, DP53, DP54, DP65, DP66, DP67, DP68 and DP69, and the JH segments JH1, JH2, JH3, JH4, JH4b, JH5 and JH6. Suitable human germline sequence for V_{L} include, for example, sequences encoded by the V*κ* gene segments DPK1, DPK2, DPK3, DPK4, DPK5, DPK6, DPK7, DPK8, DPK9, DPK10, DPK12, DPK13, DPK15, DPK16, DPK18, DPK19, DPK20, DPK21, DPK22, DPK23, DPK24, DPK25, DPK26 and DPK 28, and the JK segments J*κ* 1, J*κ* 2, J*κ* 3, J*κ* 4 and J*κ* 5.

In certain embodiments, the drug conjugate, noncovalent drug conjugate or drug fusion does not contain a mouse, rat and/or rabbit antibody that binds serum albumin or antigen-binding fragment of such an antibody.

The antigen-binding fragment can bind serum albumin with any desired affinity, on rate and off rate. The affinity (KD), on rate (Kₒₙ or kₐ) and off rate (K_{off} or k_{d} or K_{d}) can be selected to obtain a desired serum half-life for a particular drug. For example, it may be desirable to obtain a maximal serum half-life for a drug that neutralizes an inflammatory mediator of a chronic inflammatory disorder (*e.g*., a dAb that binds and neutralizes an inflammatory cytokine), while a shorter half-life may be desirable for a drug that has some toxicity *(e.g.,* a chemotherapeutic agent). Generally, a fast on rate and a fast or moderate off rate for binding to serum albumin is preferred. Drug conjugates and drug fusions that comprise an antigen-binding fragment with these characteristics will quickly bind serum albumin after being administered, and will dissociate and rebind serum albumin rapidly. These characteristics will reduce rapid clearance of the drug *(e.g.,* through the kidneys) but still provide efficient delivery and access to the drug target.

The antigen-binding fragment that binds serum albumin *(e.g.,* dAb) generally binds with a KD of about 1 nM to about 500 *µ*M. In some embodiments, the antigen-binding fragment binds serum albumin with a KD (KD=K_{off}(kd)/Kₒₙ (ka)) of about 10 to about 100 nM, or about 100 nM to about 500 nM, or about 500 nM to about 5 mM, as determined by surface plasmon resonance (*e.g*., using a BIACORE instrument). In particular embodiments, the drug conjugate, noncovalent drug conjugate or drug fusion comprises and antigen-binding fragment of an antibody *(e.g.,* a dAb) that binds serum albumin *(e.g.,* human serum albumin) with a KD of about 50 nM, or about 70 nM, or about 100 nM, or about 150 nM or about 200 nM. The improved pharmacokinetic properties *(e.g.,* prolonged t1/2*β*, increased AUC) of drug conjugates, noncovalent drug conjugates and drug fusions described herein may correlate with the affinity of the antigen-binding fragment that binds serum albumin. Accordingly, drug conjugates, noncovalent drug conjugates and drug fusions that have improved pharmacokinetic properties can generally be prepared using an antigen-binding fragment that binds serum albumin *(e.g.,* human serum albumin) with high affinity *(e.g.,* KD of about 500 nM or less, about 250 nM or less, about 100 nM or less, about 50 nM or less, about 10 nM or less, or about 1 nM or less, or about 100 pM or less).

Preferably, the drug that is conjugated or fused to the antigen-binding fragment that binds serum albumin, binds to its target (the drug target) with an affinity (KD) that is stronger than the affinity of the antigen-binding fragment for serum albumin and/or a K_{off}(kd) that is faster that the K_{off} of the antigen binding fragment for serum albumin, as measured by surface plasmon resonance *(e.g.,* using a BIACORE instrument). For example, the drug can bind its target with an affinity that is about 1 to about 100000, or about 100 to about 100000, or about 1000 to about 100000, or about 10000 to about 100000 times stronger than the affinity of antigen-binding fragment that binds SA for SA. For example, the antigen-binding fragment of the antibody that binds SA can bind with an affinity of about 10 *µ*M, while the drug binds its target with an affinity of about 100 pM.

In particular embodiments, the antigen-binding fragment of an antibody that binds serum albumin is a dAb that binds human serum albumin. For example, a V*_{κ}* dAb having an amino acid sequence selected from the group consisting of SEQ ID NO:10, SEQ ID NO:11, SEQ ID NO:12, SEQ ID NO:13, SEQ ID NO:14, SEQ ID NO:15, SEQ ID NO:24, SEQ ID NO:25 and SEQ ID NO:26, or a V_{H} dAb having an amino acid sequence selected from the group consisting of SEQ ID NO:16, SEQ ID NO:17, SEQ ID NO:18, SEQ ID NO:19, SEQ ID NO:20, SEQ ID NO:21, SEQ ID NO:22 and SEQ ID NO:23. In other embodiments, the antigen-binding fragment of an antibody that binds serum albumin is a dAb that binds human serum albumin and comprises the CDRs of any of the foregoing amino acid sequences. In other embodiments, the antigen-binding fragment of an antibody that binds serum albumin is a dAb that binds human serum albumin and comprises an amino acid sequence that has at least about 80%, or at least about 85%, or at least about 90%, or at least about 95%, or at least about 96%, or at least about 97%, or at least about 98%, or at least about 99% amino acid sequence identity with SEQ ID NO:10, SEQ ID NO:11, SEQ ID NO:12, SEQ ID NO:13, SEQ ID NO:14, SEQ ID NO:15, SEQ ID NO:24, SEQ ID NO:25, SEQ ID NO:26, SEQ ID NO:16, SEQ ID NO:17, SEQ ID NO:18, SEQ ID NO:19, SEQ ID NO:20, SEQ ID NO:21, SEQ ID NO:22 or SEQ ID NO:23. Amino acid sequence identity is preferably determined using a suitable sequence alignment algorithm and default parameters, such as BLAST P (Karlin and Altschul, Proc. Natl. Acad. Sci. USA 87(6):2264-2268 (1990)).

### Drugs

Certain drug compositions of the invention *(e.g.,* drug conjugates, noncovalent drug conjugates) can comprise any drug (*e.g*., small organic molecule, nucleic acid, polypeptide) that can be administered to an individual to produce a beneficial therapeutic or diagnostic effect, for example, through binding to and/or altering the function of a biological target molecule in the individual. Other drug compositions of the invention (e.g., drug fusions) can comprise a polypeptide or peptide drug. In preferred embodiments of drug fusions, the drug does not comprise an antibody chain or fragment of an antibody chain *(e.g.,* V_{H}, V*_{κ}*, V_{λ}). In specific embodiments, the drug is selected from an insulinotropic agent, and incretin, a glucagon-like 1 peptide, a GLP-1 peptide, a GLP-1 analogue, a GLP-1 derivative, PYY, a PYY peptide, a PYY analogue, a PYY derivative, Exendin-3, an Exendin-3 peptide, an Exendin-3 analogue, an Exendin-3 derivative, Exendin-4, an Exendin-4 peptide, an Exendin-4 analogue, an Exendin-4 derivative or a combination of two or more of these (eg, GLP-1 peptide and a PYY peptide).

Suitable drugs for use in the invention include, for example, immunosuppressive agents (e.g., cyclosporin A, rapamycin, FK506, prednisone), antiviral agents (acyclovir, ganciclovir, indinavir), antibiotics (penicillin, mynocyclin, tetracycline), anti-inflammatory agents (aspirin, ibuprofen, prednisone), cytotoxins or cytotoxic agents (e.g., paclitaxel, cytochalasin B, gramicidin D, ethidium bromide, emetine, mitomycin C, etoposide, tenoposide, vincristine, vinblastine, colchicine, doxorubicin, daunorubicin, dihydroxy anthracindione, mitoxantrone, mithramycin, actinomycin D, 1-dihydrotestosterone, glucocorticoids, procaine, tetracaine, lidocaine, propranolol, puromycin, and analogs or homologs of any of the foregoing agents. Suitable drugs also include antimetabolites (e.g., methotrexate, 6-mercaptopurine, 6-thioguanine, cytarabine, 5-fluorouracil decarbazine), alkylating agents (e.g., mechlorethamine, thioepachlorambucil, CC-1065, melphalan, carmustine (BSNU), lomustine (CCNU), cyclophosphamide, busulfan, dibromomannitol, streptozotocin, mitomycin C, and cis-dichlorodiamine platinum (II) (DDP) cisplatin), anthracyclines *(e.g.,* daunorubicin (formerly daunomycin) and doxorubicin), antibiotics *(e.g.,* dactinomycin (formerly actinomycin), bleomycin, mithramycin, and anthramycin (AMC)), radionuclides *(e.g.,* iodine-125, -126) yttrium (*e.g*., yttrium-90, -91) and praseodymium *(e.g.,* praseodymium-144, -145), and protease inhibitors *(e.g.,* inhibitors of matrix metalloproteinases). Other suitable drugs are nucleic acids such as antisense nucleic acids and RNAi. Calicheamicin is also suitable for use in the invention.

Suitable drugs also include analgesic agents, including narcotics *(e.g.,* codeine, nalmefene, naloxone, fentanyl, meperidine, morphine, tramadol, propoxyphene, oxycodone, methadone, nalbuphine), nonsteroidal anti-inflammatory agents *(e.g.,* indomethacin, ketorolac, arthrotec, ibuprofen, naproxen, salicylate, celecoxib, rofecoxib), acetaminophen, capsaicin, ziconotide and the like.

In certain embodiments, the drug is a polypeptide toxin, for example, a toxin such as abrin, ricin A, pseudomonas exotoxin, or diphtheria toxin. Other suitable polypeptide drugs include antibodies or antigen-binding fragments *(e.g.,* dAbs) of antibodies, polypeptide agonists, activators, secretagogues, antagonists or inhibitors. For example, the polypeptide or peptide drug can bind and agonise or antagonize a cell surface protein, such as a CD antigen, cytokine receptor *(e.g.,* interleukin receptor, chemokine receptor), adhesion molecule or costimulatory molecule. For example, the polypeptide drug can bind a cytokine, growth factors, cytokine receptor, growth factor receptor and other target ligand, which include but are not limited to: ApoE, Apo-SAA, BDNF, Cardiotrophin-1, CEA, CD40, CD40 Ligand, CD56, CD38, CD138, EGF, EGF receptor, ENA-78, Eotaxin, Eotaxin-2, Exodus-2, FAP*α*, FGF-acidic, FGF-basic, fibroblast growth factor-10, FLT3 ligand, Fractalkine (CX3C), GDNF, G-CSF, GM-CSF, GF-β1, human serum albumin, insulin, IFN-γ, IGF-I, IGF-II, IL-1α, IL-1β, IL-1 receptor, IL-2, IL-3, IL-4, IL-5, IL-6, IL-7, IL-8 (72 a.a.), IL-8 (77 a.a.), IL-9, IL-10, IL-11, IL-12, IL-13, IL-15, IL-16, IL-17, IL-18 (IGIF), Inhibin α, Inhibin β, IP-10, keratinocyte growth factor-2 (KGF-2), KGF, Leptin, LIF, Lymphotactin, Mullerian inhibitory substance, monocyte colony inhibitory factor, monocyte attractant protein, M-CSF, MDC (67 a.a.), MDC (69 a.a.), MCP-1 (MCAF), MCP-2, MCP-3, MCP-4, MDC (67 a.a.), MDC (69 a.a.), MIG, MIP-1α, MIP-1β, MIP-3α, MIP-3β, MIP-4, myeloid progenitor inhibitor factor-1 (MPIF-1), NAP-2, Neurturin, Nerve growth factor, β-NGF, NT-3, NT-4, Oncostatin M, PDGF-AA, PDGF-AB, PDGF-BB, PF-4, RANTES, SDF1α, SDF1β, SCF, SCGF, stem cell factor (SCF), TARC, TGF-α, TGF-β, TGF-β2, TGF-β3, tumour necrosis factor (TNF), TNF-α, TNF-β, TNF receptor I, TNF receptor II, TNIL-1, TPO, VEGF, VEGF A, VEGF B, VEGF C, VEGF D, VEGF receptor 1, VEGF receptor 2, VEGF receptor 3, GCP-2, GRO/MGSA, GRO-β, GRO-γ, HCC1, 1-309, HER 1, HER 2, HER 3 and HER 4. It will be appreciated that this list is by no means exhaustive.

Suitable drugs also include hormones, including pituitary hormone (PTH), adrenocorticotropic hormone (ACTH), renin, luteinizing hormone-releasing hormone (LHRH), gonadotropin-releasing hormone (GnRH), luteinizing hormone (LH), follicle stimulating hormone (FSH), aldosterone, and the like. Suitable drugs also include keratinocyte growth factor, interferons *(e.g.,* IFN-*α*, IFN-ß, IFN-γ), erythropoietin (EPO), proteases, elastases, LHRH analogs, agonists and antagonists, opioid receptor agonists, such as kappa opioid receptor agonists (*e.g*., dynorphin A), calcitonin and calcitonin analogs, antidiuretic hormone (vasopressin), oxytocin antagonists, vasoactive intestinal peptide, thrombin inhibitors, von Willebrand factor, surfactants and snail venom *(e.g.,* ziconotide).

Suitable drugs also include peptides and polypeptides that have anti-cancer activities *(e.g.,* proliferation inhibiting, growth inhibiting, apoptosis inducing, metastasis inhibiting, adhesion inhibiting, neovascularization inhibiting). Several such peptides and polypeptides are known in the art. (See. *e.g.,* Janin Y.L., Amino Acids, 25:1-40 (2003). The entire teaching of this reference, particularly the peptides and polypeptides disclosed therein, are incorporated herein by reference.) The amino acid sequences of several such peptides are presented in Table 8.

Other suitable drugs include peptides and polypeptides that have anti-viral activity. Several such peptides and polypeptides are known in the art, for example the peptides and polypeptides disclosed in Giannecchini, et al., J Viro., 77(6):3724-33 (2003); Wang, J., et al., Clin Chem (2003); Hilleman, M.R., Vaccine, 21(32):4626-49 (2003); Tziveleka, L.A., et al., Curr Top Med Chem, 3(13):1512-35 (2003); Poritz, M.A., et al., Virology, 313(1):170-83 (2003); Oevermann, A., et al., Antiviral Res, 59(1):23-33 (2003); Cole, A.M. et al., Curr Pharm Des, 9(18):1463-73 (2003); Pinon, J.D., et al., Virol, 77(5):3281-90 (2003); Sia, S.K., et al., Proc Natl Acad Sci USA, 99(23): 14664-9 (2002); Bahbouhi, B., et al., Biochem J, 66(Pt 3):863-72 (2002); de Soultrait, V.R., et al, J Mol Biol, 18(1):45-58 (2002); Witherell, G., Curr Opin Investig Drugs, 2(3):340-7 (2001); Ruff, M.R., et al., Antiviral Res, 52(1):63-75 (2001); Bultmann, H., et al., J. Virol, 75(6):2634-45 (2001); Egal, M., et al., Int JAntimicrob AGents, 13(1):57-60 (1999); and Robinson, W.E., Jr., JLeukoc Biol, 63(1):94-100(1998). The entire teachings of these references, particularly the peptides and polypeptides disclosed therein, are incorporated herein by reference. These peptides and polypeptides are examples of drugs that can be used in the compositions, drug fusions, drug conjugates, noncovalent drug conjugates of the present invention.

The polypeptide drug can also be a cytokine or growth factor or soluble portion of a receptor *(e.g.,* a cytokine receptor, growth factor receptor, hormone receptor) or other polypeptide such as the polypeptides listed above. For example, suitable polypeptide drugs also include receptor *(e.g.,* growth factor receptor, cytokine receptor, hormone receptor) agonists and antagonists, such as interleukin 1 receptor antagonist (Eisenberg et al., Nature 343:341-346 (1990)), thrombopoietin receptor agonists *(e.g.,* GW395058 (de Serres et al., Stem Cells 17:316-326 (1999)), melanocortin receptor antagonists *(e.g.,* MCR-4 antagonists (Cepoi et al., Brain Res. 1000:64-71 (2004)), anginex, 6DBF7 *(*Mayo et al., J. Biol. Chem. 278:45746-45752 (2003)), chemokine mimetics *(e.g.,* RANTES mimetics (Nardese et al., Nat. Struct. Biol. 8:611-615 (2001)), growth hormone *(e.g.,* human growth hormone), growth hormone analogs and growth hormone secretagogues (*e.g.,* CP-424,391 (MacAndrew et al., Eur. J. Pharmacol. 432:195-202 (2001)), growth hormone releasing hormone mimetics *(e.g.,* MK-677 (Chapman et al., J. Clin. Endocrinol. Metab. 82:3455-3463 (1997)), inhibitors of cellular adhesion molecule interactions (*e.g.*, LFA-1/ICAM-1, VLA-1/VCAM-1 (Yusuf-Makagiansar et al., Med. Res. Rev. 22:146-167 (2002)), mimetics of interferon *(e.g.,* SYR6 (Sato et al., Biochem. J. 371(Pt.2):603-608 (2003), mimetics ofherceptin *(*Nature Biotechnol. 18:137 (2000)), inhibitors of antigen presentation (Bolin et al., J. Med. Chem. 43:2135-2148 (2000)), GPIIB/IIIA antagonists (*e.g.,* FK633 (Aoki et al., Thromb. Res. 81:439-450 (1996)), alphavbeta3 antagonists *(e.g.,* SC56631 (Engleman et al., J. Clin. Invest. 99:2284-2292 (1997)), erythropoietin mimetics (*e.g*., EMP1 (Johnson et al., Biochemistry 37:3699-3710 (1998)), opioid receptor antagonists *(e.g.,* [(2S, 3R)-TMT1]DPDPE (Liao et al., J. Med. Chem. 41:4767-4776 (1998)), hematopoietic factors (e.g., erythropoietin (EPO), granulocyte colony stimulating factor (GM-CSF)).

Additional suitable peptide and polypeptide drugs include peptide antagonists that bind human type 1 IL-1 receptor (*e.g.*, AF 11377 (FEWTPGYWQPYALPL, SEQ ID NO:56), AF11869 (FEWTPGYWQJYALPL, SEQ ID NO:57 (J =1-azetidine-2-carboxylic acid), FEWTPGYWQJY (SEQ ID NO:58), FEWTPGWYQJY (SEQ ID NO:59), FEWTPGWYQJYALPL (SEQ ID NO:60), or any of the foregoing sequences optionally containing an acylated amino terminus and/or an aminated carboxyl terminus (Akeson et al., J. Biol. Chem. 271:30517-305123 (1996)), peptide antagonists of TNF-alpha-mediated cytotoxicity (e.g., those disclosed in Chirinos-Rojas et al, J. Immunol. 161:5621-5626 (1998)), peptide agonists of erythropoietin receptor (*e.g*., those disclosed in McConnel et al., Biol. Chem. 379:1279-1286 (1998) or Wrighton et al., Science 273:458-464 (1996)), glucagon-like peptide-1 (GLP-1, *e.g.,* GLP-1(7-37), GLP-1(7-36)amide and analogs thereof (see, *e.g.,* Ritzel U. et al., J. Endocrinology 159:93-102 (1998)), and interferons *(e.g.,* INF-α, INF-ß, INF-λ). Additional suitable polypeptide and peptide drugs include integrin inhibitors *(e.g.,* RGD peptides, such as H-Glu[cyclo(Arg-Gly-Asp-D-Phe-Lys)]₂ (Janssen, M.L., et al., Cancer Research 62:6146- 6151 (2002)), cyclo(Arg-Gly-Asp-D-Phe-Lys) (Kantlehner M., et al., Agnew. Chem. Int. Ed. 38:560 (1999)), cyclo(Arg-Gly-Asp-D-Tyr-Lys) (Haubner, R., et al., J. Nucl. Med. 42:326-336 (2001)), ribosome-inactivating proteins (RIPs) such as Saporin (*e.g*., SEQ ID NO:67), matrix metalloproteinase inhibitors *(e.g.,* U.S. Patent No. 5,616,605), and antiviral peptides and polypeptides, such as HIV fusion inhibitors (*e.g*.,T-1249 and T-20 (FUZEON® (enfuvirtide); Trimeris Inc.), and soluble receptor antagonists such as immunoadhesins *(e.g.,* LFA3-Ig, CTLA4-Ig).

Antimicrobial polypeptide and peptide drugs are also suitable for use in the invention. Examples of suitable antimicrobial polypeptide and peptide drugs include adenoregulin, dermcidin-1L, cathelicidins *(e.g.,* cathelicidin-like peptide, human LL-37/hCAP-18), defensins, including α-defensins (*e.g*., human neutrophil peptide 1 (HNP-1), HNP-2, HNP-3, HNP-4, human defensin 5, human defensin 6), ß-defensins (e.g., human ß-defensin-1, human ß-defensin-2), and θ-defensins *(e.g.,* θ-defensin-1), histatins *(e.g.,* histatin 1, histatin 3, histatin 5), lactoferricin-derived peptide and related peptides (see, Tomita M., et al., Acta Paediatr. Jpn. 36:585-591 (1994) and Strom, M.B., et al. Biochem Cell Biol. 80:65-74 (2002)).

In a preferred embodiment of the invention the drugs are insulinotropic drugs. Examples of suitable insulinotropic drugs include GLP-1, GLP-1 derivative, GLP-1 analogues or a derivative of a GLP-1 analogue. In addition they include Exedin-4, Exedin-4 analogues and Exedin-4 derivatives and Exedin-3, Exedin-3 derivatives and Exedin-3 analogues.

Other suitable drugs include Peptide YY (3-36) or analogues. Peptide YY (PYY) is a 36-residue peptide amide isolated originally from porcine intestine, and localized in the endocrine cells of the gastrointestinal tract and pancreas (Tatemoto, et al. Proc. Natl. Acad. Sci. 79:2514, 1982). Peptide YY has N-terminal and C-terminal tyrosine amides; accordingly, these two tyrosines give PYY its name (Y represents the amino acid tyrosine in the peptide nomenclature). In addition PYY shares a number of central and peripheral regulatory roles with its homologous peptide neuropeptide Y (NPY), which was originally isolated from porcine brain (Tatemoto, Proc. Natl. Acad. Sci. 79:5485, 1982). In contrast with the cellular location of PYY, NPY is present in submucous and myenteric neurons which innervate the mucosal and smooth muscle layers, respectively (Ekblad et al. Neuroscience 20:169, 1987). Both PYY and NPY are believed to inhibit gut motility and blood flow (Laburthe, Trends Endocrinol. Metab. 1: 168, 1990), and they are also thought to attenuate basal (Cox et al. Br. J. Pharmacol. 101:247, 1990) and secretagogue-induced intestinal secretion in rats *(*Lundberg et al. Proc. Natl. Acad. Sci USA 79:4471, 1982), as well as stimulate net absorption (MacFadyen et al. Neuropeptides 7:219, 1986). Taken together, these observations suggest that PYY and NPY are released into the circulation after a meal (Adrian et al. Gastroenterology 89:1070, 1985; Balasubramaniam et al. Neuropeptides 14:209, 1989), and thus play a physiological role in regulating intestinal secretion and absorption.

A high affinity PYY receptor system which exhibits a slightly higher affinity for PYY than NPY has been characterized in rat intestinal epithelia (Laburthe et al. Endocrinology 118:1910, 1986) and shown to be negatively coupled to adenylate cyclase (Servin et al. Endocrinology 124:692, 1989). Structure-activity studies using several partial sequences have led to the identification of PYY(22-36) as the active site for interacting with intestinal PYY receptors (Balsubramaniam et al. Pept. Res. 1:32, 1988).

In addition, PYY has been implicated in a number of physiological activities including nutrient uptake (Bilcheik et al. Digestive Disease Week 506:623, 1993), cell proliferation (Laburthe, Trends Endocrinol. Metab. 1: 168, 1990; Voisin et al. J. Biol. Chem, 1993), lipolysis *(*Valet et al., J. Clin. Invest. 291, 1990), and vasoconstriction (Lundberg et al., Proc. Natl. Acad. Sci., USA 79: 4471, 1982).

WO 03/057235 and WO 03/026591 disclose method for decreasing calorie intake, food intake and appetite by the administration of PYY or an agonist and GLP-1. These publications are incorporated herein by reference in their entirety, in particular to provide examples of PYY and GLP-1 drugs and methods that can be used in the present invention.

Further other drugs that are suitable for use in the invention include insulin, Resistin, Leptin, MC3R/MC4R antagonist, AgRP antagonist, Apolipoprotein A-IV, Enterostatin, Gastrin-Releasing Peptide (GRP), IGF1, BMP-9, IL-22, RegIV, interferon alfa, INGAP peptide, somatostatin, amylin, neurulin, interferon beta, interferon hybrids, adiponectin, endocannabinoids, C peptide, WNT10b, Orexin-A, adrenocorticotrophin, Enterostatin, Cholecystokinin; oxyntomodulin, Melanocyte Stimulating Hormones, melanocortin, Melanin concentrating hormone, BB-2, NPY Y2 agonists, NPY Y5/Y1 antagonists, OXM, Gal-1R antagonists, MCH-1R antagonists, MC-3/4 agonists, BRS-3 agonists, pancreatic polypeptide, anti-Ghrelin antibody fragment, brain-derived neurotrophic factor, human growth hormone, parathyroid hormone, follicle stimulating hormone, Gastric inhibitory peptide or an analogue thereof.

### Drug Fusions

The drug fusions of the invention are fusion proteins that comprise a continuous polypeptide chain, said chain comprising an antigen-binding fragment of an antibody that binds serum albumin as a first moiety, linked to a second moiety that is a polypeptide drug. The first and second moieties can be directly bonded to each other through a peptide bond, or linked through a suitable amino acid, or peptide or polypeptide linker. Additional moieties (*e.g*., third, fourth) and/or linker sequences can be present as appropriate. The first moiety can be in an N-terminal location, C-terminal location or internal relative to the second moiety *(i.e.,* the polypeptide drug). In certain embodiments, each moiety can be present in more than one copy. For example, the drug fusion can comprise two or more first moieties each comprising an antigen-binding fragment of an antibody that binds serum albumin *(e.g.,* a V_{H} that binds human serum albumin and a V_{L} that bind human serum albumin or two or more V_{H}s or V_{L}s that bind human serum albumin).

In some embodiments the drug fusion is a continuous polypeptide chain that has the formula:
a-(X)ₙ₁-b-(Y)ₙ₂-c-(Z)ₙ₃-d or a-(Z)ₙ₃-b-(Y)ₙ₂-c-(X)ₙ₁-d;
wherein X is a polypeptide drug that has binding specificity for a first target;
Y is a single chain antigen-binding fragment of an antibody that has binding specificity for serum albumin;
Z is a polypeptide drug that has binding specificity for a second target;
a, b, c and d are each independently absent or one to about 100 amino acid residues;
n1 is one to about 10;
n2 is one to about 10; and
n3 is zero to about 10,
with the proviso that when n1 and n2 are both one and n3 is zero, X does not comprise an antibody chain or a fragment of an antibody chain.

In one embodiment, neither X nor Z comprises an antibody chain or a fragment of an antibody chain. In one embodiment, n1 is one, n3 is one and n2 is two, three, four, five, six, seven, eight or nine. Preferably, Y is an immunoglobulin heavy chain variable domain (V_{H}) that has binding specificity for serum albumin, or an immunoglobulin light chain variable domain (V_{L}) that has binding specificity for serum albumin. More preferably, Y is a dAb *(e.g.,* a V_{H}, V*_{κ}* or V_{λ}) that binds human serum albumin. In a particular embodiment, X or Z is human GLP-1 or a GLP-1 derivatives or analogue thereof.

In certain embodiments, Y comprises an amino acid sequence selected from the group consisting of SEQ ID NO:10, SEQ ID NO:11, SEQ ID NO:12, SEQ ID NO:13, SEQ ID NO:14, SEQ ID NO:15, SEQ ID NO:24, SEQ ID NO:25 and SEQ ID NO:26. In other embodiments, Y comprises an amino acid sequence selected from the group consisting of SEQ ID NO:16, SEQ ID NO:17, SEQ ID NO:18, SEQ ID NO:19, SEQ ID NO:20, SEQ ID NO:21, SEQ ID NO:22 and SEQ ID NO:23.

In other embodiments, the drug fusion comprises moieties X' and Y', wherein X' is a polypeptide drug, with the proviso that X' does not comprise an antibody chain or a fragment of an antibody chain; and Y' is a single chain antigen-binding fragment of an antibody that has binding specificity for serum albumin. Preferably, Y' is an immunoglobulin heavy chain variable domain (V_{H}) that has binding specificity for serum albumin, or an immunoglobulin light chain variable domain (V_{L}) that has binding specificity for serum albumin. More preferably, Y' is a dAb *(e.g.,* a V_{H}, V*_{κ}* or V_{λ}) that binds human serum albumin. X' can be located amino terminally to Y', or Y' can be located amino terminally to X'. In some embodiments, X' and Y' are separated by an amino acid, or by a peptide or polypeptide linker that comprises from two to about 100 amino acids. In a particular embodiment, X' is human GLP-1 or GLP-1 derivative or analogues thereof.

In certain embodiments, Y' comprises an amino acid sequence selected from the group consisting of SEQ ID NO:10, SEQ ID NO:11, SEQ ID NO:12, SEQ ID NO:13, SEQ ID NO:14, SEQ ID NO:15, SEQ ID NO:24, SEQ ID NO:25 and SEQ ID NO:26. In other embodiments, Y' comprises an amino acid sequence selected from the group consisting of SEQ ID NO:16, SEQ ID NO:17, SEQ ID NO:18, SEQ ID NO:19, SEQ ID NO:20, SEQ ID NO:21, SEQ ID NO:22 and SEQ ID NO:23.

In particular embodiments the drug fusion comprises a dAb that binds serum albumin and human IL-1ra *(e.g.,* SEQ ID NO: 28). Preferably, the dAb binds human serum albumin and comprises human framework regions.

In other embodiments, the drug fusion or drug conjugate comprises a functional variant of human IL-1ra that has at least about 80%, or at least about 85%, or at least about 90%, or at least about 95%, or at least about 96%, or at least about 97%, or at least about 98%, or at least about 99% amino acid sequence identity with the mature 152 amino acid form of human IL-1ra and antagonizes human Interleukin-1 type 1 receptor. (See, Eisenberg et al., Nature 343:341-346 (1990).) The variant can comprise one or more additional amino acids *(e.g.,* comprise 153 or 154 or more amino acids). The drug fusions of the invention can be produced using any suitable method. For example, some embodiments can be produced by the insertion of a nucleic acid encoding the drug fusion into a suitable expression vector. The resulting construct is then introduced into a suitable host cell for expression. Upon expression, fusion protein can be isolated or purified from a cell lysate or preferably from the culture media or periplasm using any suitable method. (See *e.g.,* Current Protocols in Molecular Biology (Ausubel, F.M. et al., eds., Vol. 2, Suppl. 26, pp. 16.4.1-16.7.8 (1991)).

In a further embodiment the drug fusion or drug conjugate comprises an insulinotropic agent. In a preferred embodiment the drug fusion or drug conjugate comprises GLP-1, or an analogue or peptide of GLP-1. In a further preferred embodiment, the drug fusion or drug conjugate comprises Ser⁸GLP-1 (7-36) amide.

In a further embodiment, the drug fusion or drug conjugate comprises a GLP-1 analogue having one or more of the following substitutions: Val⁸ or Pro⁹.

Preferably, the GLP-1 analogue is Pro⁹GLP-1(7-36) or Pro⁹GLP-1(7-37). Further the GLP-1 analogue or peptide may include any one of the following C-terminal extensions: PSS, PSSGAP or PSSGAPPPS.

In another embodiment, the drug fusion or drug conjugate comprises a GLP-1 analogue comprising the sequence of Formula I

His⁷-Xaa⁸-Xaa⁹-Gly¹⁰-Xaa¹¹-Phe¹²-Thr¹³-Xaa¹⁴-Asp¹⁵-Xaa¹⁶-Xaa¹⁷-Xaa¹⁸-Xaa¹⁹-Xaa²⁰-Xaa²¹-Xaa²²-Xaa²³-Xaa²⁴-Xaa²⁵-Xaa²⁶-Xaa²⁷-Phe²⁸-Ile²⁹-Xaa³⁰-Xaa³¹-Xaa³²-Xaa³³-Xaa³⁴Xaa³⁵Xaa³⁶Xaa³⁷Xaa³⁸-Xaa³⁹-Xaa⁴⁰-Xaa⁴¹-Xaa⁴²-Xaa⁴³-Xaa⁴⁴-Xaa⁴⁵ Formula I - SEQ ID NO:171

wherein:
Xaa at position 8 is Ala, Gly, Ser, Thr, Leu, Ile, Val, Glu, Asp, or Lys;
Xaa at position 9 is Glu, or Asp;
Xaa at position 11 is Thr, Ala, Gly, Ser, Leu, Ile, Val, Glu, Asp, or Lys;
Xaa at position 14 is Ser, Ala, Gly, Thr, Leu, Ile, Val, Glu, Asp, or Lys;
Xaa at position 16 is Val, Ala, Gly, Ser, Thr,. Leu, Ile, Tyr, Glu, Asp, Trp, or Lys;
Xaa at position 17 is Ser, Ala, Gly, Thr, Leu, Ile, Val, Glu, Asp, or Lys;
Xaa at position 18 is Ser, Ala, Gly, Thr, Leu, Ile, Val, Glu, Asp, Trp, Tyr, or Lys;
Xaa at position 19 is Tyr, Phe, Trp, Glu, Asp, Gln, or Lys; Xaa at position 20 is Leu, Ala, Gly, Ser, Thr, Ile, Val, Glu, Asp, Met, Trp, Tyr, or Lys;
Xaa at position 21 is Glu, Asp, or Lys;
Xaa at position 22 is Gly, Ala, Ser, Thr, Leu, Ile, Val, Glu, Asp, or Lys;
Xaa at position 23 is Gln, Asn, Arg, Glu, Asp, or Lys;
Xaa at position 24 is Ala, Gly, Ser, Thr, Leu, Ile, Val, Arg, Glu, Asp, or Lys;
Xaa at position 25 is Ala, Gly, Ser, Thr, Leu, Ile, Val, Glu, Asp, or Lys;
Xaa at position 26 is Lys, Arg, Gln, Glu, Asp, or His;
Xaa at position 27 is Leu, Glu, Asp, or Lys;
Xaa at position 30 is Ala, Gly, Ser, Thr, Leu, Ile, Val, Glu, Asp, or Lys;
Xaa at position 31 is Trp, Phe, Tyr, Glu, Asp, or Lys;
Xaa at position 32 is Leu, Gly, Ala, Ser, Thr, Ile, Val, Glu, Asp, or Lys;
Xaa at position 33 is Val, Gly, Ala, Ser, Thr, Leu, Ile, Glu, Asp, or Lys;
Xaa at position 34 is Asn, Lys, Arg, Glu, Asp, or His;
Xaa at position 35 is Gly, Ala, Ser, Thr, Leu, Ile, Val, Glu, Asp, or Lys;
Xaa at position 36 is Gly, Arg, Lys, Glu, Asp, or His;
Xaa at position 37 is Pro, Gly, Ala, Ser, Thr, Leu, Ile, Val, Glu, Asp, or Lys, or is deleted;
Xaa at position 38 is Ser, Arg, Lys, Glu, Asp, or His, or is deleted;
Xaa at position 39 is Ser, Arg, Lys, Glu, Asp, or His, or is deleted;
Xaa at position 40 is Gly, Asp, Glu, or Lys, or is deleted; Xaa at position 41 is Ala, Phe, Trp, Tyr, Glu, Asp, or Lys, or is deleted;
Xaa at position 42 is Ser, Pro, Lys, Glu, or Asp, or is deleted;
Xaa at position 43 is Ser, Pro, Glu, Asp, or Lys, or is deleted;
Xaa at position 44 is Gly, Pro; Glu, Asp, or Lys, or is deleted;
and Xaa at position 45 is Ala, Ser, Val, Glu, Asp, or Lys, or is deleted;
provided that when the amino acid at position 37,38,39, 40,41,42,43, or 44 is deleted, then each amino acid downstream of that amino acid is also deleted.

In another embodiment the drug fusion or drug conjugate comprises a GLP-1 analogue that comprises the amino acid sequence of the Formula (II):

Xaa⁷-Xaa⁸-Glu-Gly-Thr-Phe-Thr-Ser-Asp-Xaa¹⁶-Ser-Xaa¹⁸-Xaa¹⁹-Xaa²⁰-Glu-Xaa²²-Xaa²³-Ala-Xaa²⁵-Xaa²⁶-Xaa²⁷-Phe-Ile-Xaa³⁰-Trp-Leu-Xaa³³-Xaa³⁴-Xaa³⁵-Xaa³⁶-Xaa³⁷-Xaa³⁸-Xaa³⁹-Xaa⁴⁰-Xaa⁴¹-Xaa⁴²-Xaa⁴³-Xaa⁴⁴-Xaa⁴⁵-Xaa⁴⁶ Formula (II) - SEQ ID NO:172

wherein Xaa7 is L-histidine, D-histidine, desamino-histidine, 2-amino-histidine, (3-hydroxy-histidine, homohistidine, N*α*-acetyl-histidine, *α*-fluoromethyl-histidine, *α-*methyl-histidine, 3- pyridylalanine, 2-pyridylalanine or 4-pyridylalanine ; Xaa8 is Ala, Gly, Val, Leu, Ile, Lys, Aib, (1-aminocyclopropyl) carboxylic acid, (1-aminocyclobutyl) carboxylic acid, (1-aminocyclopentyl) carboxylic acid, (1-aminocyclohexyl) carboxylic acid, (1-aminocycloheptyl) carboxylic acid, or (1-aminocyclooctyl) carboxylic acid;
Xaa¹⁶ is Val or Leu;
Xaa¹⁸ is Ser, Lys or Arg;
Xaa¹⁹ is Tyr or Gln ;
Xaa²⁰ is Leu or Met;
Xaa²² is Gly, Glu or Aib;
Xaa²³ is Gln, Glu, Lys or Arg;
Xaa²⁵ is Ala or Val;
Xaa²⁶ is Lys, Glu or Arg;
Xaa²⁷ is Glu or Leu;
Xaa³⁰ is Ala, Glu or Arg;
Xaa³³ is Val or Lys;
Xaa³⁴ is Lys, Glu, Asn or Arg;
Xaa³⁵ is Gly or Aib;
Xaa³⁶ is Arg, Gly or Lys;
Xaa³⁷ is Gly, Ala, Glu, Pro, Lys, amide or is absent;
Xaa³⁸ is Lys, Ser, amide or is absent.
Xaa³⁹ is Ser, Lys, amide or is absent;
Xaa⁴⁰ is Gly, amide or is absent;
Xaa⁴¹ is Ala, amide or is absent;
Xaa⁴² is Pro, amide or is absent;
Xaa⁴³ is Pro, amide or is absent;
Xaa⁴⁴ is Pro, amide or is absent;
Xaa⁴⁵ is Ser, amide or is absent;
Xaa⁴⁶ is amide or is absent; provided that if Xaa³⁸, Xaa³⁹, Xaa⁴⁰, Xaa⁴¹, Xaa⁴², Xaa⁴³, Xaa⁴⁴, Xaa⁴⁵ or Xaa⁴⁶ is absent then each amino acid residue downstream is also absent.

In another embodiment of the invention the drug fusion or drug conjugate comprises a GLP-1 peptide comprising the amino acid sequence of formula (III):

Xaa⁷-Xaa⁸-Glu-Gly-Thr-Phe-Thr-Ser-Asp-Val-Ser-Xaa¹⁸-Tyr-Leu-Glu-Xaa²²-Xaa²³-Ala-Ala-Xaa²⁶-Glu-Phe-Ile-Xaa³⁰-Trp-Leu-Val-Xaa³⁴-Xaa³⁵-Xaa³⁶-Xaa³⁷-Xaa³⁸ Formula (III) - SEQ ID NO:173

Wherein
Xaa⁷ is L-histidine, D-histidine, desamino-histidine, 2-amino-histidine, ß-hydroxy-histidine, homohistidine, N'1-acetyl-histidine, *α*-fluoromethyl-histidine, *α* -methyl-histidine, 3- pyridylalanine, 2-pyridylalanine or 4-pyridylalanine; Xaa⁸ is Ala, Gly, Val, Leu, Ile, Lys, *α*-aminoisobutyric acid (Aib), (1-aminocyclopropyl) carboxylic acid, (1- aminocyclobutyl) carboxylic acid, (1-aminocyclopentyl) carboxylic acid, (1-aminocyclohexyl) carboxylic acid, (1-aminocycloheptyl) carboxylic acid, or (1-aminocyclooctyl) carboxylic acid;
Xaa¹⁸ is Ser, Lys or Arg;
Xaa²² is Gly, Glu or Aib;
Xaa²³ is Gln, Glu, Lys or Arg;
Xaa²⁶ is Lys, Glu or Arg;
Xaa³⁰ is Ala, Glu or Arg;
Xaa³⁴ is Lys, Glu or Arg;
Xaa³⁵ is Gly or Aib;
Xaa³⁶ is Arg or Lys;
Xaa³⁷ is Gly, Ala, Glu or Lys;
Xaa³⁸ is Lys, amide or is absent.

In yet another embodiment of the invention the GLP-1 peptide is selected from the group consisting of: GLP-1 (7-35), GLP-1 (7-36), GLP-1 (7-36) -amide, GLP-1 (7-37), GLP-1 (7-38), GLP-1 (7-39), GLP-1 (7- 40), GLP-1 (7-41) or an analogue or peptide thereof.

In another embodiment of the invention the GLP-1 peptide is GLP-1 (A-B) wherein A is an integer from 1 to 7 and B is an integer from 37 to 45 or an analogue thereof comprising one albumin binding residue attached via a hydrophilic spacer to the C-terminal amino acid residue and, optionally, a second albumin binding residue attached to one of the other amino acid residues.

In another embodiment the GLP-1 peptide comprises no more than fifteen amino acid residues which have been exchanged, added or deleted as compared to GLP-1 (7-37) or no more than ten amino acid residues which have been exchanged, added or deleted as compared to GLP-1 (7-37).

In another embodiment the GLP-1 peptide comprises no more than six (preferably, no more than 5, 4, 3, 2 or 1) amino acid residues which have been exchanged, added or deleted as compared to GLP-1 (7-37).

In another embodiment the GLP-1 peptide comprises no more than 4 preferably, no more than 3, 2 or 1) amino acid residues which are not encoded by the genetic code.

In another embodiment the GLP-1 peptide is a DPPIV protected GLP-1 peptide.

In another embodiment the insulinotropic agent is DPPIV stabilised.

In another embodiment the GLP-1 peptide comprises an α-aminoisobutyric acid (Aib) residue in position 8.

In another embodiment the amino acid residue in position 7 of said GLP-1 peptide is selected from the group consisting of D-histidine, desamino-histidine, 2-amino-histidine, ß-hydroxy-histidine, homohistidine, Nα-acetyl-histidine, α-fluoromethyl-histidine, α-methyl-histidine, 3-pyridylalanine, 2-pyridylalanine and 4-pyridylalanine.

In another embodiment the GLP-1 peptide is selected from the group consisting of: Arg³⁴GLP-1 (7-37), Arg^{26,34}Lys³⁸GLP-1(7-38), Arg^{26,34}Lys³⁸GLP-1 (7-38)-OH, Lys³⁶Arg^{26,34}GLP-1 (7-36), Aib^{8,22,35} GLP-1 (7-37), Aib^{8,35} GLP-1 (7-37), Aib^{8,22} GLP-1 (7-37), Aib^{8,22,35}Arg^{26,34}Lys³⁸GLP-1 (7-38), Aib^{8,35}Arg^{26,34}Lys³⁸GLP-1(7-38), Aib^{8,22}Arg^{26,34}Lys³⁸GLP-1 (7-38), Aib^{8,22,35}Arg^{26,34}Lys³⁸GLP-1 (7-38), Aib^{8,35}Arg^{26,34}Lys³⁸GLP-1 (7-38), Aib^{8,22,35}Arg²⁶Lys³⁸GLP-1(7-38), Aib^{8,35}Arg²⁶Lys³⁸GLP-1(7-38), Aib^{8,22}Arg²⁶Lys³⁸GLP-1 (7-38), Aib^{8,22}, ³⁵Arg³⁴Lys³⁸GLP-1 (7-38), Aib^{8,35}Arg³⁴Lys³⁸GLP-1 (7-38), Aib^{8,22}Arg³⁴Lys³⁸GLP-1 (7-38), Aib^{8,22,35}Ala³⁷Lys³⁸GLP-1 (7-38), Aib^{8,35}Ala³⁷Lys³⁸GLP-1 (7-38), Aib^{8,22}Ala³⁷Lys³⁸GLP-1 (7-38), Aib^{8,22,35}Lys³⁷GLP-1 (7-37), Aib^{8,35}Lys³⁷GLP-1 (7-37), Aib⁸Arg^{26,34}Glu^{22,23,30}Lys³⁸GLP-1 (7-38), Gly⁸Arg^{26,34}Lys³⁶GLP-1(7-37), Aib⁸Arg^{26,34}Lys³⁸GLP-1 (7-38), Aib⁸Lys³⁸GLP-1 (7-38), Gly⁸Arg^{26,34}Lys³⁸GLP-1(7-38), GLP-1 (7-37)amide, GLP-1 (7-37) amide, Aib⁸Arg^{26,34}Lys³⁶GLP-1(7-37), Arg^{26,34}Lys³⁶GLP-1(7-37), Gly⁸Arg^{26,34}Lys³⁶GLP-1(7-37), Aib^{8,35}Lys³⁷GLP-1 (7-37)-OH, Ala⁸Arg^{26,34}Lys³⁸GLP-1(7-38), Aib^{8,22,35}Lys³⁸GLP-1 (7-38), Aib⁸Arg^{26,34}Lys³⁶GLP-1 (7-36), Gly⁸Arg^{26,34}Lys³⁶GLP-1(7-37)-OH, Aib^{8,22,35}Lys³⁷GLP-1(7-37)-NH₂, Aib⁸Arg³⁴GLP-1(7-37)-OH, Gly⁸Arg^{26,34}Lys³⁸GLP-1(7-38), Arg³⁴GLP-1(7-37)-OH, Gly⁸Glu^{22,23,30}Arg^{18,26,34}Lys³⁸GLP-1(7-38), imidazolylpropionic acid⁷Asp¹⁸Aib^{22,35}Lys³⁸GLP-1(7-38), imidazolylpropionic acid⁷ Aib^{22,35}Lys³⁸GLP-1(7-38), [3-(5-Imidazoyl)propionyl⁷Aib⁸Arg^{26,34}Lys³⁸GLP-1(7-38), and Aib^{8,22}Lys³⁷GLP-1 (7-38).

In another embodiment the GLP-1 peptide is attached to a hydrophilic spacer via the amino acid residue in position 23, 26, 34, 36 or 38 of the native GLP-1 or GLP-1 analogue.

In another embodiment the insulinotropic agent is Lys²⁰exendin-4(1-39)-NH₂.

In another embodiment the GLP-1 peptide is HGEGTFTSDLSKQMEEEAVRLFIEWLKNGGPSSGAPPSKKKKKK-amide - SEQ ID NO:174.

In another embodiment the GLP-1 peptide is HGEGTFTSDLSKQMEEEAVRLFIEWLKNGGX - SEQ ID NO:175
wherein X = P or Y, or a fragment or an analogue thereof.

In another embodiment of the invention the GLP-1 peptide is Arg¹⁸, Leu²⁰, Gln³⁴, Lys³³ (N∈-(γ-aminobutyroyl(Nα-hexadecanoyl))) Exendin-4-(7-45)-amide or Arg³³, Leu²⁰, Gln³⁴,Lys¹⁸ (N∈-(γ-aminobutyroyl(Nα-hexadecanoyl))) Exendin-4-(7-45)-amide. Examples of insulinotropic agents which can be useful as GLP-1 analogues or derivatives or GLP-1 like drugs according to the present invention are described in International Patent Application No. WO 87/06941 (The General Hospital Corporation) which relates to a peptide fragment which comprises GLP-1 (7-37) and functional derivatives thereof and to its use as an insulinotropic agent (incorporated herein by reference, particularly by way of examples of drugs for use in the present invention).

Further GLP-1 analogues are described in International Patent Application No. 90/11296 (The General Hospital Corporation) which relates to peptide fragments which comprise GLP-1 (7-36) and functional derivatives thereof and have an insulinotropic activity which exceeds the insulinotropic activity of GLP-1 (1-36) or GLP-1 (1-37) and to their use as insulinotropic agents (incorporated herein by reference, particularly by way of examples of drugs for use in the present invention).

International Patent Application No. WO 91/11457 (Buckley et al..) discloses analogues of the active GLP-1 peptides 7-34,7-35, 7-36, and 7-37 which can also be useful as GLP-1 drugs according to the present invention (incorporated herein by reference, particularly by way of examples of drugs for use in the present invention).

Further Exendin-analogs that are useful for the present invention are described in PCT patent publications WO 99/25728 (Beeley et al.), WO 99/25727 Beeley et al.), WO 98/05351 (Young et al.), WO 99/40788 (Young et al.), WO 99/07404 (Beeley et al), and WO 99/43708 (Knudsen et al) (all incorporated herein by reference, particularly by way of examples of drugs for use in the present invention).

Suitable expression vectors can contain a number of components, for example, an origin of replication, a selectable marker gene, one or more expression control elements, such as a transcription control element (*e.g.,* promoter, enhancer, terminator) and/or one or more translation signals, a signal sequence or leader sequence, and the like. Expression control elements and a signal sequence, if present, can be provided by the vector or other source. For example, the transcriptional and/or translational control sequences of a cloned nucleic acid encoding an antibody chain can be used to direct expression.

A promoter can be provided for expression in a desired host cell. Promoters can be constitutive or inducible. For example, a promoter can be operably linked to a nucleic acid encoding an antibody, antibody chain or portion thereof, such that it directs transcription of the nucleic acid. A variety of suitable promoters for procaryotic (*e.g.,* lac, tac, T3, T7 promoters for *E*. *coli*) and eucaryotic (*e.g.,* simian virus 40 early or late promoter, Rous sarcoma virus long terminal repeat promoter, cytomegalovirus promoter, adenovirus late promoter) hosts are available.

In addition, expression vectors typically comprise a selectable marker for selection of host cells carrying the vector, and, in the case of a replicable expression vector, an origin or replication. Genes encoding products which confer antibiotic or drug resistance are common selectable markers and may be used in procaryotic (*e.g.,* lactamase gene (ampicillin resistance), *Tet* gene for tetracycline resistance) and eucaryotic cells (*e.g.,* neomycin (G418 or geneticin), gpt (mycophenolic acid), ampicillin, or hygromycin resistance genes). Dihydrofolate reductase marker genes permit selection with methotrexate in a variety of hosts. Genes encoding the gene product of auxotrophic markers of the host (*e.g., LEU2, URA3, HIS3*) are often used as selectable markers in yeast. Use of viral (*e.g.,* baculovirus) or phage vectors, and vectors which are capable of integrating into the genome of the host cell, such as retroviral vectors, are also contemplated. Suitable expression vectors for expression in mammalian cells and prokaryotic cells (*E. coli*), insect cells (Drosophila Schnieder S2 cells, Sf9) and yeast *(P. methanolica, P. pastoris, S. cerevisiae*) are well-known in the art.

Recombinant host cells that express a drug fusion and a method of preparing a drug fusion as described herein are provided. The recombinant host cell comprises a recombinant nucleic acid encoding a drug fusion. Drug fusions can be produced by the expression of a recombinant nucleic acid encoding the protein in a suitable host cell, or using other suitable methods. For example, the expression constructs described herein can be introduced into a suitable host cell, and the resulting cell can be maintained (*e.g.,* in culture, in an animal) under conditions suitable for expression of the constructs. Suitable host cells can be prokaryotic, including bacterial cells such as *E*. *coli, B. subtilis* and or other suitable bacteria, eucaryotic, such as fungal or yeast cells (*e.g., Pichia pastoris, Aspergillus species, Saccharomyces cerevisiae, Schizosaccharomyces pombe, Neurospora crassa*), or other lower eucaryotic cells, and cells of higher eucaryotes such as those from insects (*e.g*., Sf9 insect cells (WO 94/26087 (O'Connor)) or mammals (*e.g.*, COS cells, such as COS-1 (ATCC Accession No. CRL-1650) and COS-7 (ATCC Accession No. CRL-1651), CHO *(e.g.,* ATCC Accession No. CRL-9096), 293 (ATCC Accession No. CRL-1573), HeLa (ATCC Accession No. CCL-2), CV1 (ATCC Accession No. CCL-70), WOP (Dailey et al., J. Virol. 54:739-749 (1985)), 3T3, 293T *(*Pear et al., Proc. Natl. Acad. Sci. U.S.A., 90:8392-8396 (1993)), NSO cells, SP2/0, HuT 78 cells, and the like (see, *e.g.,* Ausubel, F.M. et al., eds. Current Protocols in Molecular Biology, Greene Publishing Associates and John Wiley & Sons Inc., (1993)).

The invention also includes a method of producing a drug fusion, comprising maintaining a recombinant host cell of the invention under conditions appropriate for expression of a drug fusion. The method can further comprise the step of isolating or recovering the drug fusion, if desired. In another embodiment, the components of the drug fusion (*e.g.,* dAb that binds human serum albumin and IL-1ra) are chemically assembled to create a continuous polypeptide chain.

### Conjugates

In another aspect, the invention provides conjugates comprising an antigen-binding fragment of an antibody that binds serum albumin that is bonded to a drug. Such conjugates include "drug conjugates," which comprise an antigen-binding fragment of an antibody that binds serum albumin to which a drug is covalently bonded, and "noncovlaent drug conjugates," which comprise an antigen-binding fragment of an antibody that binds serum albumin to which a drug is noncovalently bonded. Preferably, the conjugates are sufficiently stable so that the antigen-binding fragment of an antibody that binds serum albumin and drug remain substantially bonded (either covalently or noncovalently) to each other under *in vivo* conditions (*e.g.,* when administered to a human). Preferably, no more than about 20%, no more than about 15%, no more than about 10%, no more than about 9%, no more than about 8%, no more than about 7%, no more than about 6%, no more than about 5%, no more than about 4%, no more than about 3%, no more than about 2%, no more than about 1% or substantially none of the conjugates dissociate or break down to release drug and antigen-binding fragment under *in vivo* conditions. For example, stability under "*in vivo*" conditions can be conveniently assessed by incubating drug conjugate or noncovalent drug conjugate for 24 hours in serum (*e.g.,* human serum) at 37°C. In one example of such a method, equal amounts of a drug conjugate and the unconjugated drug are diluted into two different vials of serum. Half of the contents of each vial is immediately frozen at -20°C, and the other half incubated for 24 hours at 37°C. All four samples can then be analyzed using any suitable method, such as SDS-PAGE and/or Western blotting. Western blots can be probed using an antibody that binds the drug. All drugs in the drug conjugate lanes will run at the size of the drug conjugate if there was no dissociation. Many other suitable methods can be used to assess stability under *"in vivo"* conditions, for example, by analyzing samples prepared as described above using suitable analytic methods, such as chromatography (*e.g.,* gel filtration, ion exchange, and reverse phase), ELISA, mass spectroscopy and the like.

### Drug Conjugates

In another aspect, the invention provides a drug conjugate comprising an antigen-binding fragment of an antibody that has binding specificity for serum albumin, and a drug that is covalently bonded to said antigen-binding fragment, with the proviso that the drug conjugate is not a single continuous polypeptide chain.

In some embodiments, the drug conjugate comprises an immunoglobulin heavy chain variable domain (V_{H}) that has binding specificity for serum albumin, or an immunoglobulin light chain variable domain (V_{L}) that has binding specificity for serum albumin, and a drug that is covalently bonded to said V_{H} or V_{L}, with the proviso that the drug conjugate is not a single continuous polypeptide chain. Preferably the drug conjugate comprises a single V_{H} that binds serum albumin or a single V_{L} that binds serum albumin. In certain embodiments, the drug conjugate comprises a Vₖ dAb that binds human serum albumin and comprises an amino acid sequence selected from the group consisting of SEQ ID NO:10, SEQ ID NO:11, SEQ ID NO:12, SEQ ID NO:13, SEQ ID NO:14, SEQ ID NO:15, SEQ ID NO:24, SEQ ID NO:25 and SEQ ID NO:26. In other embodiments, the drug conjugate comprises a V_{H} dAb that binds human serum albumin and comprises an amino acid sequence selected from the group consisting of SEQ ID NO:16, SEQ ID NO:17, SEQ ID NO:18, SEQ ID NO:19, SEQ ID NO:20, SEQ ID NO:21, SEQ ID NO:22 and SEQ ID NO:23.

The drug conjugates can comprise any desired drug and can be prepared using any suitable methods. For example, the drug can be bonded to the antigen-binding fragment of an antibody that binds serum albumin directly or indirectly through a suitable linker moiety at one or more positions, such as the amino-terminus, the carboxyl-terminus or through amino acid side chains. In one embodiment, the drug conjugate comprises a dAb that binds human serum albumin and a polypeptide drug (*e.g.,* human IL-1ra or a functional variant of human IL-1ra), and the amino-terminus of the polypeptide drug (*e.g.,* human IL-1ra or a functional variant of human IL-1ra) is bonded to the carboxyl-terminus of the dAb directly or through a suitable linker moiety. In another embodiment, the drug conjugate comprises a dAb that binds human serum albumin and an insulinotropic drug (*e.g.,* GLP-1or a GLP-1 analogue) and the amino-terminus of the insulinotropic drug is free (i.e. not coupled or bonded in the conjugate) and the carboxyl terminus is bonded to the amino-terminus of the dAb directly or through a suitable linker moiety. In other embodiments, the drug conjugate comprises a dAb that binds human serum albumin and two or more different drugs that are covalently bonded to the dAb. For example, a first drug can be covalently bonded (directly or indirectly) to the carboxyl terminus of the dAb and a second drug can be covalently bonded (directly or indirectly) to the amino-terminus or through a side chain amino group (*e.g.*, ∈ amino group of lysine). In a preferred embodiment the amino-terminus of the insulinotropic drug (eg. GLP-1 or a GLP-1 analogue) is free. Such drug conjugates can be prepared using well-known methods of selective coupling. (See, *e.g.,* Hermanson, G. T., Bioconjugate Techniques, Academic Press: San Diego, CA (1996).)

A variety of methods for conjugating drugs to an antigen-binding fragment of an antibody that has binding specificity for serum albumin can be used. The particular method selected will depend on the drug to be conjugated. If desired, linkers that contain terminal functional groups can be used to link the antigen-binding fragment and the drug. Generally, conjugation is accomplished by reacting a drug that contains a reactive functional group (or is modified to contain a reactive functional group) with a linker or directly with an antigen-binding fragment of an antibody that binds serum albumin. Covalent bonds form by reacting a drug that contains (or is modified to contain) a chemical moiety or functional group that can, under appropriate conditions, react with a second chemical group thereby forming a covalent bond. If desired, a suitable reactive chemical group can be added to the antigen-binding fragment or to a linker using any suitable method. (See, *e.g.,* Hermanson, G. T., Bioconjugate Techniques, Academic Press: San Diego, CA (1996).) Many suitable reactive chemical group combinations are known in the art, for example an amine group can react with an electrophilic group such as tosylate, mesylate, halo (chloro, bromo, fluoro, iodo), N-hydroxysuccinimidyl ester (NHS), and the like. Thiols can react with maleimide, iodoacetyl, acrylolyl, pyridyl disulfides, 5-thiol-2-nitrobenzoic acid thiol (TNB-thiol), and the like. An aldehyde functional group can be coupled to amine- or hydrazide-containing molecules, and an azide group can react with a trivalent phosphorous group to form phosphoramidate or phosphorimide linkages. Suitable methods to introduce activating groups into molecules are known in the art (see for example, Hermanson, G. T., Bioconjugate Techniques, Academic Press: San Diego, CA (1996)).

In some embodiments, the antigen-binding fragment of an antibody that has binding specificity for serum albumin is bonded to a drug by reaction of two thiols to form a disulfide bond. In other embodiments, the antigen-binding fragment of an antibody that has binding specificity for serum albumin is bonded to a drug by reaction of an isothiocyanate group and a primary amine to produce an isothiourea bond.

Suitable linker moieties can be linear or branched and include, for example, tetraethylene glycol, C₂-C₁₂ alkylene, -NH-(CH₂)ₚ-NH- or -(CH₂)ₚ-NH- (wherein p is one to twelve), -CH₂-O-CH₂-CH₂-O-CH₂-CH₂-O-CH-NH-, a polypeptide chain comprising one to about 100 (preferably one to about 12) amino acids and the like.

### Noncovalent Drug Conjugates

Some noncovalent bonds (*e.g.,* hydrogen bonds, van der Waals interactions) can produce stable, highly specific intermolecular connections. For example, molecular recognition interactions achieved through multiple noncovalent bonds between complementary binding partners underlie many important biological interactions, such as the binding of enzymes to their substrates, the recognition of antigens by antibodies, the binding of ligands to their receptors, and stabilization of the three dimensional structure of proteins and peptide. Accordingly, such weak noncovalent interactions (*e.g.,* hydrogen bonding, van Der Waals interactions, electrostatic interactions, hydrophobic interactions and the like) can be utilized to bind a drug to the antigen-binding fragment of an antibody that has binding specificity for serum albumin.

Preferably, the noncovalent bond linking the antigen-binding fragment and drug be of sufficient strength that the antigen-binding fragment and drug remain substantially bonded to each under *in vivo* conditions (*e.g.,* when administered to a human). Generally, the noncovalent bond linking the antigen-binding fragment and drug has a strength of at least about 10¹⁰ M⁻¹. In preferred embodiments, the strength of the noncovalent bond is at least about 10¹¹ M⁻¹, at least about 10¹² M⁻¹, at least about 10¹³ M⁻¹, at least about 10¹⁴ M⁻¹ or at least about 10¹⁵ M⁻¹. The interactions between biotin and avidin and between biotin and streptavidin are known to be very efficient and stable under many conditions, and as described herein noncovalent bonds between biotin and avidin or between biotin and streptavidin can be used to prepare a noncovalent drug conjugate of the invention.

The noncovalent bond can be formed directly between the antigen-binding fragment of an antibody that has a specificity for serum albumin and drug, or can be formed between suitable complementary binding partners (*e.g.*, biotin and avidin or streptavidin) wherein one partner is covalently bonded to drug and the complementary binding partner is covalently bonded to the antigen-binding fragment. When complementary binding partners are employed, one of the binding partners can be covalently bonded to the drug directly or through a suitable linker moiety, and the complementary binding partner can be covalently bonded to the antigen-binding fragement of an antibody that binds serum albumin directly or through a suitable linker moiety.

Complementary binding partners are pairs of molecules that selectively bind to each other. Many complementary binding partners are known in the art, for example, antibody (or an antigen-binding fragment thereof) and its cognate antigen or epitope, enzymes and their substrates, and receptors and their ligands. Preferred complementary binding partners are biotin and avidin, and biotin and streptavidin.

Direct or indirect covalent bonding of a member of a complementary binding pair to an antigen-binding fragment that has binding specificity for serum albumin or a drug can be accomplished as described above, for example, by reacting a complementary binding partner that contains a reactive functional group (or is modified to contain a reactive functional group) with an antigen-binding fragment of an antibody that binds serum albumin, with or without the use of a linker. The particular method selected will depend on the compounds (*e.g.*, drug, complementary binding partner, antigen-binding fragment of an antibody that binds serum albumin) to be conjugated. If desired, linkers (*e.g.,* homobifunctional linkers, heterobifunctional linkers) that contain terminal reactive functional groups can be used to link the antigen-binding fragment and/or the drug to a complementary binding partner. In one embodiment, a heterobifunctional linker that contains two distinct reactive moieties can be used. The heterobifunctional linker can be selected so that one of the reactive moieties will react with the antigen-binding fragment of an antibody that has binding specificity for serum albumin or the drug, and the other reactive moiety will react with the complementary binding partner. Any suitable linker (*e.g.,* heterobifunctional linker) can be used and many such linkers are known in the art and available for commercial sources (*e.g.,* Pierce Biotechnology, Inc., IL).

### Compositions and Therapeutic and Diagnostic Methods

Compositions comprising drug compositions of the invention (*e.g.,* drug conjugates, noncovalent drug conjugates, drug fusions), including pharmaceutical or physiological compositions (*e.g.*, for human and/or veterinary administration) are provided. Pharmaceutical or physiological compositions comprise one or more drug compositions (*e.g.,* drug conjugate, noncovalent drug conjugate, drug fusion), and a pharmaceutically or physiologically acceptable carrier. Typically, these carriers include aqueous or alcoholic/aqueous solutions, emulsions or suspensions, including saline and/or buffered media. Parenteral vehicles include sodium chloride solution, Ringer's dextrose, dextrose and sodium chloride and lactated Ringer's. Suitable physiologically-acceptable adjuvants, if necessary to keep a polypeptide complex in suspension, may be chosen from thickeners such as carboxymethylcellulose, polyvinylpyrrolidone, gelatin and alginates. Intravenous vehicles include fluid and nutrient replenishers and electrolyte replenishers, such as those based on Ringer's dextrose. Preservatives and other additives, such as antimicrobials, antioxidants, chelating agents and inert gases, may also be present (Mack (1982) Remington's Pharmaceutical Sciences, 16th Edition).

The compositions can comprise a desired amount of drug composition (*e.g.,* drug conjugate, noncovalent drug conjugate, drug fusion). For example the compositions can comprise about 5% to about 99% drug conjugate, noncovalent drug conjugate or drug fusion by weight. In particular embodiments, the composition can comprise about 10% to about 99%, or about 20% to about 99%, or about 30% to about 99% or about 40% to about 99%, or about 50% to about 99%, or about 60% to about 99%, or about 70% to about 99%, or about 80% to about 99%, or about 90% to about 99%, or about 95% to about 99% drug composition (*e.g.,* drug conjugate, noncovalent drug conjugate, drug fusion), by weight. In one example, the composition is freeze dried (lyophilized).

The drug compositions (*e.g.,* drug conjugates, noncovalent drug conjugates, drug fusions), described herein will typically find use in preventing, suppressing or treating inflammatory states (*e.g.,* acute and/or chronic inflammatory diseases), such as chronic obstructive pulmonary disease (*e.g.*, chronic bronchitis, chronic obstructive bronchitis, emphysema), allergic hypersensitivity, cancer, bacterial or viral infection, pneumonia, such as bacterial pneumonia (*e.g.,* Staphylococcal pneumonia)), autoimmune disorders (which include, but are not limited to, Type I diabetes, multiple sclerosis, arthritis (*e.g.,* osteoarthritis, rheumatoid arthritis, juvenile rheumatoid arthritis, psoriatic arthritis, lupus arthritis, spondylarthropathy (*e.g.,* ankylosing spondylitis)), systemic lupus erythematosus, inflammatory bowel disease (*e.g.,* Crohn's disease, ulcerative colitis), Behcet's syndrome and myasthenia gravis), endometriosis, psoriasis, abdominal adhesions (*e.g.,* post abdominal surgery), asthma, and septic shock. The drug compositions (*e.g.,* drug conjugates, noncovalent drug conjugates, drug fusions), described herein can be used for preventing, suppressing or treating pain, such as chronic or acute traumatic pain, chronic or acute neuropathic pain, acute or chronic musculoskeletal pain, chronic or acute cancer pain and the like. The drug compositions (*e.g.,* drug conjugates, noncovalent drug conjugates, drug fusions), described herein can also be administered for diagnostic purposes.

Cancers that can be prevented, suppressed or treated using the drug compositions (*e.g.,* drug conjugates, noncovalent drug conjugates, drug fusions), described herein include lymphomas (*e.g.,* B cell lymphoma, acute myeloid lymphoma, Hodgkin's lymphoma, non-Hodgkin's lymphoma), myelomas (*e.g.,* multiple myeloma), lung cancer (*e.g.,* small cell lung carcinoma, non-small cell lung carcinoma), colorectal cancer, head and neck cancer, pancreatic cancer, liver cancer, stomach cancer, breast cancer, ovarian cancer, bladder cancer, leukemias (*e.g.,* acute myelogenous leukemia, chronic myelogenous leukemia, acute lymphocytic leukemia, chronic lymphocytic leukemia), adenocarcinomas, renal cancer, haematopoetic cancers (*e.g*., myelodysplastic syndrome, myeloproliferative disorder (*e.g.,* polycythemia vera, essential (or primary) thrombocythemia, idiopathic myelofibrosis), and the like.

The drug compositions (*e.g.,* drug conjugates, noncovalent drug conjugates, drug fusions) described herein are also suitable for use in preventing, suppressing or treating endometriosis, fibrosis, infertility, premature labour, erectile dysfunction, osteoporosis, diabetes (*e.g.,* type II diabetes), growth disorder, HIV infection, respiratory distress syndrome, tumours and bedwetting.

In a preferred embodiment the present invention relates to the use of a compound according to the invention for the preparation of a medicament for the treatment of hyperglycemia, type 2 diabetes, impaired glucose tolerance, type 1 diabetes, obesity, hypertension, syndrome X, dyslipidemia, (ß-cell apoptosis, j3-ce) i deficiency, myocardial infarction, inflammatory bowel syndrome, dyspepsia, cognitive disorders, e. g. cognitive enhancing, neuroprotection, atherosclerosis, coronary heart disease and other cardiovascular disorders. In specific embodiments for these indications, the drug is selected from an insulinotropic agent, and incretin, a glucagon-like 1 peptide, a GLP-1 peptide, a GLP-1 analogue, a GLP-1 derivative, PYY, a PYY peptide, a PYY analogue, a PYY derivative, Exendin-3, an Exendin-3 peptide, an Exendin-3 analogue, an Exendin-3 derivative, Exendin-4, an Exendin-4 peptide, an Exendin-4 analogue, an Exendin-4 derivative or a combination of two or more of these (eg, GLP-1 peptide and a PYY peptide).

In another embodiment the present invention relates to the use of a compound according to the invention for the preparation of a medicament for the treatment of small bowel syndrome, inflammatory bowel syndrome or Crohns disease. In specific embodiments for these indications, the drug is selected from an insulinotropic agent, and incretin, a glucagon-like 1 peptide, a GLP-1 peptide, a GLP-1 analogue, a GLP-1 derivative, PYY, a PYY peptide, a PYY analogue, a PYY derivative, Exendin-3, an Exendin-3 peptide, an Exendin-3 analogue, an Exendin-3 derivative, Exendin-4, an Exendin-4 peptide, an Exendin-4 analogue, an Exendin-4 derivative or a combination of two or more of these (eg, GLP-1 peptide and a PYY peptide).

In another embodiment the present invention relates to the use of a compound according to the invention for the preparation of a medicament for the treatment of hyperglycemia, type 1 diabetes, type 2 diabetes or ß-cell deficiency. In specific embodiments for these indications, the drug is selected from an insulinotropic agent, and incretin, a glucagon-like 1 peptide, a GLP-1 peptide, a GLP-1 analogue, a GLP-1 derivative, PYY, a PYY peptide, a PYY analogue, a PYY derivative, Exendin-3, an Exendin-3 peptide, an Exendin-3 analogue, an Exendin-3 derivative, Exendin-4, an Exendin-4 peptide, an Exendin-4 analogue, an Exendin-4 derivative or a combination of two or more of these (eg, GLP-1 peptide and a PYY peptide).

The treatment with a compound according to the present invention may also be combined with a second or more pharmacologically active substances which may or may not be part of the drug conjugate or fusion. For example, an active selected from antidiabetic agents, antiobesity agents, appetite regulating agents, antihypertensive agents, agents for the treatment and/or prevention of complications resulting from or associated with diabetes and agents for the treatment and/or prevention of complications and disorders resulting from or associated with obesity. In the present context the expression "antidiabetic agent" includes compounds for the treatment and/or prophylaxis of insulin resistance and diseases wherein insulin resistance is the pathophysiological mechanism.

Examples of these pharmacologically active substances are: Insulin, GLP-1 agonists, sulphonylureas (e. g. tolbutamide, glibenclamide, glipizide and gliclazide), biguanides e. g. metformin, meglitinides, glucosidase inhibitors (e. g. acorbose), glucagon antagonists, DPP-IV (dipeptidyl peptidase-IV) inhibitors, inhibitors of hepatic enzymes involved in stimulation of gluconeogenesis and/or glycogenolysis, glucose uptake modulators, thiazolidinediones such as troglitazone and ciglitazone, compounds modifying the lipid metabolism such as antihyperlipidemic agents as HMG CoA inhibitors (statins), compounds lowering food intake, RXR agonists and agents acting on the ATP-dependent potassium channel of the (ß-cells, e. g. glibenclamide, glipizide, gliclazide and repaglinide; Cholestyramine, colestipol, clofibrate, gemfibrozil, lovastatin, pravastatin, simvastatin, probucol, dextrothyroxine, neteglinide, repaglinide ; (ß-blockers such as alprenolol, atenolol, timolol, pindolol, propranolol and metoprolol, ACE (angiotensin converting enzyme) inhibitors such as benazepril, captopril, enalapril, fosinopril, lisinopril, alatriopril, quinapril and ramipril, calcium channel blockers such as nifedipine, felodipine, nicardipine, isradipine, nimodipine, diltiazem and verapamil, and a-blockers such as doxazosin, urapidil, prazosin and terazosin; CART (cocaine amphetamine regulated transcript) agonists, NPY (neuropeptide Y) antagonists, MC4 (melanocortin 4) agonists, orexin antagonists, TNF (tumor necrosis factor) agonists, CRF (corticotropin releasing factor) agonists, CRF BP (corticotropin releasing factor binding protein) antagonists, urocortin agonists, ß3 agonists, MSH (melanocyte-stimulating hormone) agonists, MCH (melanocyte-concentrating hormone) antagonists, CCK (cholecystokinin) agonists, serotonin re-uptake inhibitors, serotonin and noradrenaline re-uptake inhibitors, mixed serotonin and noradrenergic compounds, 5HT (serotonin) agonists, bombesin agonists, galanin antagonists, growth hormone, growth hormone releasing compounds, TRH (thyreotropin releasing hormone) agonists, UCP 2 or 3 (uncoupling protein 2 or 3) modulators, leptin agonists, DA agonists (bromocriptin, doprexin), lipase/amylase inhibitors, RXR (retinoid X receptor) modulators, TR ß agonists; histamine H3 antagonists.

Further insulin can be in the form of one of the following analogues: AspB28-human insulin, LysB28, ProB29-human insulin, LysB3 GluB29-human insulin, GlyA21, ArgB31, ArgB32-human insulin and des (B30) human insulin.

Further other active drugs include, human growth hormone or an analogue thereof, parathyroid hormone or an analogue thereof, a growth factor such as platelet-derived growth factor (PDGF), transforming growth factor α (TGF-α), transforming growth factor-ß (TGF-ß), epidermal growth factor (EGF), vascular endothelial growth factor (VEGF), a somatomedin such as insulin growth factor I (IGF-I), insulin growth factor 11 (IFG-II), erythropoietin (EPO), thrombopoietin (TPO) or angiopoietin, interferon, prourokinase, urokinase, tissue plasminogen activator (t-PA), plasminogen activator inhibitor 1, plasminogen activator inhibitor 2, von Willebrandt factor, a cytokine, e. g. an interleukin such as interleukin (IL) 1, IL-1 Ra, IL-2, IL-4, IL-5, IL-6, IL-9, IL-11, IL-12, IL-13, IL-15, IL-16, IL-17, IL-18, IL-20 or IL-21, a colony stimulating factor (CFS) such as GM-CSF, stem cell factor, a tumor necrosis factor such as TNF- α, lymphotoxin-α, Iymphotoxin-ß, CD40L, or CD30L, a protease inhibitor e. g. aprotinin, human follicle stimulating hormone or an analogue thereof, an enzyme such as superoxide dismutase, asparaginase, arginase, arginine deaminase, adenosine deaminase, ribonuclease, catalase, uricase, bilirubin oxidase, trypsin, papain, alkaline phosphatase, (3-glucoronidase, purine nucleoside phosphorylase or batroxobin, an opioid, e. g. endorphins, enkephalins or non-natural opioids, a hormone or neuropeptide, e. g. calcitonin, glucagon, gastrins, adrenocorticotropic hormone (ACTH), cholecystokinins, lutenizing hormone, gonadotropin-releassing hormone, chorionic gonadotropin, corticotrophin-releasing factor, vasopressin, oxytocin, antidiuretic hormones, thyroid-stimulating hormone, thyrotropin- releasing hormone, relaxin, prolactin, peptide YY, neuropeptide Y, pancreastic polypeptide, leptin, CART (cocaine and amphetamine regulated transcript), a CART related peptide, perilipin, melanocortins (melanocyte-stimulating hormones) such as MC-4, melanin-concentrating hormones, natriuretic peptides, adrenomedullin, endothelin, secretin, amylin, vasoactive intestinal peptide (VIP), pituary adenylate cyclase activating polypeptide (PACAP), bombesin, bombesin-like peptides, thymosin, heparin-binding protein, soluble CD4, hypothalmic releasing factor, melanotonins and analogues thereof.

The drug conjugate or drug fusion described herein can also be administered for diagnostic purposes or as an imaging agent.

In the instant application, the term "prevention" involves administration of the protective composition prior to the induction of the disease. "Suppression" refers to administration of the composition after an inductive event, but prior to the clinical appearance of the disease. "Treatment" involves administration of the protective composition after disease symptoms become manifest.

Animal model systems which can be used to screen the effectiveness of drug compositions (e.g., drug conjugates, noncovalent drug conjugates, drug fusions) in protecting against or treating the disease are available. Methods for the testing of systemic lupus erythematosus (SLE) in susceptible mice are known in the art (Knight et al. (1978) J. Exp. Med., 147: 1653; Reinersten et al. (1978) New Eng. J. Med., 299: 515). Myasthenia Gravis (MG) is tested in SJL/J female mice by inducing the disease with soluble AchR protein from another species (Lindstrom et al. (1988) Adv. Immunol., 42: 233). Arthritis is induced in a susceptible strain of mice by injection of Type II collagen (Stuart et al. (1984) Ann. Rev. Immunol., 42: 233). A model by which adjuvant arthritis is induced in susceptible rats by injection of mycobacterial heat shock protein has been described (Van Eden et al. (1988) Nature, 331: 171). Effectiveness for treating osteoarthritis can be assessed in a murine model in which arthritis is induced by intra-articular injection of collagenase (Blom, A.B. et al., Osteoarthritis Cartilage 12:627-635 (2004). Thyroiditis is induced in mice by administration of thyroglobulin as described (Maron et al. (1980) J. Exp. Med., 152: 1115). Insulin dependent diabetes mellitus (IDDM) occurs naturally or can be induced in certain strains of mice such as those described by Kanasawa et al. (1984) Diabetologia, 27: 113. EAE in mouse and rat serves as a model for MS in human. In this model, the demyelinating disease is induced by administration of myelin basic protein (see Paterson (1986) Textbook of Immunopathology, Mischer et al., eds., Grune and Stratton, New York, pp. 179-213; McFarlin et al. (1973) Science, 179: 478: and Satoh et al. (1987) J. Immunol., 138: 179).

The drug compositions (*e.g.,* drug conjugates, noncovalent drug conjugates, drug fusions) of the present invention may be used as separately administered compositions or in conjunction with other agents. These can include various immunotherapeutic drugs, such as cylcosporine, methotrexate, adriamycin or cisplatinum, immunotoxins and the like. Pharmaceutical compositions can include "cocktails" of various cytotoxic or other agents in conjunction with the drug composition (*e.g.,* drug conjugate, noncovalent drug conjugate, drug fusion) of the present invention, or combinations of drug compositions (*e.g.,* drug conjugates, noncovalent drug conjugates, drug fusions) according to the present invention comprising different drugs.

The drug compositions (*e.g.,* drug conjugates, noncovalent drug conjugates, drug fusions) can be administered to any individual or subject in accordance with any suitable techniques. A variety of routes of administration are possible including, for example, oral, dietary, topical, transdermal, rectal, parenteral (*e.g.,* intravenous, intraarterial, intramuscular, subcutaneous, intradermal, intraperitoneal, intrathecal, intraarticular injection), and inhalation (*e.g.,* intrabronchial, intranasal or oral inhalation, intranasal drops) routes of administration, depending on the drug composition and disease or condition to be treated. Administration can be local or systemic as indicated. The preferred mode of administration can vary depending upon the drug composition (*e.g.,* drug conjugate, noncovalent drug conjugate, drug fusion) chosen, and the condition (*e.g.,* disease) being treated. The dosage and frequency of administration will depend on the age, sex and condition of the patient, concurrent administration of other drugs, counterindications and other parameters to be taken into account by the clinician. A therapeutically effective amount of a drug composition (*e.g.,* drug conjugate, noncovalent drug conjugate, drug fusion) is administered. A therapeutically effective amount is an amount sufficient to achieve the desired therapeutic effect, under the conditions of administration.

In a preferred embodiment of the invention pharmaceutical compositions containing a GLP-1 drug or GLP-1 analogue or derivative according to the present invention may be administered parenterally to patients in need of such a treatment. Parenteral administration may be performed by subcutaneous, intramuscular or intravenous injection by means of a syringe, optionally a pen-like syringe. Alternatively, parenteral administration can be performed by means of an infusion pump. A further option is a composition which may be a powder or a liquid for the administration of the GLP-1 drug or GLP-1 analogue or derivative in the form of a nasal or pulmonal spray. As a still further option, the GLP-1 drug or GLP-1 analogue or derivative of the invention can also be administered transdermally, e. g. from a patch, optionally an iontophoretic patch, or transmucosally, e. g. bucally. In other embodiments the compositions are administered orally, eg as a pill, capsule, drink (eg, marketed as a weight-loss drink for obesity treatment).

A composition for parenteral administration of GLP-1 compounds may, for example, be prepared as described in WO 03/002136 (incorporated herein by reference).

A composition for nasal administration of certain peptides may, for example, be prepared as described in European Patent No. 272097 (to Novo Nordisk A/S) or in WO 93/18785 (all incorporated herein by reference).

The term "subject" or "individual" is defined herein to include animals such as mammals, including, but not limited to, primates (*e.g.,* humans), cows, sheep, goats, horses, dogs, cats, rabbits, guinea pigs, rats, mice or other bovine, ovine, equine, canine, feline, rodent or murine species.

The drug composition (*e.g.,* drug conjugate, noncovalent drug conjugate, drug fusion) can be administered as a neutral compound or as a salt. Salts of compounds (*e.g.,* drug compositions, drug conjugates, noncovalent drug conjugates, drug fusions) containing an amine or other basic group can be obtained, for example, by reacting with a suitable organic or inorganic acid, such as hydrogen chloride, hydrogen bromide, acetic acid, perchloric acid and the like. Compounds with a quaternary ammonium group also contain a counteranion such as chloride, bromide, iodide, acetate, perchlorate and the like. Salts of compounds containing a carboxylic acid or other acidic functional group can be prepared by reacting with a suitable base, for example, a hydroxide base. Salts of acidic functional groups contain a countercation such as sodium, potassium and the like.

The invention also provides a kit for use in administering a drug composition *(e.g.,* drug conjugate, noncovalent drug conjugate, drug fusion) to a subject (*e.g.,* patient), comprising a drug composition (*e.g.,* drug conjugate, noncovalent drug conjugate, drug fusion), a drug delivery device and, optionally, instructions for use. The drug composition (*e.g.,* drug conjugate, noncovalent drug conjugate, drug fusion) can be provided as a formulation, such as a freeze dried formulation. In certain embodiments, the drug delivery device is selected from the group consisting of a syringe, an inhaler, an intranasal or ocular administration device (*e.g.,* a mister, eye or nose dropper), and a needleless injection device.

The drug composition (*e.g.,* drug conjugate, noncovalent drug conjugate, drug fusion) of this invention can be lyophilized for storage and reconstituted in a suitable carrier prior to use. Any suitable lyophilization method (*e.g.,* spray drying, cake drying) and/or reconstitution techniques can be employed. It will be appreciated by those skilled in the art that lyophilisation and reconstitution can lead to varying degrees of antibody activity loss (*e.g.,* with conventional immunoglobulins, IgM antibodies tend to have greater activity loss than IgG antibodies) and that use levels may have to be adjusted to compensate. In a particular embodiment, the invention provides a composition comprising a lyophilized (freeze dried) drug composition (*e.g.,* drug conjugate, noncovalent drug conjugate, drug fusion) as described herein. Preferably, the lyophilized (freeze dried) drug composition (*e.g.,* drug conjugate, noncovalent drug conjugate, drug fusion) loses no more than about 20%, or no more than about 25%, or no more than about 30%, or no more than about 35%, or no more than about 40%, or no more than about 45%, or no more than about 50% of its activity (*e.g.,* binding activity for serum albumin) when rehydrated. Activity is the amount of drug composition (*e.g.,* drug conjugate, noncovalent drug conjugate, drug fusion) required to produce the effect of the drug composition before it was lyophilized. For example, the amount of drug conjugate or drug fusion needed to achieve and maintain a desired serum concentration for a desired period of time. The activity of the drug composition (*e.g.,* drug conjugate, noncovalent drug conjugate, drug fusion) can be determined using any suitable method before lyophilization, and the activity can be determined using the same method after rehydration to determine amount of lost activity.

Compositions containing the drug composition (*e.g.,* drug conjugate, noncovalent drug conjugate, drug fusion) or a cocktail thereof can be administered for prophylactic and/or therapeutic treatments. In certain therapeutic applications, an amount sufficient to achieve the desired therapeutic or prophylactic effect, under the conditions of administration, such as at least partial inhibition, suppression, modulation, killing, or some other measurable parameter, of a population of selected cells is defined as a "therapeutically-effective amount or dose." Amounts needed to achieve this dosage will depend upon the severity of the disease and the general state of the patient's own immune system and general health, but generally range from about 10 µg/kg to about 80 mg/kg, or about 0.005 to 5.0 mg of drug conjugate or drug fusion per kilogram of body weight, with doses of 0.05 to 2.0 mg/kg/dose being more commonly used. For example, a drug composition (e.g., drug fusion, drug conjugate, noncovalent drug conjugate) of the invention can be administered daily (*e.g.,* up to four administrations per day), every two days, every three days, twice weekly, once weekly, once every two weeks, once a month, or once every two months, at a dose of, for example, about 10 µg/kg to about 80 mg/kg, about 100 µg/kg to about 80 mg/kg, about 1 mg/kg to about 80 mg/kg, about 1 mg/kg to about 70 mg/kg, about 1 mg/kg to about 60 mg/kg, about 1 mg/kg to about 50 mg/kg, about 1 mg/kg to about 40 mg/kg, about 1 mg/kg to about 30 mg/kg, about 1 mg/kg to about 20 mg/kg, about 1 mg/kg to about 10 mg/kg, about 10 µg/kg to about 10 mg/kg, about 10 µg/kg to about 5 mg/kg, about 10 µg/kg to about 2.5 mg/kg, about 1 mg/kg, about 2 mg/kg, about 3 mg/kg, about 4 mg/kg, about 5 mg/kg, about 6 mg/kg, about 7 mg/kg, about 8 mg/kg, about 9 mg/kg or about 10 mg/kg. For prophylactic applications, compositions containing the drug composition (e.g., drug conjugate, noncovalent drug conjugate, drug fusion) or cocktails thereof may also be administered in similar or slightly lower dosages. A composition containing a drug composition (*e.g.,* drug conjugate, noncovalent drug conjugate, drug fusion) according to the present invention may be utilised in prophylactic and therapeutic settings to aid in the alteration, inactivation, killing or removal of a select target cell population in a mammal.

### EXAMPLES

Interleukin 1 receptor antagonist (IL1-ra) is an antagonist that blocks the biologic activity of IL-1 by competitively inhibiting IL-1 binding to the interleukin-1 type 1 receptor (IL-1R1). IL-1 production is induced in response to inflammatory stimuli and mediates various physiologic responses including inflammatory and immunological responses. IL-1 has a range of activities including cartilage degredation and stimulation of bone resorption. In rheumatoid arthritis patients, the amount of locally produed IL-1 is elevated and the levels of naturally occurring IL1-ra are insufficient to compete with these abnormally increased amounts. There are several treatments avavilable for RA including disease modifying antirheumatic drugs (DMARDS) such as methotrexate, and biologics such as KINERET® (anakinra, Amgen).

KINERET® (anakinra, Amgen) is a recombinant, nonglycosylated form of the human interleukin-1 receptor antagonist which consists of 153 amino acids and has a molecular weight of 17.3 kilodaltons. (The amino acid sequence of KINERET® (anakinra, Amgen) corresponds to the 152 amino acids in naturally occurring IL-1ra and an additional N-terminal methionine.) KINERET® (anakinra, Amgen) is indicated for the reduction in signs and symptoms of moderate to severe rheumatoid arthritis in patients 18 years of age or older who have failed one or more DMARDs. Dosage is a single use daily subcutaneous injection of 100mgs of drug. The T_{*β*½} is 4-6 hours and 71 % of patients develop injection site reactions in 14-28 days.

Here we demonstrate that linking a therapeutic polypeptide to a serum-albumin binding dAb results in a compound which (i) has activity similar to the therapeutic polypeptide alone and (ii) also binds serum albumin. Furthermore, the present invention provides a method to create a long serum half-life version of the therapeutic polypeptide. For example, we have linked a serum albumin binding dAb to IL1-ra which results in a compound of longer serum half life than IL1-ra alone.

### Example 1 Selection of domain antibodies that bind mouse, rat and human serum albumin

This example explains a method for making a single domain antibody (dAb) directed against serum albumin. Selection of dAbs against mouse serum albumin (MSA), human serum albumin (HSA) and rat serum albumin (RSA) is described.

The dAbs against mouse serum albumin were selected as described in WO 2004/003019 A2. Three human phage display antibody libraries were used. Each library was based on a single human framework for V_{H} (V3-23/DP47 and J_{H}4b) or V*ₖ* (o12/o2/DPK9 and Jₖ1) with side chain diversity encoded by NNK codons incorporated in complementarity determining regions (CDR1, CDR2 and CDR3).

### Library 1 (V_{H}):

Diversity at positions: H30, H31, H33, H35, H50, H52, H52a, H53, H55, H56, H58, H95, H97, H98.
Library size: 6.2 x 10⁹

### Library 2 (V_{H}):

Diversity at positions: H30, H31, H33, H35, H50, H52, H52a, H53, H55, H56, H58, H95, H97, H98, H99, H100, H100A, H100B.
Library size: 4.3 x 10⁹

### Library 3 (Vκ):

Diversity at positions: L30, L31, L32, L34, L50, L53, L91, L92, L93, L94, L96
Library size: 2 x 10⁹

The V_{H} and V*κ* libraries had been preselected for binding to generic ligands protein A and protein L respectively so that the majority of clones in the selected libraries were functional. The sizes of the libraries shown above correspond to the sizes after preselection.

Two rounds of selection were performed on serum albumin using each of the libraries separately. For each selection, antigen was coated on immunotube (nunc) in 4 mL of PBS at a concentration of 100 *µ*g/ml. In the first round of selection, each of the three libraries was panned separately against HSA (Sigma) or MSA (Sigma). In the second round of selection, phage from each of the six first round selections was panned against (i) the same antigen again (eg 1st round MSA, 2nd round MSA) and (ii) against the reciprocal antigen (eg 1^{st} round MSA, 2nd round HSA) resulting in a total of twelve 2nd round selections. In each case, after the second round of selection 48 clones were tested for binding to HSA and MSA. Soluble dAb fragments were produced as described for scFv fragments by Harrison et al, Methods Enzymol. 1996; 267: 83-109 and standard ELISA protocol was followed (Hoogenboom et al. (1991) Nucleic Acids Res., 19: 4133) except that 2% tween PBS was used as a blocking buffer and bound dAbs were detected with either protein L-HRP (Sigma) (for the V*κ*S) and protein A-HRP (Amersham Pharmacia Biotech) (for the V_{H}s).

dAbs that gave a signal above background indicating binding to MSA, HSA or both were tested in ELISA insoluble form for binding to plastic alone but all were specific for serum albumin. Clones were then sequenced (see Table 1) revealing that 21 unique dAb sequences had been identified. The minimum similarity (at the amino acid level) between the V*κ* dAb clones selected was 86.25% ((69/80) X100; the result when all the diversified residues are different, e.g., clones 24 and 34). The minimum similarity between the V_{H} dAb clones selected was 94 % ((127/136) X100).

Next, the serum albumin binding dAbs were tested for their ability to capture biotinylated antigen from solution. ELISA protocol (as above) was followed except that ELISA plate was coated with 1 *µ*g/ml protein L (for the V*κ* clones) and 1 *µ*g/ml protein A (for the V_{H} clones). Soluble dAb was captured from solution as in the protocol and detection was with biotinylated MSA or HSA and streptavidin HRP. The biotinylated MSA and HSA had been prepared according to the manufacturer's instructions, with the aim of achieving an average of 2 biotins per serum albumin molecule. Twenty four clones were identified that captured biotinylated MSA from solution in the ELISA. Two of these (clones 2 and 38 below) also captured biotinylated HSA. Next, the dAbs were tested for their ability to bind MSA coated on a CM5 Biacore chip. Eight clones were found that bound MSA on the Biacore.

dAbs against human serum albumin and rat serum albumin were selected as previously described for the anti-MSA dAbs except for the following modifications to the protocol: The phage library of synthetic V_{H} domains was the libray 4G, which is based on a human V_{H}3 comprising the DP47 germline gene and the J_{H}4 segment. The diversity at the following specific positions was introduced by mutagenesis (using NNK codons; numbering according to Kabat) in CDR1: 30, 31, 33, 35; in CDR2: 50, 52, 52a, 53, 55, 56; and in CDR3: 4-12 diversified residues: *e.g.* H95, H96, H97, and H98 in 4G H11 and H95, H96, H97, H98, H99, H100, H100a, H100b, H100c, H100d, H100e and H100f in 4G H19. The last three CDR3 residues are FDY so CDR3 lengths vary from 7-15 residues. The library comprises >1x10¹⁰ individual clones.

A subset of the V_{H} and V*κ* libraries had been preselected for binding to generic ligands protein A and protein L respectively so that the majority of clones in the unselected libraries were functional. The sizes of the libraries shown above correspond to the sizes after preselection.

Two rounds of selection were performed on rat and human serum albumin using subsets of the V_{H} and V*κ* libraries separately. For each selection, antigen was either (i) coated on immunotube (nunc) in 4ml of PBS at a concentration of 100*µ*g/ml, or (ii) bitotinylated and then used for soluble selection followed by capture on streptavidin beads (in the 1^{st} round) and neutravidin beads (in the 2^{nd} round). (See Table 1 for details of the selection strategy used to isolate each clone.) In each case, after the second round of selection 24 phage clones were tested for binding to HSA or RSA.

If a significant proportion of the clones in one of the selections were positive in the phage ELISA, then DNA from this selection was cloned into an expression vector for production of soluble dAb, and individual colonies were picked. Soluble dAb fragments were produced as described for scFv fragments by Harrison et al (Methods Enzymol. 1996;267:83-109) and standard ELISA protocol was followed (Hoogenboom et al. (1991) Nucleic Acids Res., 19: 4133) except that 2% TWEEN PBS was used as a blocking buffer and bound dAbs were detected with anti-myc-HRP . Clones that were positive in ELISA were then screened for binding to MSA, RSA or HSA using a BIACORE surface plasmon resonance instrument (Biacore AB). dAbs which bound to MSA, RSA or HSA were further analysed. Clones were then sequenced and unique dAb sequences identified.

**Table 1. Selection protocols for dAbs that bind serum albumin**

| dAb | Library | R1 selection | R2 selection | Biacore binding |
|---|---|---|---|---|
| DOM7r-1 | 4G V*κ* | 10*µ*g/ml tube RSA | 10*µ*g/ml tube RSA | RSA |
| DOM7r-3 | 4G V*κ* | 10*µ*g/ml tube RSA | 10*µ*g/ml tube RSA | RSA |
| DOM7r-4 | 4G V*κ* | 10*µ*g/ml tube RSA | 10*µ*g/ml tube RSA | RSA, MSA |
| DOM7r-5 | 4G V*κ* | 10*µ*g/ml tube RSA | 10*µ*g/ml tube RSA | RSA |
| DOM7r-7 | 4G V*κ* | 10*µ*g/ml tube RSA | 10*µ*g/ml tube RSA | RSA, MSA |
| DOM7r-8 | 4G V*κ* | 10*µ*g/ml tube RSA | 10*µ*g/ml tube RSA | RSA, MSA |
| DOM7h-1 | 4G V*κ* | 10*µ*g/ml tube HSA | 10*µ*g/ml tube HSA | HSA |
| DOM7h-2 | 4G V*κ* | Soluble 100nM HSA | Soluble 50nM HSA | HSA |
| DOM7h-3 | 4G V*κ* | 10*µ*g/ml tube HSA | 10*µ*g/ml tube HSA | - |
| DOM7h-4 | 4G V*κ* | 10*µ*g/ml tube HSA | 10*µ*g/ml tube HSA | - |
| DOM7h-6 | 4G V*κ* | | | |
| DOM7h-7 | 4G V*κ* | | | |
| DOM7h-8 | 4G V*κ* | Soluble 200nM HSA | Soluble 50nM RSA | HSA, RSA, MSA |
| DOM7r-13 | 4G V*κ* | Soluble 200nM HSA | Soluble 50nM RSA | RSA, MSA |
| DOM7r-14 | 4G V*κ* | Soluble 200nM HSA | Soluble 50nM RSA | RSA, MSA |
| DOM7h-21 | 4G VH | 100*µ*g/ml HSA tube | 100 *µ*g/ml HSA tube | HSA |
| DOM7h-22 | 4G VH | 100*µ*g/ml HSA tube | 100*µ*g/ml HSA tube | HSA |
| DOM7h-23 | 4G VH | 100*µ*g/ml HSA tube | 100*µ*g/ml HSA tube | HSA |
| DOM7h-24 | 4G VH | 100*µ*g/ml HSA tube | 100*µ*g/ml HSA tube | HSA |
| DOM7h-25 | 4G VH | 100*µ*g/ml HSA tube | 100*µ*g/ml HSA tube | HSA |
| DOM7h-26 | 4G VH | 100*µ*g/ml HSA tube | 100*µ*g/ml HSA tube | HSA |
| DOM7h-27 | 4G VH | 100*µ*g/ml HSA tube | 100*µ*g/ml HSA tube | HSA |

dAbs that bound serum albumin on a BIACORE chip (Biacore AB) were then further analysed to obtain information on affinity. The analysis was performed using a CM5 chip (carboxymethylated dextran matix) that was coated with serum albumin. Flow cell 1 was an uncoated, blocked negative control, flow cell 2 was coated with HSA, flow cell 3 was coated with RSA and flow cell 4 was coated with MSA. The serum albumins were immobilised in acetate buffer pH 5.5 using the BIACORE coating wizard which was programmed to aim for 500 resonance units (RUs) of coated material. Each dAb of interest was expressed in the periplasm of *E. coli* on a 200 mL-500 mL scale and purified from the supernatant using batch absorption to protein A-streamline affinity resin (Amersham, UK) for the V_{H}s and to protein L-agarose affinity resin (Affitech, Norway) for the V*_{κ}*s followed by elution with glycine at pH 2.2 and buffer exchange to PBS. A range of concentrations of dAb were prepared (in the range 5nM to 5*µ*M) by dilution into BIACORE HBS-EP buffer and flowed across the BIACORE chip.

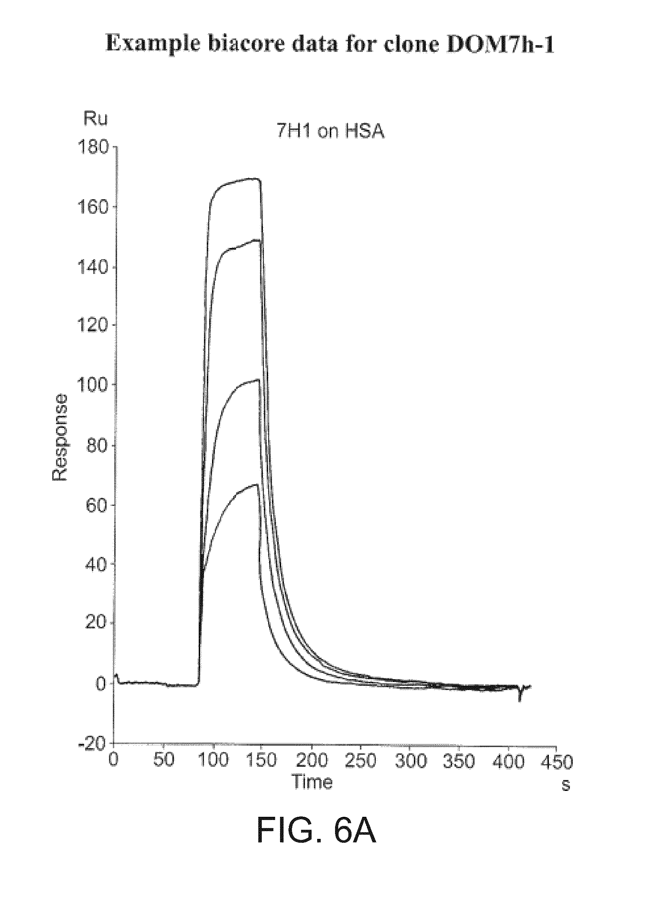

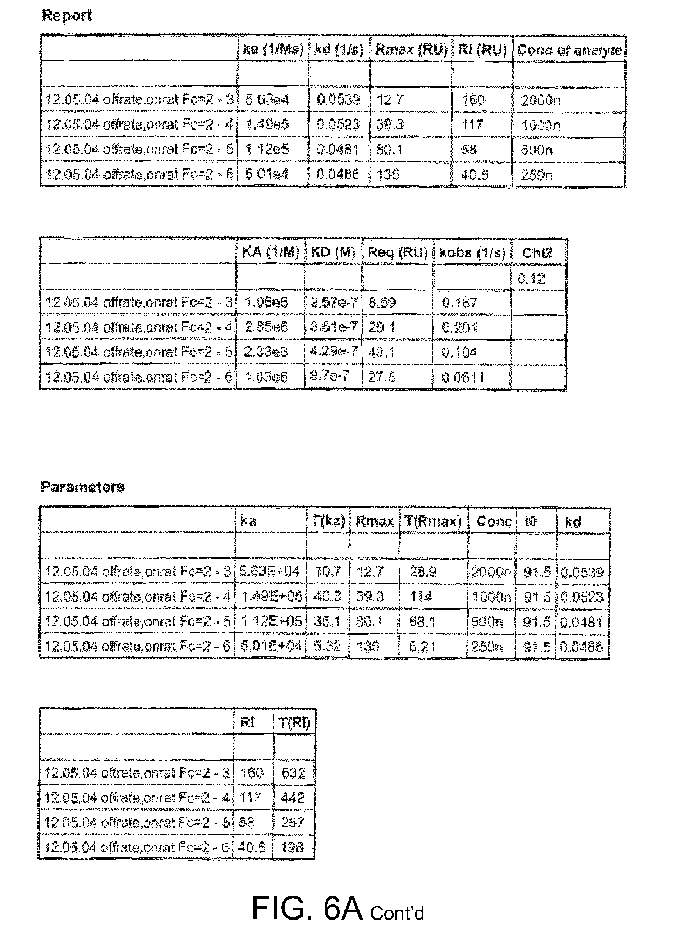

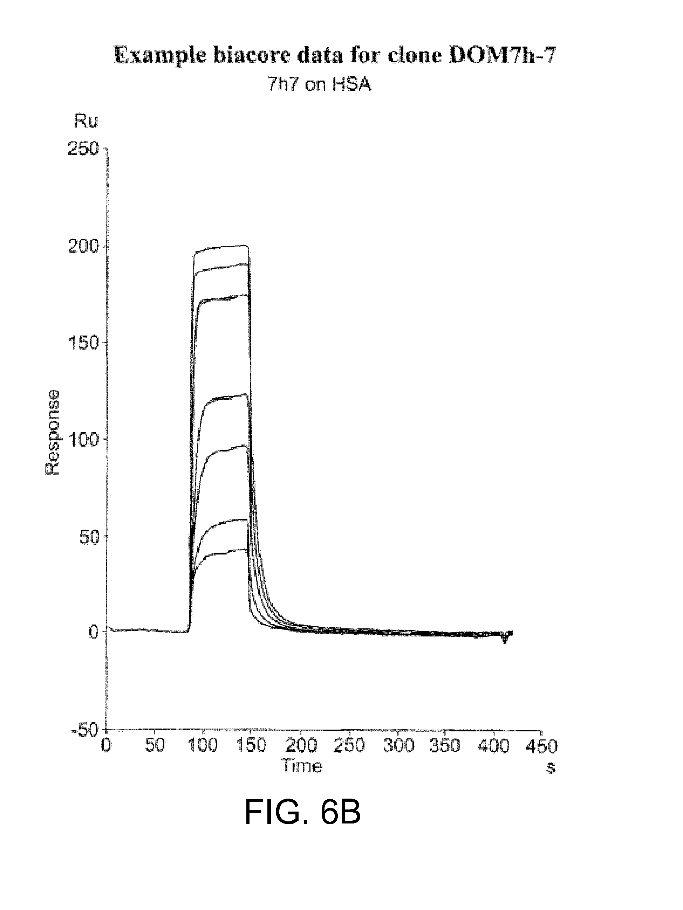

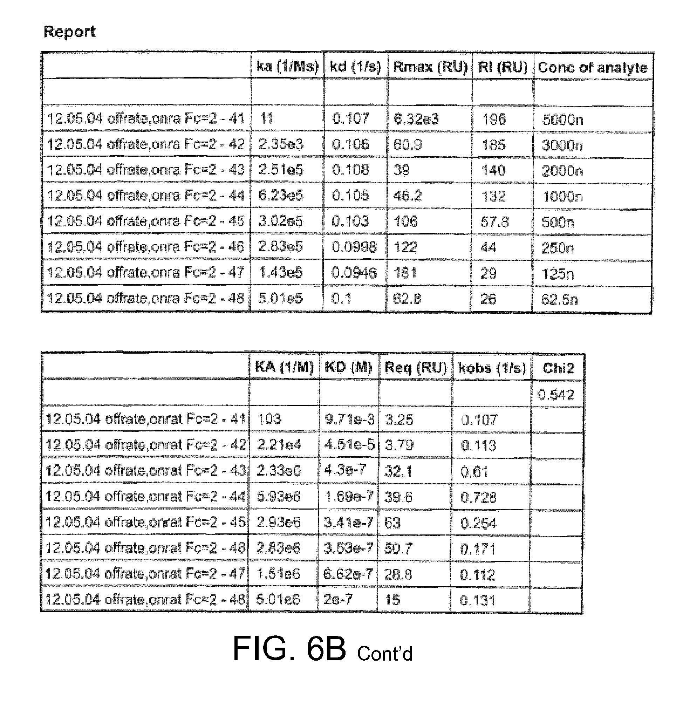

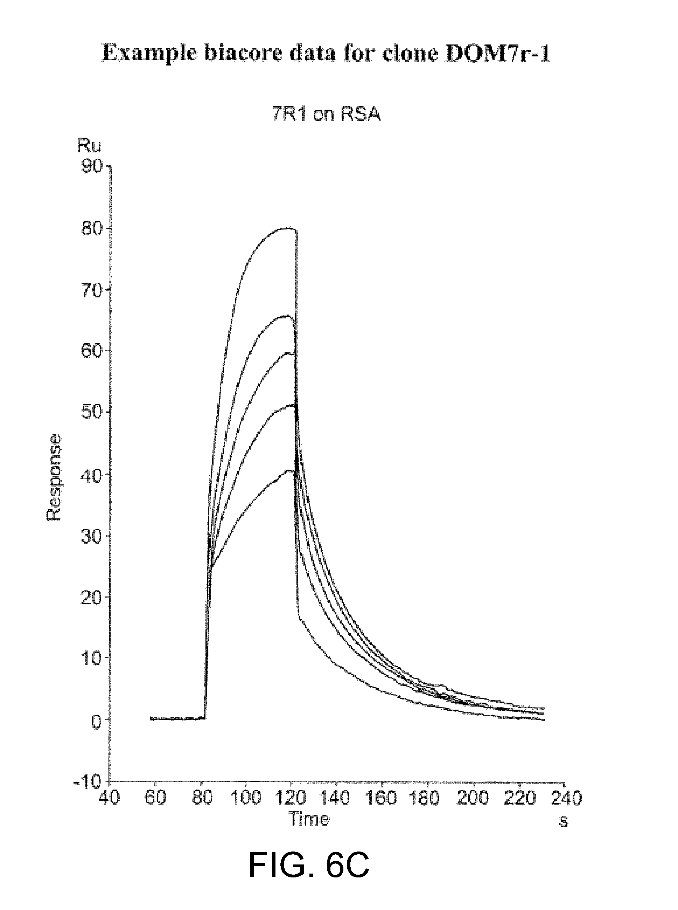

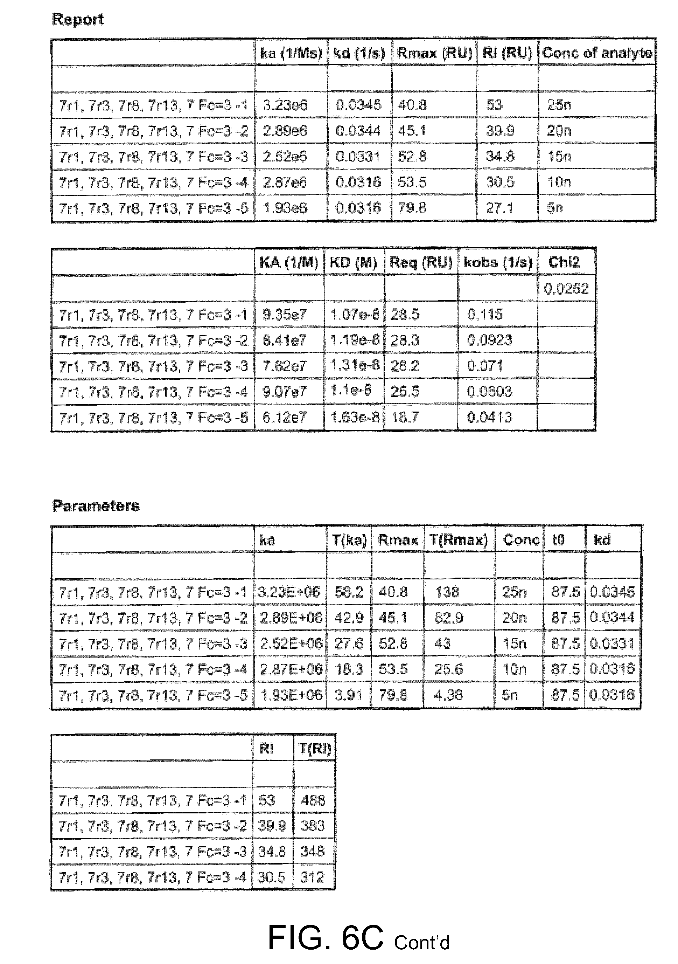

Affinity (KD) was calculated from the BIACORE traces by fitting on-rate and off-rate curves to traces generated by concentrations of dAb in the region of the KD. dAbs with a range of different affinities to serum albumin were identified. Included in the range 10-100nM, were the affinities of DOM7h-8 for HSA, DOM7h-2 for HSA and DOM7r-1 for RSA. Included in the range 100nM to 500nM were the affinities of DOM7h-7 for HSA, DOM7h-8 for RSA and DOM7h-26 for HSA. Included in the range 500nM to 5*µ*M were the affinities of DOM7h-23 for HSA and DOM7h-1 for HSA. Example traces are included in FIGS. 6A-6C.

### Example 2. Formatting anti-serum albumin antibodies as a fusion with IL-1 receptor antagonist (IL-1ra)

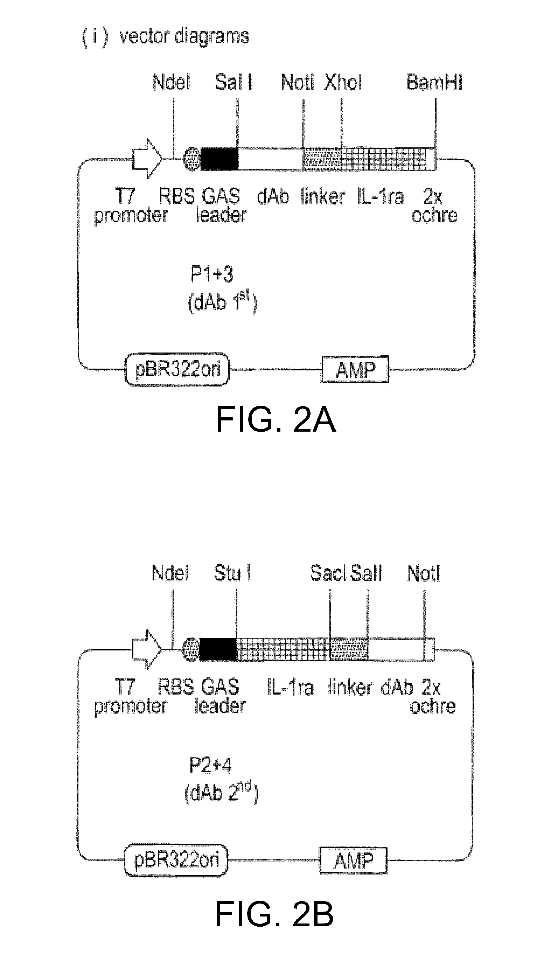

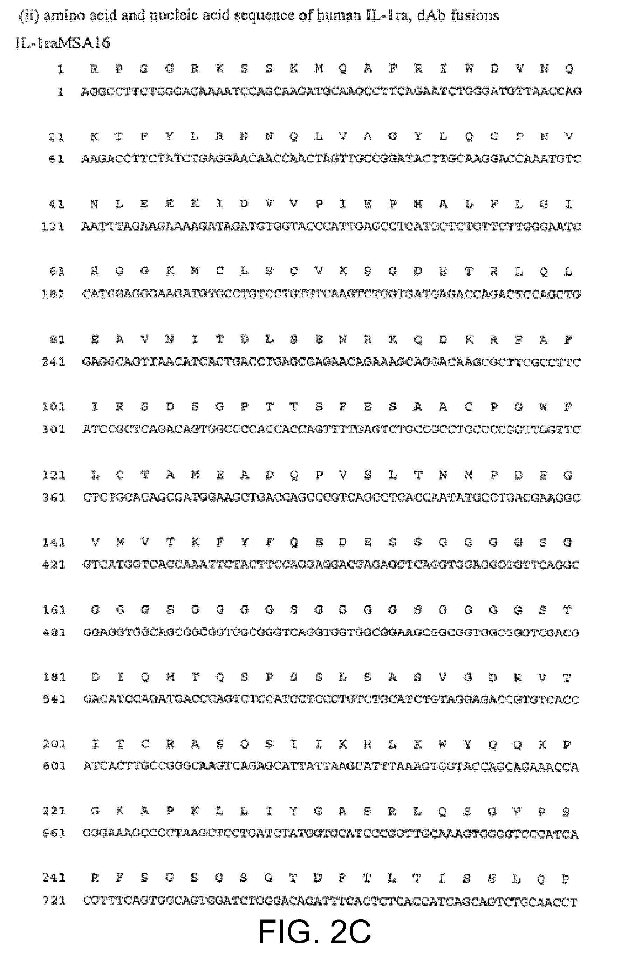

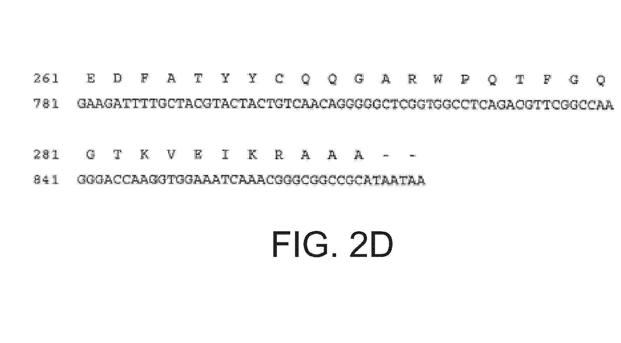

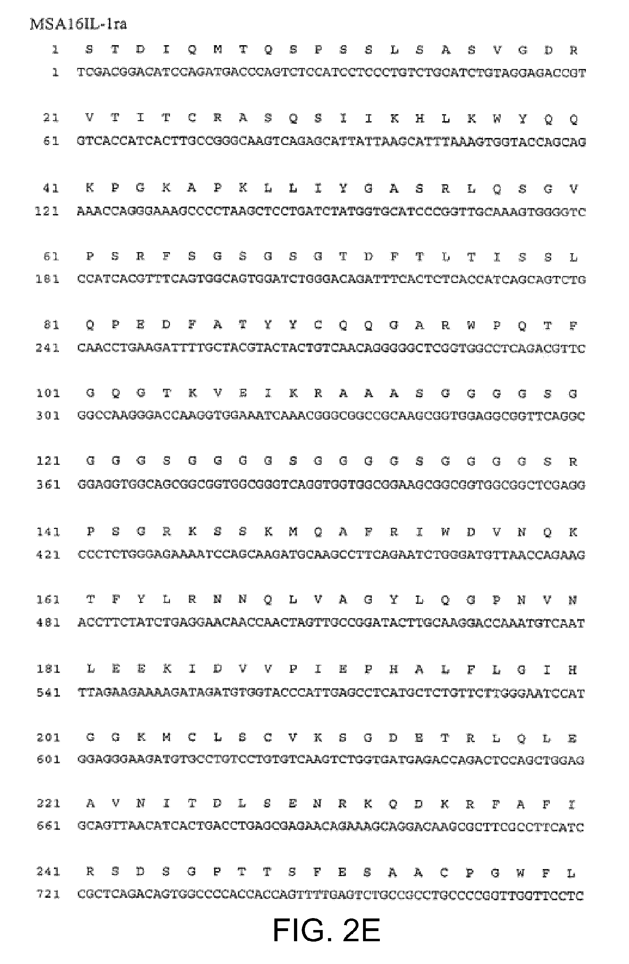

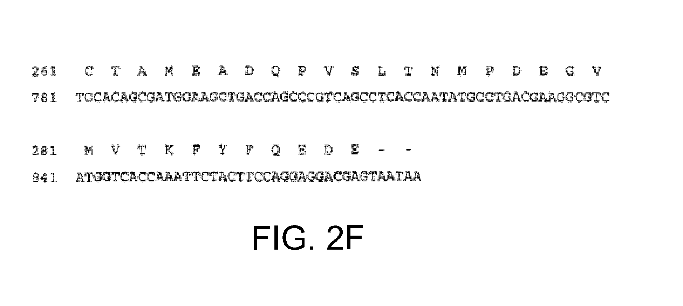

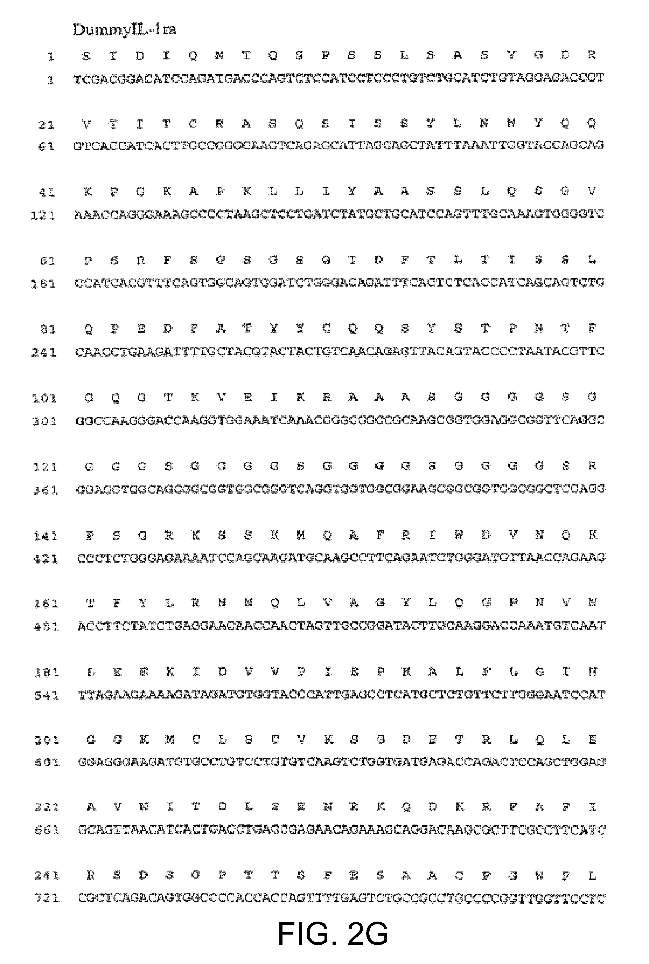

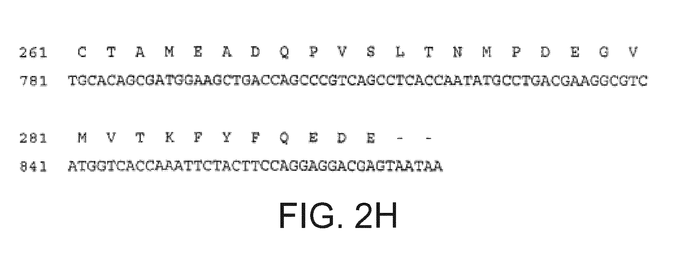

This example describes a method for making a fusion protein comprising IL-1ra and a dAb that binds to serum albumin. Two fusions were made, one with the dAb N-terminal of the IL-1ra (MSA16IL1-ra) and one with the dAb C-terminal of the IL-1ra (IL1-raMSA 16). The sequences of the fusions and the vector are shown in FIG. 2C and 2D. A control fusion that did not bind MSA was also produced, and its sequence is shown in FIG. 2E.

KINERET (anakinra, Amgen) has a short half life of 4-6 hours, and the recommended dosing regime calls for daily injections. This regime lead to injection site reaction in 14-28 days in 71% of cases. Therefore a form of human IL-1ra that has a longer serum half life would be beneficially and could increase efficacy and reduce dosing frequency. These are both desirable properties for a pharmaceutical.

### Cloning

Briefly, two multiple cloning sites (MCSs) were designed as detailed below and inserted into an expression vector with a T7 promotor. The restriction sites were designed for the insertion of IL1-ra, dAb, GAS leader and linker. One (MCS 1+3) encodes a protein with the dAb N terminal of the IL-1ra and the other (MCS 2 + 4) encode a protein with the dAb C terminal of the IL-1ra.

Cloning site 1+3 for dAbIL1-ra fusion
NdeI, stuffer, SalI, NotI, stuffer, XhoI, BamHI
gcgcatatgttagtgcgtcgacgtcaaaaggccatagcgggcggccgctgcaggtctcgagtgcgatggatcc (SEQ ID NO:35)
Cloning site 2+4 for IL1-radAb fusion
NdeI, stuffer, StUI, SacI, stuffer, SalI, NotI, TAA TAA BamHI

gcgcatatgttaagcgaggccttctggagagagctcaggagtgtcgacggacatccagatgacccaggcggccgctaataa
ggatccaatgc     (SEQ ID NO:36)

The GAS leader was then inserted into each vector by digesting the MCS using the appropriate restriction enzymes and ligating annealed primers coding for the leader. Next, linker DNA coding for the linker was inserted in a similar manner. DNA coding for IL-1ra was obtained by PCR (using primers designed to add the required restriction sites) from a cDNA clone and inserted into a TOPO cloning vector. After confirming the correct sequence by nucleic acid sequencing, DNA coding for IL-1ra was excised from the TOPO vector and ligated into the vectors containing leader and linker. Lastly, DNA coding for the dAb was excised from the dAb expression vector and inserted into the vectors by SalI/NotI digest of insert (purified by gel purification) and vector.

### Expression and purification

MSA16IL1-ra, IL1-raMSA16 and dummyIL-1ra were expressed in the periplasm of *E. coli* and purified from the supernatant using batch absorbtion to protein L-agarose affinity resin (Affitech, Norway) followed by elution with glycine at pH 2.2. The purified dAbs were then analysed by SDS-PAGE gel electrophoresis followed by coomassie staining. For one of the proteins (IL-1raMSA 16), > 90% of the protein was of the expected size and therefore was analysed for activity without further purification. The other proteins (MSA16IL1-ra and dummy IL-1ra) were contaminated by a smaller band and were therefore further purified by FPLC ion exchange chromatography on the RESOURSEQ ion exchange column at pH 9. Protein was eluted using a linear salt gradient form 0-500 mM NaCl. After analysis by SDS-PAGE gel electrophoresis, fractions containing a protein of the expected size were combined yielding a combined fraction of >90% purity. This protein was used for further analysis

### Example 3. Determination of activity of dAb IL1-ra fusion in vitro

### MRC-5 IL-8 assay

MSA16IL-1ra fusions were tested for the ability to neutralise the induction of IL-8 secretion by IL-1 in MRC-5 cells (ATCC Accession No. CCL-171; American Type Culture Collection, Manassas, VA). The method is adapted from Akeson, L. et al (1996) Journal of Biological Chemistry 271, 30517-30523, which describes the induction of IL-8 by IL-1 in HUVEC, MRC-5 cells were used instead of the HUVEC cell line. Briefly, MRC-5 cells plated in microtitre plates were incubated overnight with dAbIL-1ra fusion proteins or IL-1ra control, and IL-1 (100 pg/mL). Post incubation the supernatant was aspirated off the cells and IL-8 concentration measured via a sandwich ELISA (R&D Systems).

The activity of IL-1ra in the fusion proteins led to a reduction in IL-8 secretion. The reduction of IL-8 secretion resulting from activity of the MSA16IL1-ra fusion and from activity of the IL-1raMSA16 fusion was compared to the reduction seen with the IL-1ra control (recombinant human IL-1ra, R&D systems). The neutralizing dose 50 (ND₅₀) of each of the tested proteins was determined and is presented in Table 2.

**Table 2**

| Protein | ND₅₀ |
|---|---|
| IL-1ra | 0.5 nM |
| MSA16IL-1ra | 2 nM |
| IL-1raMSA16 | 8 nM |

The results demonstrate that IL-1ra remained active as part of a fusion construct with an anti-serum albumin dAb. The MSA16IL-1ra protein was further studied to assess its pharmacokinetics (PK study).

### Serum Albumin, anti IL-1ra sandwich ELISA

Three dAb/IL-1ra fusions were tested for the ability to bind serum albumin and simultaneously be detected by a monoclonal anti-IL1ra antibody. The fusions tested were MSA16IL-1ra, IL-1raMSA16 and dummyIL-1ra. Briefly, ELISA plate was coated overnight with mouse serum albumin at 10 *µ*g/ml, washed 5 x with 0.05% Tween PBS and then blocked for 1 hour with 4% Marvel PBS. After blocking, the plate was washed 5 x with 0.05% Tween PBS and then incubated for 1 hour with each dAb, Il-1ra fusion diluted in 4% MPBS. Each fusion was incubated at 1 *µ*M concentration and at 7 sequential 4-fold dilutions (ie down to 60pM). After the incubation, plates were washed 5 x with 0.05% Tween PBS and then incubated for 1 hour with the manufacturers recommended dilution of a rabbit polyclonal antibody (ab-2573) to human IL-1 receptor antagonist (Abcam, UK) diluted in 4% MPBS. After this incubation, plates were washed 5 x with 0.05% Tween PBS and then incubated for 1h with a 1/2000 dilution of secondary antibody (anti-rabbit IgG-HRP) diluted in 4% MPBS. Following incubation with the secondary antibody, plates were washed 3 x with 0.05% Tween PBS and 2 x with PBS and then developed with 50*µ*l per well of TMB microwell peroxidase substrate (KPL, MA) and the reaction stopped with 50 *µ*l per well of HCL. Absorption was read at 450 nM.

Both the MSA16IL-1ra and IL-1raMSA16 proteins were detected at more than 2 x background level at 1 *µ*M concentration in the sandwich ELISA. The MSA16IL-1ra protein was detected at 2 x background or higher at dilutions down to 3.9 nM, whereas the IL-1raMSA16 protein was detected at 2 x background only down to 500 nM. Binding of the MSA16IL-1ra fusion to serum albumin was shown to be specific for serum albumin as the control construct (dummyIL-1ra) did not bind serum albumin.

### Example 4. Determination of serum half life of drug fusions in mouse PK studies.

### A. Determination of the serum half-life in mouse of a MSA binding dAb/HA epitope tag fusion protein.

The MSA binding dAb/HA epitope tag fusion protein was expressed in the periplasm of *E. coli* and purified using batch absorption to protein L-agarose affinity resin (Affitech, Norway) followed by elution with glycine at pH 2.2. Serum half life of the fusion protein was determined in mouse following a single intravenous (i.v.) injection at approx 1.5 mg/kg into CD1 strain male animals. Analysis of serum levels was by ELISA using goat anti-HA (Abcam, UK) capture and protein L-HRP (Invitrogen, USA) detection which was blocked with 4% Marvel. Washing was with 0.05% Tween-20, PBS. Standard curves of known concentrations of MSA binding dAb/HA fusion were set up in the presence of 1x mouse serum to ensure comparability with the test samples. Modelling with a 1 compartment model (WinNonlin Software, Pharsight Corp., USA) showed the MSA binding dAb/HA epitope tag fusion protein had a terminal phase t1/2 of 29.1 hours and an area under the curve of 559 hr.µg/ml. This demonstrates a large improvement over the predicted half life for a HA epitope tag peptide alone which could be a short as only several minutes.

The results of this study using the HA epitope tag as a drug model, demonstrate that the *in vivo* serum half life of a drug can be extended when the drug is prepared as a drug fusion or drug conjugate with an antigen-binding fragment of (e.g., dAb) of an antibody that binds serum albumin.

The *in vivo* half life in mice of the anti-MSA dAbs DOM7m-16 and DOM7m-26, and a control dAb that does not bind MSA were also assessed. Again, DOM7m-16, DOM7m-26 and the control dAb contained an HA epitope tag, which serves as a model for a drug (e.g., a peptide drug). In this study, the control dAb, that does not bind MSA, had an *in vivo* half life of 20 minutes, whereas the *in vivo* half lives of DOM7m-16 and DOM7m-26 were significantly extended. (FIG. 12) DOM7m-16 was found to have an *in vivo* half life in mice of 29.5 hours in further studies.

In another study, the *in vivo* half life (t½ *β*) of DOM7h-8 which contained an HA epitope tag was evaluated in mice. Modelling with a 2 compartment model (WinNonlin Software, Pharsight Corp., USA) showed that DOM7h-8 had a t1/2*β* of 29.1 hours.

The results of each of these study using the HA epitope tag as a model for a drug *(e.g.,* a peptide drug), demonstrate that the *in vivo* serum half life of a drug can be dramatically extended when the drug is prepared as a drug fusion or drug conjugate with an antigen-binding fragment of (*e.g.,* dAb) of an antibody that binds serum albumin.

### B. Determination of the serum half-life in mouse of MSA binding dAb/IL-1ra fusion protein.

The MSA binding dAb/IL-1ra fusion protein (MSA16IL-1ra) was expressed in the periplasm of *E. coli* and purified using batch absorption to protein L-agarose affinity resin (Affitech, Norway) followed by elution with glycine at pH 2.2. Serum half life of the MSA16IL-1ra (DOM7m-16/IL-1ra), an IL-1ra fusion with a dAb that does not bind MSA (Dummy dAb/IL-1ra), and an anti-MSA dAb fused to the HA epitope tag (DOM7m-16 HA tag) was determined in mice following a single i.v. injection at approximately 1.5 mg/kg into CD 1 strain male animals.

Analysis of serum levels was by Il-1ra sandwich ELISA (R&D Systems, USA). Standard curves of known concentrations of dAb/IL-1ra fusion were set up in the presence of 1x mouse serum to ensure comparability with the test samples. Modelling was performed using the WinNonlin pharmacokinetics software (Pharsight Corp., USA).

It was expected that the IL-1ra fusion with the anti-MSA dAb would increase the serum half-life considerably when compared with the control which was a fusion of a non-MSA binding dAb with IL-1ra. The control non-MSA binding dAb/IL-1ra fusion was predicted to have a short serum half-life.

The results of the study are presented in Table 3, and show that the IL-1ra fusion with anti-MSA dAb (DOM7m-16/IL-1ra had a serum half life that was about 10 times longer than the IL-1ra fusion with a dAb that does not bind MSA (Dummy dAb/IL-1ra). The results also revealed that there was a > 200 fold improvement (increase) in the area under the concentration time curve for DOM7m-16/IL-1ra (AUC: 267 hr.µg/ml) as compared to dummy/IL-1ra (AUC: 1.5 5 hr.µg/ml)

**Table 3**

| Agent | Serum Half Life |
|---|---|
| DOM7m-16/IL-1ra | 4.3 hours |
| dummy/IL-1ra | 0.4 hours |
| DOM7m-16 HA tag | 29 hours |

The results of these studies demonstrate that the *in vivo* serum half life and AUC of a drug can be significantly extended when the drug is prepared as a drug fusion or drug conjugate with an antigen-binding fragment of (*e.g*., dAb) of an antibody that binds serum albumin.

### Example 5. Determination of the serum half-life in rats of RSA binding dAb/HA epitope tag fusion proteins.

Anti-rat serum albumin dAbs were expressed with C-terminal HA tags in the periplasm of *E. coli* and purified using batch absorption to protein L-agarose affinity resin (Affitech, Norway) for Vk dAbs and batch absorption to protein A affinity resin for VH dAbs, followed by elution with glycine at pH 2.2. In order to determine serum half life, groups of 4 rats were given a single i.v. injection at 1.5 mg/Kg of DOM7r-27, DOM7r-31, DOM7r-16, DOM7r-3 or a control dAb (HEL4) that binds an irrelevant antigen. Serum samples were obtained by serial bleeds from a tail vein over a 7 day period and analyzed by sandwich ELISA using goat anti-HA (Abcam, Cambridge UK) coated on an ELISA plate, followed by detection with protein A-HRP (for the V_{H} dAbs) or protein L-HRP (for V*κ* dAbs). Standard curves of known concentrations of dAb were set up in the presence of 1x rat serum to ensure comparability with the test samples. Modelling with a 2 compartment model (using WinNonlin pharmacokinetics software (Pharsight Corp., USA)) was used to calculate t1/2*β* and area under the curve (AUC) (Table 4).

**Table 4**

| Agent | Scaffold | Affintity (KD) for rat serum albumin | t1/2*β* | AUC (µg.hr/mL) |
|---|---|---|---|---|
| DOM7r-3 | V*_{κ}* | 12 nM | 13.7 hours | 224 |
| DOM7r-16 | V*_{κ}* | 1 µM | 34.4 hours | 170 |
| DOM7r-27 | V_{H} | 250 nM | 14.8 hours | 78.9 |
| DOM7r-31 | V_{H} | 5 µM | 5.96 hours | 71.2 |

The results of this rat study using the HA epitope tag as a model for a drug (*e.g.,* a peptide drug), demonstrate that the *in vivo* serum half life of a drug can be dramatically extended when the drug is prepared as a drug fusion or drug conjugate with an antigen-binding fragment of (*e.g.,* dAb) of an antibody that binds serum albumin.

### Prediction of half life in humans.

The *in vivo* half life of a dAb, drug fusion or drug conjugate in humans can estimated from half life data obtained in animals using allometric scaling. The log of the *in vivo* half lifes determined in 3 animals is plotted against the log of the weight of the animal. A line is drawn through the plotted points and the slope and y-intercept of the line are used to calculate the *in vivo* half life in humas using the formula log Y = log(a) + b log(W), in which Y is the *in vivo* half life in humans, log(a) is the y-intercept, b is the slope, and W is the weight of a human. The line can be produced using *in vivo* half life data obtain in animals that weigh about 35 grams (*e.g.,* mice), about 260 grams (*e.g.,* rats) and about 2,710 grams. For this calculation, the weight of a human can be considered to be 70,000 grams.

### Example 6. Efficacy of anti-SA dAb/IL-1ra drug fusion in mouse collagen induced arthritis model of rheumatoid arthritis.

Efficacy of the fusion DOM7m-16/IL-1ra and efficacy of IL-1ra in a recognized mouse model of rheumatoid arthritis (type II collagen induced arthritis (CIA) in DBA/1 mice) was assessed. Throughout the study, mice were maintained in a test facility in standard type 2 cages that were housed in a HEPA-filtered Scantainer at 20-24°C with a 12-hours light, 12-hours dark cycle. Food (Harlan-Teklad universal diet 2016) and UV sterilized water were provided *ad libitum.* The mice were imported to the test facility at least 7 days before the start the study to assure proper acclimitization.

DBA/1 mice at 7-8 weeks of age (obtained from Taconic M and B, Domholtveg, Denmark) were injected once with an emulsion of Arthrogen-CIA adjuvant and Arthrogen-CIA collagen (both MD biosciences) emulsified at a 1:1 ratio until the emulsion was stable. The emulsion was considered to be stable when a drop of the emulsion added to a beaker of water formed a solid clump. The mice were then injected with the emulsion.

Twenty-one days after the emulsion was injected, the 20 animals with the most advanced arthritic disease were eliminated from the study, and the remaining mice were divided into groups of 10 animals (each group contained 5 males and 5 females). The mice were treated as shown in Table 5, and all treatments were delivered at a concentration calculated so that 10 ml/Kg were administered.

**Table 5 .**

| Group | Treatment |
|---|---|
| 1 | IL-1ra, 1 mg/Kg (intrapertoneal (ip.) bolus) |
| 2 | IL-1ra, 10 mg/Kg (ip. bolus) |
| 3 | DOM7m-16/IL-1ra, 1 mg/Kg (ip. bolus) |
| 4 | DOM7m-16/IL-lra, 10 mg/Kg (ip. bolus) |
| 5 | ENBREL® (entarecept; Immunex Corporation), 5 mg/Kg (ip. bolus) |
| 6 | saline (negative control), 10 ml/Kg (ip. bolus) |
| 7 | Dexamethasone (positive control), 0.4 mg/Kg (subcutaneous injection) |

Clinical scores for the severity of arthritis were recorded 3 times a week from day 21 to day 49. Mice were euthanized at day 49. Individual mice were euthanized earlier if they presented an arthritic score of 12 or more, or had serious problems moving.

For clinical scoring, each limb was scored according to the criteria below and the scores for all four limbs were added to produce the total score for the mouse. This method resulted is a score of 0 to 16 for each mouse. Scoring critera were: 0 = normal; 1 = mild but definite redness and swelling of the ankle or wrist, or apparent redness and swelling limited to individual digits, regardless of the number of affected digits; 2 = moderate redness and swelling of ankle and wrist; 3 = severe redness and swelling of the entire paw including digits; 4 = maximally inflamed limb with involvement of multiple joints.

Group average arthritic scores were calculated for each treatment group on every treatment day using clinical scores from individual mice. Any animals that had been removed from the study for ethical reasons were allocated the maximum score of 16. The group average arthritic scores were plotted against time (FIG. 13).

Statistical analysis of the group average arthritic scores on day 49 were performed using the Wilcoxon test. This statistical analysis revealed that the two groups treated with DOM7m-16/IL-1ra (at 1 mg/Kg or 10 mg/Kg (Groups 3 and 4)) had significantly improved arthtritic scores at day 49 (at the P <1% and P <0.05% significance levels respectively) when compared to the saline control group (Group 6). In contrast, treatment with IL-1ra at 1 mg/Kg (Group 1) did not result in statistically significant improvement in the arthritic score at day 49, while treatment with IL-1ra at 10 mg/Kg (Group 2) resulted in a significant improvement at the P <5% significance level. Treatment with ENBREL® (entarecept; Immunex Corporation) (Group 5) resulted in significant improvement in the arthric score at day 49 at the P <10% significance level.

Treatment with DOM7m-16/IL-1ra at the 10 mg/Kg dose (Group 4), was effective at improving the arthtritic score at day 49 (significant at the P<0.5% level) when compared to standard treatment with ENBREL® (entarecept; Immunex Corporation) at 5mg/Kg (Group 5). In addition, treatment with DOM7m-16/IL-1ra at the lower 1mg/Kg dose (Group 3), was more efficacious at improving the arthtritic score at day 49 than treatment with IL-1ra alone at the same dosage (Group 1) (significant at the P<10% level).

The results of the study show that at certain doses DOM7m-16/IL-1ra was more effective than IL-1ra or ENBREL® (entarecept; Immunex Corporation) in this study. The response to IL-1ra was dose dependant, as expected, and the response to DOM7m-16/IL-1ra was also dose dependant. The average scores for treatment with DOM7m-16/IL-1ra at 1mg/Kg were consistently lower than the average scores obtained by treatment with IL-1ra at 10 mg/kg. These plotted results (FIG. 13) indicate that treatment with DOM7m-16/IL-1ra was about 10 times more effective than IL-1ra in this study.

This superior efficacy of DOM7m-16/IL-1ra was observed even though the DOM7-16/IL-1ra fusion protein contains about half the number of IL-1 receptor binding epitopes as IL-1ra on a weight basis (*e.g.,* 1 mg of DOM7m-16/IL-1ra (MW . 31.2 kD) contains about half the number of IL-1 receptor binding epitopes as 1 mg of IL-1ra (MW. 17.1 kD).

The results of this study demonstrate that a dAb that binds serum albumin can be linked to IL-1ra (a clinically proven therapy for RA) and that the resulting drug fusion has both long serum half life properties (conferred by the dAb) and IL-1 receptor binding properties (conferred by the IL-1ra). Due to the serum residence time of the drug fusion, the dose of DOM7-16/IL-1ra that was effective for treating CIA was dramatically reduced relative to IL-1ra.

The results of this study demonstrate that in addition to the benefits of extended half life and increased AUC, drugs prepared as drug fusions or drug conjugates with an antigen-binding fragment of (*e.g.,* dAb) of an antibody that binds serum albumin are highly effective therapeutic agents that provide advantages over drug alone. For example, as demonstrated in the mouse CIA model, a lower dose of drug fusion was effective and inhibited the joint inflammation and joint damage caused by IL-1 over a longer period of time in comparison to IL-1ra alone, and provided greater protection against disease progression.

### Example 7. Anti-SA dAb/Saporin noncovalent drug conjugate

The ribosome-inactivating protein Saporin (an anti-cancer drug) is highly stable to denaturants and proteases and has been used as a targeted toxin to T lymphocytes. A non-covalent drug conjugate was prepared by coupling Saporin to DOM7h-8 via a biotin-streptavidin link. Results obtained with this non-covalent drug conjugate demonstrates that the DOM7h-8 retains its serum albumin binding characteristics when coupled to a drug.

A variant DOM7h-8 referred to as DOM7h-8cys, in which the C-terminal arginine at position 108 (amino acid 108 of SEQ ID NO: 24) was replaced with a cysteine residue was prepared by expression of a recombinant nucleic acid in HB2151 cells. The cells were grown and induced at 30°C in overnight expression autoinduction TB readymix (Merck KGa, Germany) for 72 hours before recovery of the supernatant by centrifugation. DOM7h-8cys was purified from the supernatant using affinity capture on protein L-agarose. The resin was then washed with 10 column volumes of 2 x PBS and DOM7h-8cys was eluted with 0.1 M glycine pH2. Eluted DOM7h-8cys was neutralised with 0.2 x volume of Tris pH8 and concentrated to 1mg/ml (using a CENTRICON 20 ml concentrator (Millipore Corp., MA).

Concentrated DOM7h-8cys was buffer exchanged to PBS using a NAP5 desalting column (GE Healthcare/Amersham Biosciences, NJ) and concentration determined. The dAb was then biotinylated (via primary amines) using EZ-LINK sulfo-NHS-LC-biotin (Pierce Biotechnology Inc., IL). The biotinylated dAb was mixed with streptavidin-saporin in a 1:1 molar ratio.

In order to confirm that the dAb/saporin complex was formed, a sandwich ELISA was used to detect intact complexes. Human serum albumin (HAS) was coated onto half of the wells of an ELISA plate (Nunc, NY) overnight at 10 *µ*g/ml in a volume of 100 *µ*l per well. After overnight incubation, the plate was washed 3 times with PBS, 0.05% Tween and then the whole plate was blocked for 2 hours with 2% PBS. After blocking, the plate was washed 3 times with PBS, 0.05% Tween and then incubated for 1 hour with DOM7h-8/saporin non-covalent conjugate diluted to 0.5 *µ*M in 2% Tween PBS. As controls on the same ELISA plate, uncoupled saporin at 0.5 *µ*M and uncoupled DOM7h8 at 0.5 *µ*M were incubated in 2% Tween PBS. Additional controls were the same three diluted proteins incubated on wells of the ELISA plate not coated with HSA and blocked with 2% Tween. After the incubation, the plate was washed 3 times with PBS, 0.05% Tween and then incubated for 1 hour with 1/2000 dilution of goat anti-saporin polyclonal antibody (Advanced Therapeutic Systems) diluted in 2% Tween PBS. After the incubation, the plate was washed 3 times with PBS, 0.05% Tween and then incubated for 1 hour with the secondary detection antibody (of 1/2000 anti-goat Ig HRP conjugate). After the incubation, the plate was washed 3 times with PBS, 0.05% Tween and once with PBS and tapped dry on paper. The ELISA was developed with 100 *µ*l 3, 3', 5, 5'-tetramethylbenzidine as substrate and the reaction stopped with 50 *µ*l 1M hydrochloric acid. The presence of non-covalent conjugates of DOM7h-8 and saporin was confirmed by comparing the OD600 of the conjugate with that of either of the unconjugated parts.

**Table 6**

| | DOM7h-8/Saporin | DOM7h-8 alone | Saporin alone |
|---|---|---|---|
| OD600 (plate coated with HAS) | 0.311 | 0.060 | 0.079 |
| OD600 (plate blocked with 2% Tween PBS) | 0.078 | 0.068 | 0.075 |

The results of this study demonstrate that a drug can be conjugated to an antigen-binding fragement of an antibody that binds serum albumin, and that the conjugated antigen-binding fragment retains serum albumin-binding activity. In addition, due to the stability and strength of the biotin-streptavidin integration, the results show that covalently bonded and noncovalently bonded conjugates can be prepared that retain the serum albumin-binding activity of the antigen-binding fragment of an antibody that binds serum albumin.

### Example 8. Anti-SA dAb/Fluorescein conjugate

Fluorescein isothiocyanate (FITC) can be cross linked with amino, sulfhydryl, imidazoyl, tyrosyl or carbonyl groups on a protein. It has a molecular weight of 389 Da which is comparable in size to many small molecule drugs. Results obtained with this conjugate demonstrate that the anti-sa dAb maintains its serum albumin binding characteristics when coupled to a small chemical entity, and indicate that small molecule drugs can be conjugated to anti-SA dAbs.

Concentrated DOM7h-8cys was prepared as described in Example 7. The concentrated dAb was buffer exchanged to 50 mM Borate pH 8 (coupling buffer) using a NAP5 desalting column (GE Healthcare/Amersham Biosciences, NJ) and then concentrated to 2.3 mg/ml using a 2 ml CENTRICON concentrator (Millipore Corp., MA). The FITC (Pierce Biotechnology Inc.) was diluted to 10 mg/ml in dimethyl formamide (DMF) according to the manufacturer's instructions and then mixed with the dAb in coupling buffer at a molar ratio of 24:1 FITC:dAb. The reaction was allowed to proceed for 30 minutes. At this point, excess unreacted FITC was removed from the reaction using a PD10 desalting column (GE Healthcare/Amersham Biosciences, NJ) that was pre-equilibrated with PBS, and the DOM7h-8cys/FITC,conjugate was eluted with PBS.

In order to confirm that the FITC/dAb coupling reaction was successful, a sandwich ELISA was used to detect coupled dAb. Human serum albumin (HSA) was coated onto half of the wells of an ELISA plate (Nunc, NY) overnight at 10 *µ*g/ml in a volume of 100 *µ*l per well. After overnight incubation, the whole plate was washed 3 times with PBS, 0.05% Tween and then all the wells were blocked for 2 hours with 2% Tween PBS. After blocking, the plate was washed 3 times with PBS, 0.05% Tween and then incubated for 1 hour with DOM7h-8cys/FITC diluted to 1 *µ*M in 2% Tween PBS. As controls on the same ELISA plate, a control FITC coupled antibody at 1 *µ*M and uncoupled DOM7h-8 at 1 *µ*M were incubated in 2% Tween PBS. Additional controls were the same three diluted proteins incubated on wells of the ELISA plate not coated with HSA and blocked with 2% Tween. After the incubation, the plate was washed 3 times with PBS, 0.05% Tween and then incubated for 1 hour with 1/500 dilution of rat anti FITC antibody (Serotec) diluted in 2% Tween PBS. After the incubation, the plate was washed 3 times with PBS, 0.05% Tween, and then incubated for 1 hour with the secondary detection antibody diluted in 2% Tween PBS (1/5000 anti-rat Ig HRP conjugate). After the incubation, the plate was washed 3 times with PBS, 0.05% Tween and once with PBS and tapped dry on paper. The ELISA was developed with 100 µl per well 3,3',5,5'-tetramethylbenzidine as substrate and the reaction stopped with 50 µl per well 1M hydrochloric acid. The presence of conjugates of DOM7h-8 and FITC was confirmed by comparing the OD600 of the conjugate with that of either of the unconjugated parts.

**Table 7**

| | DOM7h-8/FITC | DOM7h-8 alone | FITC coupled antibody (negative control) |
|---|---|---|---|
| OD600 (plate coated with HSA) | 0.380 | 0.042 | 0.049 |
| OD600 (plate blocked with 2% Tween PBS) | 0.041 | 0.041 | 0.045 |

### Example 9. anti-SA dAb/peptide conjugates.

Many peptides have therapeutic effects. Model peptides with an N or C terminal cysteine can be coupled to an anti-serum albumin dAb.

In this case, four different peptides will be used: peptide 1 YPYDVPDYAKKKKKKC (SEQ ID NO:68); peptide 2 CKKKKKKYPYDVPDYA (SEQ ID NO:69); peptide 3 HHHHHHKKKKKKC (SEQ ID NO:70) and peptide 4: CKKKKKKHHHHHH (SEQ ID NO:71). Peptides 1 and 2 include the sequence of the hemagglutinin tag (HA tag) and peptides 3 and 4 include the sequence of the His tag. Concentrated DOM7h-8cys will be prepared as described in Example 7.

The concentrated dAb will be reduced with 5 mM dithiothreitol and then buffer exchanged to coupling buffer (20 mM BisTris pH 6.5, 5 mM EDTA, 10% glycerol) using a NAP5 desalting column (GE Healthcare/Amersham Biosciences, NJ). Cysteins will be blocked (to prevent the dAb dimerising with itself) using a final concentration of 5 mM dithiodipyridine which will be added to the dAb solution form a stock of 100 mM dithiodipyridine in DMSO. The dAb and dithiodipyrdine will be left to couple for 20-30 minutes. Unreacted dithiodipyridine will then be removed using a PD10 desalting column and the dAb will be eluted in coupling buffer (20 mM BisTris pH 6.5, 5 mM EDTA, 10% glycerol). The resulting protein will then be frozen until required.

Peptides 1-4 will be individually dissolved in water at a concentration of 200 *µ*M, will be reduced using 5 mM DTT and then will be desalted using a NAP5 desalting column (GE Healthcare/Amersham Biosciences, NJ). Each peptide will then be added to a solution of reduced and blocked dAb at a 20:1 ratio, for the peptide-dAb coupling to occur. In order to confirm success of the peptide, dAb coupling reactions, a sandwich ELISA will be used to detect anti-SA dAb/peptide conjugates.

Human serum albumin will be coated onto an ELISA plate (Nunc, NY) overnight at 10 *µ*g/ml in a volume of 100 *µ*l per well. After overnight incubation, the plate will be washed 3 times with PBS, 0.05% Tween and then will be blocked for 2 hours with 4% Marvel PBS. After blocking, the plate will be washed 3 times with PBS, 0.05% Tween and then will be incubated for 1 hour with DOM7h-8/peptide conjugates diluted to 1 *µ*M in 4% Marvel PBS. As controls on the same ELISA plate, uncoupled peptide at 20 *µ*M and uncoupled DOM7h-8 at 1 *µ*M will be incubated in 4% MPBS. After the incubation, the plate will be washed 3 times with PBS, 0.05% Tween and then will be incubated for 1 hour with 1/2000 dilution of goat anti-HA antibody (Abcam) for peptides 1 and 2, and a 1/2000 dilution of Ni NTA-HRP (for peptides 3 and 4) diluted in 4% Marvel PBS. After incubation, the plate will be washed 3 times with PBS, 0.05% Tween and the wells with the goat anti HA antibody will be incubated for 1h with seconday anti-goat HRP antibody diluted 1/2000 in 4% MPBS (other wells were blocked for 1h). After the incubation, the plate will be washed 3 times with PBS, 0.05% Tween and once with PBS and will then be tapped dry on paper. The ELISA will be developed with 3, 3', 5, 5'-tetramethylbenzidine as substrate and the reaction will be stopped with 1M hydrochloric acid. The presence of conjugates of DOM7h-8/peptide conjugate will be confirmed by comparing the OD600 of the conjugate with that of either of the unconjugated parts.

| Table 8 Anticancer Peptides | | |
|---|---|---|
| Peptide Category | Peptide Sequence | Action/Application |
| LH-RH Agonistis and Antagonists | p-Glu-His-Trp-Ser-Tyr-Gly-Leu-Arg-Pro-Gly-NH2 | Treatment of sex hormone dependent malignant diseases |
| | SEQ ID NO:89 | |
| Gastrin Releasing Peptide | p-Glu-Gln-Arg-Leu-Gly-Asn-Gln-Trp-Ala-Val-Gly-His-Leu-Met-NH2 | Small Cell Lung Carcinoma |
| | SEQ ID NO:90 | |
| Somatostatin | p-Ala-Gly-Cys-Lys-Asn-Phe-Trp-Lys-Thr-Phe-Thr-Ser-Cys | Tumors (general) |
| | SEQ ID NO:91 | |
| GH-RH | Gln-Trp-Ala-Val-Gly-His-Leu-psi(CH2-NH)-Leu-NH2 (RC-3094) | Glioblastoma Tumor, Prostate Tumor |
| | SEQ ID NO:92 | |
| VEGF | Arg-Arg-Lys-Arg-Arg-Arg | Human Colon Carcinoma |
| | SEQ ID NO:93 | |
| | Ala-Thr-Trp-Leu-Pro-Pro-Arg | Tumor Cell Proliferation |
| | SEQ ID NO:94 | |
| | Arg-Thr-Glu-Leu-Asn-Val-Gly-Ile-Asp-Phe-Asn-Trp-Glu-Tyr-Pro-Ala-Ser-Lys | Tumor Cell Proliferation and |
| | SEQ ID NO:95 | Migration |
| | His-His-Glu-Val-Val-Lys-Phe-Mel-Asp-Val-Tyr-Gln | Inhibit endothelial cell responses |
| | SEQ ID NO:96 | |
| | Asn-Ile-Thr-Val-Thr-Leu-Lys-Lys-Phe-Pro-Leu | Angiogenesis Inhibitor |
| | SEQ ID NO:97 | |
| EGF | Cys-His-Ser-Gly-Tyr-Val-Gly-Val-Arg-Cys | Inhibits EGF based cell proliferation |
| | SEQ ID NO:98 | |
| | Tyr-Cys-Asp-Gly-Phe-Tyr-Ala-Cys-Tyr-Met-Asp-Val-Nh2 | Binds to HER2 |
| | SEQ ID NO:99 | |
| IL-6 | Gly-Gly-Cys-Lys-Leu-Trp-Thr-Ile-Pro-Glu-Cys-Gly-Gly | Inhibits cellular growth |
| | SEQ ID NO:100 | |
| IL-8 | Ala-Val-Leu-Pro-Arg | Apoptosis induction and antitumor effect *in vivo* |
| | SEQ ID NO:101 | |
| PDGF | Tyr-Gly-Arg-Pro-Arg-Glu-Ser-Gly-Lys-Lys-Arg-Lys-Arg-Lys-Arg-Leu-Lys-Pro-Thr | Inhibits growth of malignant glioma |
| | SEQ ID NO:102 | |
| TNF | AcCys-Pro-Ser-Glu-Gly-Leu-Cys-NH2 | Inhibit Tumor Growth |
| | SEQ ID NO:103 | |
| | Ac-Cys-Pro-Ser-Glu-Gly-Thr-Pro-Ser-Thr-His-Val-Leu-Cys-NH2 | |
| | SEQ ID NO:104 | |
| | Ac-Leu-Ala-Asn-Gly-Val-Glu | |
| | SEQ ID NO:105 | |
| | Pro-Gln-Ala-Glu-Gly-Gln-Leu-NH2 | |
| | SEQ ID NO:106 | |
| | Val-Ala-Asn-Pro-Gln-Ala-Glu-Gly-Gln-Leu | |
| | SEQ ID NO:107 | |
| | Cyclic Lys-Gly-Asp-Gln-Leu-Ser | |
| | SEQ ID NO:108 | |
| | Cyclic Tyr-Ser-Cln-Val-Leu-Phe-Lys-Gly | |
| | SEQ ID NO:109 | |
| Alpha-feto Protein | Glu-Met-Thr-Pro-Val-Asn-Pro-Gly | Inhibits Estrogen Dependent Breast Cancer Cells |
| | SEQ ID NO:110 | |
| Sialyl-Lewis mimics | Ile-Glu-Leu-Leu-Gln-Ala-Arg | Inhibits lung colonization of tumor cells |
| | SEQ ID NO:111 | |
| Urokinase-type Plasminogen activator | Cys-Val-Ser-Asn-Lys-Tyr-Phe-Ser-Asn-Ile-His-Trp-Cys | Antagonist for uPA/uPAR |
| | SEQ ID NO:112 | |
| | Phe-X-X-Tyr-Lys-Trp | Antagonist for uPA/uPAR |
| | SEQ ID NO:113 | |
| | Lys-Trp-X-X-Ar | Antagonist for uPA/uPAR |
| | SEQ ID NO:114 | |
| | Leu-Asn-Phe-Ser-Gln-Tyr-Leu-Trp-Tyr-Thr-NH2 | Antagonist for uPA/uPAR |
| | SEQ ID NO:115 | |
| | Ac-Lys-Pro-Ser-Ser-Pro-Pro-Glu-Glu-NH2 | Inhibits tumor progression and angiogenesis |
| | SEQ ID NO:116 | |
| p53 | Ac-Met-Pro-Arg-Phe-Met-Asp-Tyr-Trp-Glu-Gly-Leu-Asn-NH2 | Inhibits Hdm2 and p53 binding |
| | SEQ ID NO:117 | |
| | Met-Val-Arg-Arg-Phe-Leu-Val-Thr-Leu-Arg-Ile-Arg-Arg-Ala-Cys-Gly-Pro-Pro-Arg-Val | Prevents p53 ubiquitination |
| | SEQ ID NO:118 | |
| | Gly-Ser-Arg-Ala-His-Ser-Ser-His-Leu-Lys-Ser-Lys-Gly-Gln-Ser-Thr-Ser-Arg-His-Lys-Lys-Leu | Activate p53 |
| | SEQ ID NO:119 | |
| p34cdc2 | Cys-Ala-Phe-Tyr-Ile | Inhibit interaction between p34/p33 and pRb2 and p107 |
| | SEQ ID NO:120 | |
| | Leu-Cys-Ala-Phe-Tyr-Ile-Met-Ala-Lys | |
| | SEQ ID NO:121 | |
| | Met-Cys-Ser-Met-Tyr-Gly-Ile-Cys-Lys | |
| | SEQ ID NO:122 | |
| | Tyr-Ser-Phe-Val-His-Gly-Phe-Phe-Asn- | |
| Cdk2 | Phe-Arg-Val-Ser-Trp-Arg-Glu-Met-Leu-Ala | Inhibits interaction between Cdk2 and histone H1 |
| | SEQ ID NO:123 | |
| p21WAF1 | Lys-Arg-Arg-Gln-Thr-Ser-Met-Thr-Ala-Phe-Tyr-His-Ser-Lys-Arg-Arg-Leu-Ile-Phe-Ser | Induces G1/S growth arrest |
| | SEQ ID NO:124 | |
| | Lys-Arg-Arg-Leu-Ile-Phe-Ser-Lys | |
| | SEQ ID NO:125 | |
| | Phe-Leu-Asp-Thr-Leu-Val-Val-Leu-His-Arg | |
| | SEQ ID NO:126 | |
| E2F/DP transcription | Arg-Cys-Val-Arg-Cys-Arg-Phe-Val-Val-Trp-Ile-Gly-Leu-Arg-Val-Arg-Cys-Leu-Val | Inhibited E2F function in vitro |
| | SEQ ID NO:127 | |
| | Leu-Asn-Trp-Ala-Trp-Ala-Ala-Glu-Val-Leu-Lys-Val-Gln-Lys-Arg-Arg-Ile-Tyr-Asp-Ile-Thr-Asn-Val | |
| | SEQ ID NO:128 | |
| | Leu-Glu-Gly-Ile-Gln-Leu-Ile-Ala-NH2 | |
| | SEQ ID NO:129 | |
| | Phe-Trp-Leu-Arg-Phe-Thr | |
| | SEQ ID NO:130 | |
| | Trp-Val-Arg-Trp-His-Phe | |
| | SEQ ID NO:131 | |
| | Trp-Val-Arg-Trp-His | |
| | SEQ ID NO:132 | |
| | Trp-His-Phe-Ile-Phe-Trp | |
| | SEQ ID NO:133 | |
| | Ile-Trp-Leu-Ser-Gly-Leu-Ser-Arg-Gly-Val-Trp-Val-Ser-Phe-Pro | |
| | SEQ ID NO:134 | |
| | Gly-Ser-Arg-Ile-Leu-Thr-Phe-Arg-Ser-Gly-Ser-Trp-Tyr-Ala-Ser | |
| | SEQ ID NO:135 | |
| | Asp-Glu-Leu-Lys-Arg-Ala-Phe-Ala-Ala-Leu-Arg-Asp-Gln-Ile | |
| | SEQ ID NO:136 | |
| Bcl2 | Lys-Lys-Leu-Ser-Glu-Cys-Leu-Lys-Lys-Arg-Ile-Gly-Asp-Glu-Leu-Asp-Ser | Trigger apoptosis in a cell free system |
| | SEQ ID NO:137 | |
| | Gly-Gln-Val-Gly-Arg-Gln-Leu-Ala-Ile-Ile-Gly-Asp-Asp-Ile-Asn-Arg | |
| | SEQ ID NO:138 | |
| | Arg-Asn-Ile-Ala-Arg-His-Leu-Ala-Gln-Val-Gly-Asp-Ser-Met-Asp-Arg | |
| | SEQ ID NO:139 | |
| Integrins | Tyr-Ile-Gly-Ser-Arg-NH2 | Inhibit tumor cell binding to ECMs |
| | SEQ ID NO:140 | |
| | Ac-Tyr-Ile-Gly-Ser-Arg-NH2 | |
| | SEQ ID NO:141 | |
| | Ac-Tyr-Ile-Gly-Ser-Arg-NHCH3 | |
| | SEQ ID NO:142 | |
| | Ac-Tyr-Ile-Gly-Ser-Arg-N(CH3)2 | |
| | SEQ ID NO:143 | |
| | Phe(pNH2)-Ile-Gly-Ser-Arg-NH2 | |
| | SEQ ID NO:144 | |
| | Ac-Tyr-Ile-Gly-Ser-Arg-NHCH(CH3)2 | |
| | SEQ ID NO:145 | |
| | CO(Asp-Tyr-Ile-Gly-Ser-Arg-NHPr)2 | |
| | SEQ ID NO:146 | |
| | Arg-Gly-Asp | |
| | SEQ ID NO:147 | |
| | Tyr-Ile-Gly-Ser-Arg | |
| | SEQ ID NO:148 | |
| | Ile-Pro-Cys-Asn-Asn-Lys-Gly-Ala-His-Ser-Val-Gly-Leu-Met-Trp-Trp-Met-Leu-Ala-Arg | |
| | SEQ ID NO:149 | |
| Angiostatin Analogues | Ser-Pro-His-Arg-Pro-Arg-Phe-Ser-Pro-Ala | |
| | SEQ ID NO:150 | |
| | Ser-Pro-His-Ala-His-Gly-Tyr-Ile-Pro-Ser | |
| | SEQ ID NO:151 | |
| | Thr-Pro-His-Thr-His-Asn-Arg-Thr-Pro-Glu | |
| | SEQ ID NO:152 | |
| | Thr-Pro-His-Arg-His-Gln-Lys-Thr-Pro-Glu | |
| | SEQ ID NO:153 | |
| | Glu-Pro-His-Arg-His-Ser-Ile-Phe-Thr-Pro-Glu | |
| | SEQ ID NO:154 | |
| Cadherins | Ac-Cys-His-Ala-Val-Cys-NH2 | Angiogenesis Inhibitor |
| | SEQ ID NO:155 | |
| Histone Deacetylase | Cys-Glu-Lys-His-Ile-Met-Glu-Lys-Ile-Gln-Gly-Arg-Gly-Asp-Asp-Asp-Asp | Leukemia Inhibition |
| | SEQ ID NO:156 | |
| MMP2 | Cys-Thr-Thr-His-Trp-Gly-Phe-Thr-Leu-Cys | Tumor Metastasis |
| | SEQ ID NO:157 | |

### Example 10. Analysis of a GLP Drug Composition

The potency of an insulinotropic agent can be determined by calculating the EC50 value from the dose-response curve. Purified plasma membranes from a stable transfected cell line, BHK467-12A (tk-ts13), expressing the human GLP-1 receptor will be stimulated with GLP-1 and peptide analogues, and the potency of cAMP production will be measured using the AlphaScreen™ cAMP Assay Kit from Perkin Elmer Life Sciences.

A stable transfected cell line will be prepared and a high expressing clone selected for screening. The cells will then be grown at 5% C02 in DMEM, 5% FCS, 1% Pen/Strep and 0. 5 mg/ml G418.

Cells at approximate 80% confluence will be washed 2X with PBS and harvested with Versene, centrifuged 5 min at 1000 rpm and the supernatant removed. The additional steps will be made on ice. The cell pellet will be homogenized by the Ultrathurax for 20-30 sec. in 10 mi of Buffer 1 (20 mM Na-HEPES, 10 mM EDTA, pH7.4), centrifuged 15 min at 20.000 rpm and the pellet resuspended in 10 ml of Buffer 2 (20 mM Na-HEPES, 0.1 mM EDTA, pH7.4). The suspension will be homogenized for 20-30 sec and centrifuged 15 min at 20.000 rpm. Suspension in Buffer 2, homogenization and centrifugation will be repeated once and the membranes resuspended in Buffer 2 and ready for further analysis or stored at-80°C.

The functional receptor assay will be carried out by measurering the peptide induced cAMP production by The AlphaScreen Technology. The basic principle of The AlphaScreen Technology is a competition between endogenous cAMP and exogenously added biotin-cAMP. The capture of cAMP is achieved by using a specific antibody conjugated to acceptor beads. Formed cAMP will be counted and measured with an AlphaFusion Microplate Analyzer. The EC50 values will be calculated using the Graph-Pad Prisme software.

Resistance of a peptide to degradation by dipeptidyl aminopeptidase IV can be determined by the following degradation assay: Aliquots of the peptides will be incubated at 37 °C with an aliquot of purified dipeptidyl aminopeptidase IV for 4-22 hours in an appropriate buffer at pH 7-8 (buffer not being albumin). Enzymatic reactions will be terminated by the addition of trifluoroacetic acid, and the peptide degradation products will be separated and quantified using HPLC or LC-MS analysis. The mixtures will be applied onto a Zorbax 300SB-C18 (30 nm pores, 5 um particles) 150 x 2.1 mm column and eluted at a flow rate of 0.5 ml/min with a linear gradient of acetonitrile in 0. 1% trifluoroacetic acid (0%-100% acetonitrile over 30 min). Peptides and their degradation products may be monitored by their absorbance at 214 nm (peptide bonds) or 280 nm (aromatic amino acids), and will be quantified by integration of their peak areas. The degradation pattern can be determined by using LC-MS where MS spectra of the separated peak can be determined. Percentage intact/degraded compound at a given time is used for estimation of the peptides DPPIV stability.

A peptide is defined as DPPIV stabilised when it is 10 times more stable than the natural peptide based on percentage intact compound at a given time. Thus, a DPPIV stabilised GLP-1 compound is at least 10 times more stable than GLP-1 (7-37).

### Stimulation of adenylate cyclase

BRIN-BD11 cells will be seeded into 24-well plates (3x10⁵/well) and cultured for 48 h before being preincubated in media supplemented with tritiated adenine (2mCi) for 16 h. The cells will be washed twice with cold Hanks buffered saline (HBS) and test solution (400ul; 37C) added. The cells will then be exposed to varying concentrations (10-10-10-5 M) of GLP-1 compounds in HBS buffer, in the presence of 1mM IBMX and 5.6mM glucose (20 min; 37C). Following incubation, test solutions will be removed and 300ul of lysis solution (5% TFA, 3% SDS, 5mM of unlabelled ATP, and 300 µM of unlabelled cAMP) added. Dowex and alumina exchange resins will be used to separate tritiated cAMP from tritiated adenine and ATP in the cell lysate, as described (Miguel JC, et al. Biochem. Pharmacol. 2003, 65:283).

Insulin secretory responses can be measured in the pancreatic *β*-cell BRIN-BD11 cells. Cells will be seeded into 24-multiwell plates at a density of 1x10⁵/well, and allowed to attach during overnight culture. Acute studies of insulin release will be preceded by 40 min pre-incubation at 37C in 1.0 ml Krebs-Ringer bicarbonate buffer (115mM NaCl, 4.7mM KCI, 1.28mM CaCl₂·2H₂O, 1.2mM KH₂PO₄, 1.2mM MgSO₄·H₂O, 10mM NaHCO, and 5 g/L bovine serum albumin, pH 7.4) supplemented with 1.1mM glucose. Test incubations will be performed at 37C in the presence of 5.6mM glucose with a range of concentrations of GLP-1 compounds (10-12-10-6 M). After 20 min incubation, the buffer will be removed from each well and aliquots stored at -20C for measurement of insulin.

### Glucose-lowering and insulin secretory activity in obese diabetic (ob/ob) mice

The *in vivo* biological activity of GLP-1 compounds can be assessed in 12-16 week old obese diabetic (ob/ob) mice. The animals will be housed individually in an air-conditioned room at 22±2C with a 12 h light: 12 h dark cycle. Animals will be allowed drinking water ad libitum and continuous access to standard rodent maintenance diet. Mice will be fasted for 18 h and intraperitoneally administered 8 ml/kg body weight with saline (9 g/L NaCl), glucose alone (18mM/kg bodyweight), or in combination with a GLP-1 compound (25 nM/kg body weight). Blood samples will be collected into chilled fluoride/heparin microcentrifuge tubes immediately prior to injection and at 15, 30, and 60 min post injection, and the plasma obtained stored at -20C.

### Other Assays

Plasma glucose levels can be determined using an Analox glucose analyser (Hammersmith, London, UK), which employs the glucose oxidase method (Stevens JF, Clin. Chim. Acta 1971, 32:199). Insulin levels can be assayed by dextran-coated charcoal radioimmunoassay (Flatt PR and Bailey CJ, Diabetologia 1981,20:573). Incremental areas under plasma glucose and insulin curves (AUC) can be calculated using GraphPad PRISM version 3.0 (Graphpad Software, San Diego, CA, USA).

The activity of GLP-1 compound can be part of the drug composition of the present invention as long as the GLP-1 drug is able to bind and induce signaling through the GLP-1 receptor. GLP-1 receptor binding and signal transduction can be assessed using *in vitro* assays such as those described in Examples 2, 3, and 4 of EP 619,322 and Examples 1, 2, and 3 of U. S. Patent No. 5,120,712, respectively (incorporated herein by reference).

Pharmacokinetics studies can be performed as described in Example 7 of WO 02/46227 (incorporated herein by reference).

Half-life extension of GLP-1 derivatives after i. v. or s. c. administration.

The half-life extension of GLP-1 analogues can be determined by monitoring the concentration thereof in plasma after sc administration to healthy pigs. For comparison the concentration in plasma of GLP-1 (7-37) (natural active of form GLP-1 and used as a control) after sc. administration can be followed.

The test substances will be dissolved in a vehicle suitable for subcutaneous or intravenous administration. The concentration will be adjusted so the dosing volume is approximately 1 ml. The study will be performed in 12 male Göttingen minipigs from Ellegaard Göttingen Minipigs ApS. An acclimatisation period of approximately 10 days will be allowed before the animals entered the study. At start of the acclimatisation period the minipigs will be about 5 months old and in the weight range of 8-10 kg.

The study will be conducted in a suitable animal room with a room temperature set at 21-23°C and the relative humidity approximately 50%. The room will be illuminated to give a cycle of 12 hours light and 12 hours darkness. Light will be from 06.00 to 18.00h. The animals will be housed in pens with straw as bedding, six together in each pen. The animals will have free access to domestic quality drinking water during the study, but will be fasted from approximately 16.00h the day before dosing until approximately 12 hours after dosing. The animals will be weighed on arrival and on the days of dosing.

The animals will receive a single intravenous or subcutaneous injection. The subcutaneous injection will be given on the right side of the neck, approximately 5-7 cm from the ear and 7-9 cm from the middle of the neck. The injections will be given with a stopper on the needle, allowing 0.5 cm of the needle to be introduced. Each test substance will be given to three animals. Each animal received a dose of 2 nmol/kg body weight. Six animals will be dosed per week while the remaining six rested.

A full plasma concentration-time profile will be obtained from each animal. Blood samples will be collected according to the following schedule: After intravenous administration: Predose (0), 0.17 (10 minutes), 0.5, 1,2, 4,6, 8,12, 24,48, 72,96, and 120 hours after injection. After subcutaneous administration: Predose (0), 0.5, 1, 2, 4, 6, 8, 12, 24,48, 72,96, and 120 hours after injection.

At each sampling time, 2 ml of blood will be drawn from each animal. The blood samples will be taken from a jugular vein. The blood samples will be collected into test tubes containing a buffer for stabilisation in order to prevent enzymatic degradation of the GLP-1 analogues. Plasma will be immediately transferred to Micronic-tubes. Approximately 200 µl plasma will be transferred to each Micronic-tube. The plasma was stored at-20°C until assayed. The plasma samples will be assayed for the content of GLP-1 analogues using an immunoassay.

The plasma concentration-time profiles will be analysed by a non-compartmental pharmacokinetic analysis. The following pharmacokinetic parameters will be calculated at each occasion: AUC, AUC/Dose, AUC_{% Extrap}, Cₘₐₓ, tₘₐₓ, λ_{z}, t_{½}, CL, CL/f, V_{z}, V_{z}/f and MRT.

### Compostions of the invention can also be tested in Danish Landrace pigs.

Pigs (50% Duroc, 25% Yorkshire, 25% Danish Landrace, app 40 kg) will be fasted from the beginning of the experiment. To each pig 0.5 nmol of test composition per kg body weight will be administered in a 50 pM isotonic solution (5 mM phosphate, pH 7.4, 0.02% Tween®-20 (Merck), 45 mg/ml mannitol (pyrogen free, Novo Nordisk). Blood samples will be drawn from a catheter in venajugularis. 5 ml of the blood samples will be poured into chilled glasses containing 175 µl of the following solution: 0.18 M EDTA, 15000 KIE/ml aprotinin (Novo Nordisk) and 0.30 mM Valine-Pyrrolidide (Novo Nordisk), pH 7.4. Within 30 min, the samples will be centrifuged for 10 min at 5-6000*g. Temperature will be kept at 4°C. The supernatant will be pipetted into different glasses and kept at minus 20°C until use.

The plasma concentrations of the peptides will be determined in a sandwich ELISA or by RIA using different mono-or polyclonal antibodies. Choice of antibodies depends of the GLP-1 analogue. The time at which the peak concentration in plasma is achieved varies within wide limits, depending on the particular GLP-1 analogue selected.

### General assay protocol for sandwich ELISA in 96-wells microtitre plate

Coating buffer (PBS): Phosphate buffered saline, pH7.2
Wash-buffer (PBS-wash): Phosphate buffered saline, 0.05 % v/v Tween 20, pH 7.2
Assay-buffer (BSA-buffer): Phosphate buffered saline, 10 g/l Bovin Serum Albumin (Fluka 05477), 0.05 % v/v Tween 20, pH 7.2
Streptavidin-buffer: Phosphate buffered saline, 0.5 M NaCl, 0.05 % v/v Tween 20, pH 7. 2
Standard: Individual compounds in a plasma-matrix
A-TNP: Nonsense antibody
AMDEX: Streptavin-horseradish-peroxodase (Amersham RPN4401V)
TMB-substrate: 3, 3', 5, 5'tetramethylbenzidine (<0.02 %), hydrogen peroxide

The assay can be carried out as follows (volume/well):
1.) Coat with 100 µl catching antibody 5 µg/ml in PBS-buffer, incubate o/n, 4 °C, 5x PBS-wash, blocked with last wash in minimum 30 minute, then empty the plate
2.) 20 µl sample + 100 µl biotinylated detecting antibody 1 µg/ml in BSA-buffer with 10 ug/ml A-TNP; incubate 2 h, room temperature, on a shaker; 5x PBS-wash, then empty the plate.
3.) 100 µl AMDEX 1: 8000 in Streptavidin-buffer, incubate 45-60 minute, room temperature, on a shaker; 5x PBS-wash, then empty the plate.
4.) 100 µlTMB-substrate, incubate at room temperature on a shaker; stop the reaction with 100 µl 4 M H₃PO₄. Read the absorbance at 450 nm with 620 nm as reference. The concentration in the samples can be calculated from standard curves.

General assay protocol for RIA.

DB-buffer: 80 mM.phosphate buffer, 0.1 % Human serum albumin, 10 mM EDTA, 0.6 mM thiomersal, pH 7.5.

FAM-buffer: 40 mM phosphate buffer, 0.1 % Human Serum Albumin, 0.6 mM thiomersal, pH 7.5.

Charcoal : 40 mM phosphate buffer, 0.6 mM thiomersal, 16.7 % bovine plasma, 15 g/l activated carbon, pH 7.5 (mix the suspension minimum 1 h before use at 4 °C) Standard: Individual compounds in a plasma-matrix.

The assay will be carried out in minisorp tubes 12x75 mm (volumen/tube) as follows:

| | DB-buffer | Sample | Antibody | FAM | Tracer | Charcoal | H₂O |
|---|---|---|---|---|---|---|---|
| Dayl | | | | | | | |
| Total | | | | | 100 µl | | |
| NSB | 330 µl | | | 100 µl | | | |
| Sample | 300 µl | 30 µl | 100 µl | | 100 µl | | |

| Mix, incubate o/n at 4°C Day 2 | | | | | | | |
|---|---|---|---|---|---|---|---|
| Total | | | | | | | 1.5 ml |
| NSB | | | | | | 1.5 ml | |
| Sample | | | | | | 1.5 ml | |

Mix and incubate 30 min at 4 °C. Centrifuge at 3000 rpm for 30 min and immediately after, transfer supernatants to new tubes, close with stopper and count on gamma-counter for 1 minute. The concentration in the samples will be calculated from individual standard curves.

### GLP-1 radio receptor assay (RRA):

The GLP-1 radio receptor assay is a radiometric-ligand binding assay using LEAD*seeker* imaging particles. The assay is composed of membrane fragments containing the GLP-1 receptor, unlabeled GLP-1 analogues, human GLP-1 labelled with ¹²⁵I and PS LEAD*seeker* particles coated with wheat germ agglutinin (WGA). Cold and ¹²⁵I-labelled GLP-1 will compete for the binding to the receptor. When the LEAD*seeker* particles are added they will bind to carbohydrates residues on the membrane fragments via the WGA-residues. The proximity between the ¹²⁵I-molecules and the LEADseeker particles causes light emission from the particles. The LEAD*seeker* will image the emitted light and it will be reversibly correlated to the amount of GLP-1 analogue present in the sample.

### Reagents & Materials:

Pre treatment of animal plasma: Animal plasma will be heat treated for 4 hrs at 56°C and centrifuged at 10,000 rpm for 10 minutes. Afterwards, Val-Pyr (10 µM) and aprotenin (500 KIE/ml) will be added and stored at -18°C until use.

GLP-1 analogues standards: GLP-1 analogues will be spiked into heat-treated plasma to produce dilution lines ranging from approximately 1 µM to 1 pM.

GLP-1 RRA assay buffer: 25 mM Na-HEPES (pH 7.5), 2.5 mM CaCl₂, 1 mM MgCl₂, 50 mM NaCl, 0.1% ovalbumin, 0.003% Tween 20, 0.005% bacitracin, 0.05% NaN₃.

GLP-1 receptor suspension: GLP-1 receptor membrane fragments will be purified from baby hamster kidney (BHK) cells expressing the human pancreatic GLP-1 receptor. Stored at- 80°C until use.

WGA-coupled polystyrene *LEADseeker* imaging beads (RPNQ0260, Amersham): The beads will be reconstituted with GLP-1 RRA assay buffer to a concentration of 13.3 mg/ml. The GLP-1 receptor membrane suspension will then be added and incubated cold (2-8°C) for at least 1 hr prior to use.

### Materials

¹²⁵I-GLP-1 (7-36) amide (Novo Nordisk A/S). Stored at -18°C until use.

Ethanol 99.9% vol (De Dansk Sprotfabrikker A/S). Stored at -18°C until use.

MultiScreen® Solvinert 0.45µm hydrophobic PTFE plate (MSRPN0450, Millipore Corp.).

Polypropylene 384-well plates (cat. No. 781075, Greiner Bio-One).

### Appartus:

Horizontal plate mixture
Centrifuge with a standard swinging bucket microtitre plate rotor assembly.
UltrVap, Drydown Sample concentrator (Provair)
LEAD*seeker*® Multimodality Imaging System (Amersham)

### Procedure:

Sample Preparation: Mount the MultiScreen® Solvinert filter plate on a chemical-comparable receiver plate (ie polypropylene plates) to collect the filtrate.

Add 150 µl ice-cold ethanol 99.9% into the empty wells of the MultiScreen Solvinertfilter plate followed by 50 µl calibrator or plasma sample. Place the storage lid on the filter plate and incubate 15 minutes at 18-22°C on a horizontal plate mixer.

The assembled filter and receiver plate, with the lid, will be placed into a standard swinging-bucket microtitre plate rotor assembly. The filtrate will be collected in the empty wells of the receiver plate at 1500 rpm for 2 minutes. The filtrate will be dried down using the UltraVap with heated N₂ (40°C) for 15 miuntes. The dry material will be reconstituted by adding 100 µl GLP-1 RRA assay buffer into each well. This will be incubated for 5 minutes on a horizontal mixer.

GLP-1 radio receptor assay: Use the following pipetting scheme and white polystyrene 384-well plates:
1. 35µl GLP-1 RRA assay buffer
2. 5µl reconstituted filtrate
3. 10µl ¹²⁵I-GLP-1(7-36)amide. The stock solution was diluted in GLP-1 RRA assay buffer to 20,000 cpm/well prior to use.
4. 15 µL GLP-1 receptor membrane fragments (0. 5 µg/well) pre-coated to

WGA- polystyrene LEADseeker imaging beads (0.2 mg/well).

The plates will be sealed and incubated over night at 18-22°C. The light emission from each well will be detected by using the LEADseeker Multimodality Imaging System for duration of 10 minutes.

Stimulation of cAMP formation in a cell line expressing the cloned human GLP-1 receptor.

Purified plasma membranes from a stable transfected cell line, BHK467-12A (tk-ts13), expressing the human GLP-1 receptor will be stimulated with GLP-1 and peptide analogues, and the potency of cAMP production will be measured using the AlphaScreen™ cAMP Assay Kit from Perkin Elmer Life Sciences.

A stable transfected cell line will be prepared and a high expressing clone will be selected for screening. The cells will be grown at 5% CO₂ in DMEM, 5% FCS, 1% Pen/Strep and 0. 5 mg/ml G418.

Cells at approximate 80% confluence will be washed 2X with PBS and harvested with Versene, centrifuged 5 min at 1000 rpm and the supernatant removed. The additional steps will be all made on ice. The cell pellet will be homogenized by the Ultrathurax for 20-30 sec. in 10 ml of Buffer 1 (20 mM Na-HEPES, 10 mM EDTA, pH 7.4), centrifuged 15 min at 20,000 rpm and the pellet resuspended in 10 ml of Buffer 2 (20 mM Na-HEPES, 0.1 mM EDTA, pH 7.4). The suspension will be homogenized for 20-30 sec and centrifuged 15 min at 20.000 rpm. Suspension in Buffer 2, homogenization and centrifugation will be repeated once and the membranes will be resuspended in Buffer 2 and ready for further analysis or stored at -80°C. The functional receptor assay will be carried out by measuring the peptide induced cAMP production by The AlphaScreen Technology. The basic principle of The AlphaScreen Technology is a competition between endogenous cAMP and exogenously added biotin-cAMP. The capture of cAMP will be achieved by using a specific antibody conjugated to acceptor beads. Formed cAMP will be counted and measured on an AlphaFusion Microplate Analyzer. The EC₅₀ values will be calculated using the Graph-Pad Prisme software.

### Example 11. Bacterial expression of a genetic fusion of glucagon like peptide-1 and iDom7h-8 using the GAS leader.

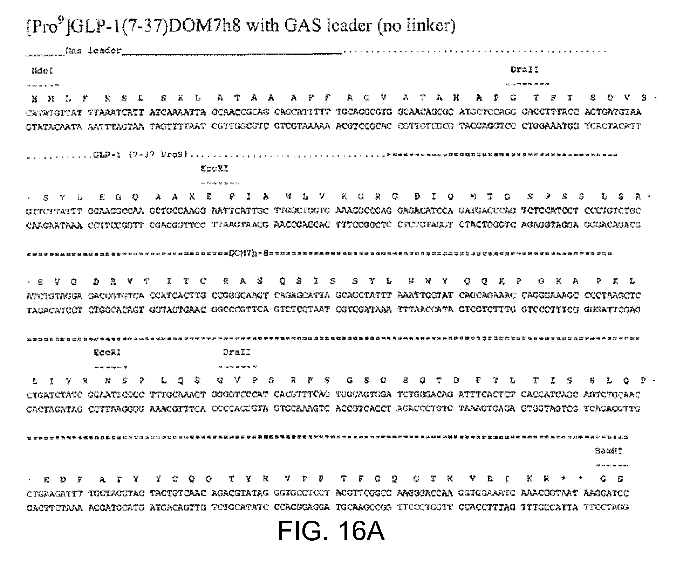

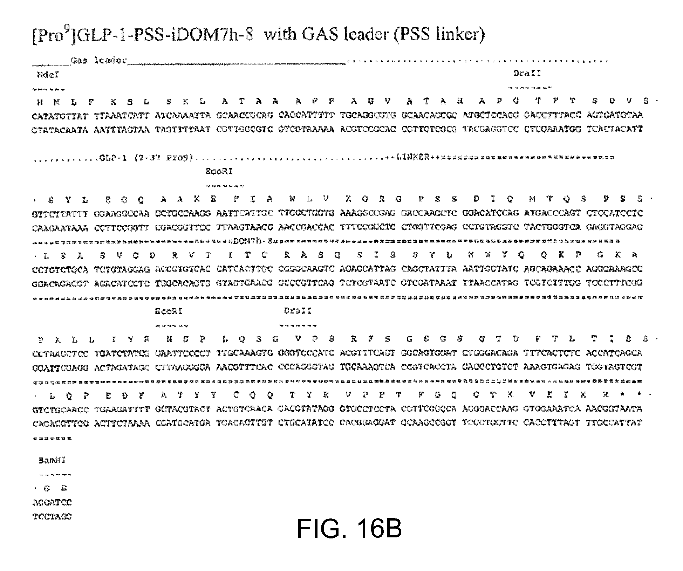

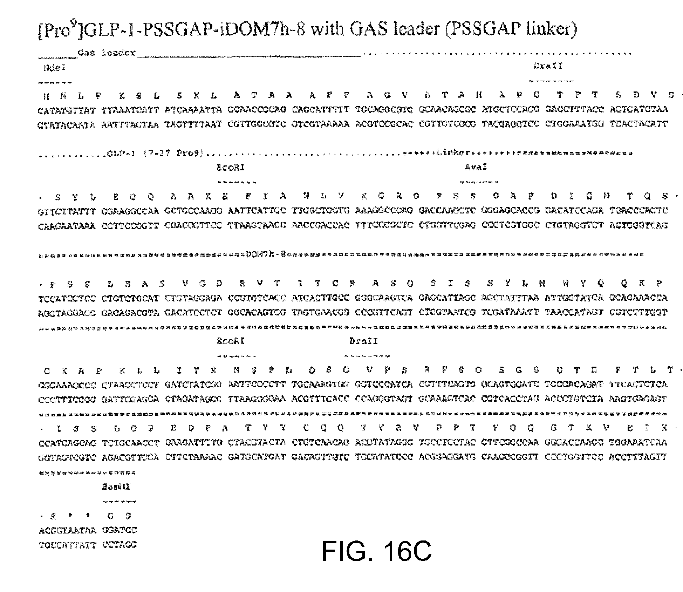

GLP-1 (7-37), with glutamate at position 9 replaced by proline ([Pro⁹] GLP-1(7-37)), was cloned as a fusion with iDOM7h-8 (a P96E mutation by Kabat numbering in CDR3) into the pET 12a vector with a GAS leader (see WO 05/093074). The GLP-1 glutamate to proline 9 replacement was in order to render the GLP-1 part of the fusion resistant to degradation by dipeptidyl peptidase IV (DPPIV) cleavage (Brian D. Green et al. (2003) Metabolic Stability, Receptor Binding, cAMP Generation, Insulin secretion and Antihyperglycaemic Activity of Novel N-terminal Glu9-substituted Analogues of Glucagon-like-peptide-1: Biol. Chem. (384) 1543-1555). In total, three constructs were made, one with no linker, one with PSS amino acids between [Pro⁹]GLP-1(7-37) and iDOM7h-8 and one with PSSGAP amino acids between [Pro⁹]GLP-1(7-37) and iDOM7h-8 (shown in Figure 16 as Dom7h-8 the albumin binding form). Expression was in BL21 DE3 Plys S cells at 30°C for 48 hours using overnight expression autoinduction TB readymix (Novagen) before recovery of the supernatant by centrifugation. [Pro⁹] GLP-1(7-37) iDom7h-8 fusion was purified from the supernatant using affinity capture on protein L-agarose. The resin was then washed with 10 column volumes of PBS and bound protein eluted with 0.1 M glycine pH2. [Pro⁹]GLP-1(7-37)-iDom7h-8 fusion was then loaded in the glycine buffer, onto a cation exchange column (1 ml S-column, GE healthcare) that was pre-equilibrated with 20mM citrate buffer at pH 6.2. Elution was with a 0 - 50% gradient of the same buffer supplemented with 1M NaCl. Peaks were collected and the size of the proteins determined by SDS PAGE electrophoresis. Peaks with protein of the expected size were pooled and buffer exchanged to PBS. Identity of the protein was confirmed by mass spectrometry and N-terminal sequencing.

### Example 12. GLP-1 activity determination of [Pro⁹]GLP-1(7-37)-PSSGAP-iDOM7h-8 fusion

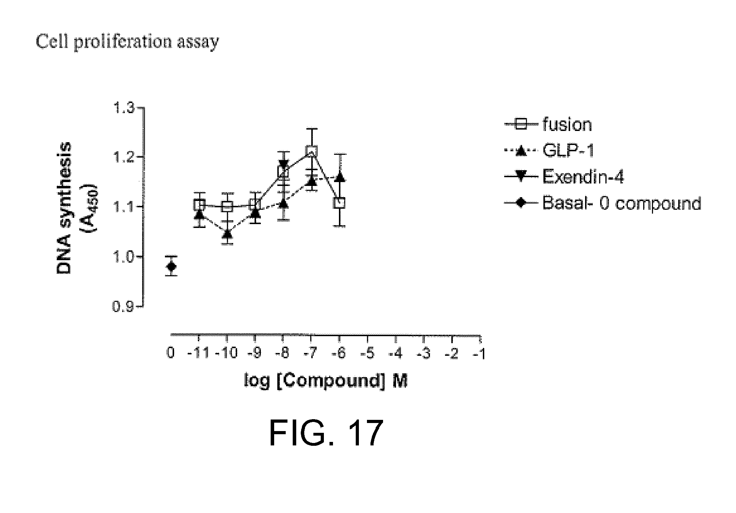

In order to confirm that the [Pro⁹]GLP-1(7-37)-PSSGAP-iDOM7h-8 fusion demonstrated GLP-1 activity, the fusion was subjected to two different biological assays. In the first assay, the RINm5f rat insulinoma cell line (developed in 1980 by *Gadzar et al* from x-ray induced transplantable insulinoma of the rat) was incubated with varying concentrations (10pM to 0.1µM) of GLP-1 and the [Pro⁹]GLP-1(7-37)-PSSGAP-iDOM7h-8 fusion for 60 min. Additionally, a single point assay of Exendin-4, a GLP-1 analogue resistant to degradation by dipeptidyl peptidase IV, and a single point buffer only assay were added as controls. Although the RINm5f rat cells respond poorly to glucose, when exposed to nutrients or non-secretagogues, they display secretory responses similar to beta cells. Therefore, the effects of the compounds on cell proliferation were assessed by measuring the incorporated levels of 5-bromo-2'-deoxyuridine (BrdU) during DNA synthesis in proliferating cells using the Cell proliferation ELISA system (Amersham, Little Chalfont, UK) see Figure 17. Using OD450 to measure DNA levels, there was a dose dependent increase in DNA level with increasing levels of [Pro⁹]GLP-1(7-37)-PSSGAP-iDOM7h-8 fusion up to a concentration of 100nM of the fusion. GLP-1 also showed the expected dose dependent response.

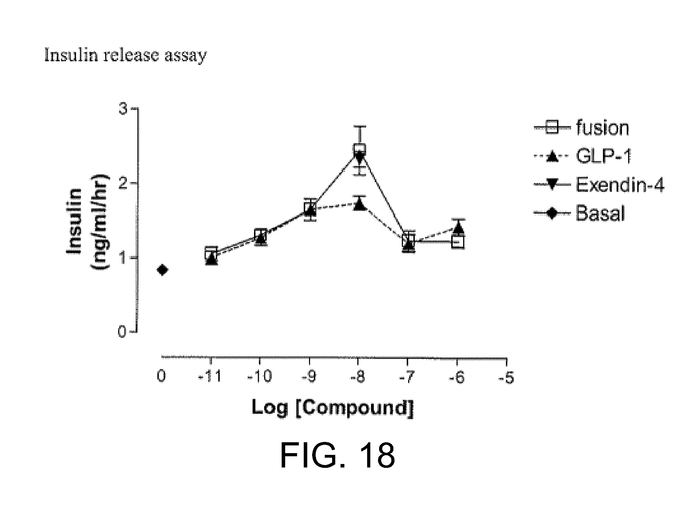

In the second assay, RINm5f cells were incubated with varying concentrations (10pM to 0.1µM) of GLP-1 and the [Pro⁹]GLP-1(7-37)-PSSGAP-iDOM7h-8 fusion in 5.6mM glucose for 60 min. Additionally, a single point assay of Exendin-4, a GLP-1 analogue resistant to degradation by DPPIV and a single point Krebs-Ringer bicarbonate buffer (KRB) only assay were added as controls. Insulin secretion was assayed after incubation for 60 min at 37°C using KRB buffer supplemented GLP-1, 3A23 or exendin-4. The medium was collected, centrifuged and the supernatant assayed for insulin concentration using radioimmunoassay. Insulin concentration was normalised to cell number within each well. Insulin concentration (measured in ng/ml/hr) was then plotted against compound concentration. There was a dose dependent increase in insulin release at escalating doses of [Pro⁹]GLP-1(7-37)-PSSGAP-iDOM7h-8 fusion up to a fusion concentration of 10nM (see Figure 18). This agrees well with published data on GLP-1 alone.

### Example 13. Bacterial expression of a genetic fusion of glucagon like peptide-1 and iDom7h-8 using the OMP-T leader.

The same 3 constructs described in Example 11 (one with no linker, one with PSS amino acids between [Pro⁹]GLP-1(7-37) and iDOM7h-8 and one with PSSGAP amino acids between [Pro⁹]GLP-1(7-37) and iDOM7h-8) were remade with the OMP-T leader. For clarity, the order of the elements in the construct were OMP-T leader, [Pro⁹]GLP-1, Linker (where appropriate) and the iDom7h-8. Expression was in BL21 DE3 Plys S cells at 25°C for 4 hours in TB media induced with 0.5mM IPTG at OD 16 before recovery of the cell pellet by centrifugation. Secreted proteins were then recovered by periplasmic preparation. GLP-1 iDom7h-8 fusions were purified from the periplasmic fraction using affinity capture on protein L-agarose. The resin was then washed with 10 column volumes of PBS and bound protein eluted with 0.1 M glycine pH2. [Pro⁹]GLP-1(7-37) fusion was then loaded in the glycine buffer, onto a cation exchange column (1 ml S-column, GE healthcare) that was pre-equilibrated with 20mM citrate buffer at pH 6.2. Elution was with a 0 - 50% gradient of the same buffer supplemented with 1M NaCl. In this case, washing the column with 20mM citrate buffer at pH 6.2 (0% NaCl) led to flow through of the band of the expected size and so this was concentrated using a 5K vivaspin column (Vivascience).

### Example 14. Pichia pastoris expression of a genetic fusion of glucagon like peptide-1 and iDom7h-8.

The [Pro⁹]GLP-1-PSS-iDOM7h-8 fusion construct (as described in Figure 16b but using iDom7h-8) will be cloned into the pPICzα vector both alone and with an N-terminal EAEA extension and transformed into Pichia pastoris KM71h. Protein will be expressed (i) using methanol induction over 4 days at 30°C and (ii) using methanol induction over 2 days at 25°C. Supernatant will be recovered by centrifugation and protein checked for size on an SDS PAGE gel.

It is expected that the fusions will have the correct size by SDS Page and be active in the GLP-1 assay as described in Example 10 and in Example 12.

### Example 15. E.coli expression of glucagon like peptide-1 and iDom7h-8 in BL21 DE3 inclusion bodies.

The same fusions as described in Example 11 will be cloned into the pET21 expression vector (Novagen), which is designed for protein expression in the cytoplasm. Optionally, a protease cleavage site will be included in the constructs between a sacrificial N-terminus and the HAP... of the [Pro⁹]GLP-1(7-37). This will enable the protein to be digested to ensure a fully native N-terminus. Enzymes that could be used for this include Factor Xa, thrombin or DPPI. Protein will then be expressed at high levels in BL21(DE3) cells upon IPTG induction and will accumulate in intracellular inclusion bodies. Inclusion bodies will be isolated from the BL21 cells and solublised in guanidine HCl. Following reduction, inclusion bodies will be refolded in a redox shuffling buffer system (Buchner, J., Pastan, I. and Brinkmann, U. (1992). A method for increasing the yield of properly folded recombinant fusion proteins. Anal. Biochem. 205, 263-270. After refolding, the protein will be dialysed and concentrated in a 5K vivaspin column (Vivascience) and purified by S-column (GE healthcare).

It is expected that the fusions will have the correct size by SDS Page and be active in the GLP-1 assay as described in Example 10 and in Example 12.

### Example 16. Mammalian expression of glucagon like peptide-1 and a Dom7h-8

The [Pro⁹]GLP-1-PSS-DOM7h-8 fusion construct (as described in Figure 16b) will be cloned into the PcDNA(-) vector using a murine secretory signal peptide to promote secretion of the translated protein into the media. 1mg of DNA will be prepared in *E.coli* using alkaline lysis (mega prep kit, qiagen, CA) and transfected into 1.5L of HEK293 cells grown in Dulbecco's modified Eagle's medium (Gibco) for transient protein expression. Protein will be expressed by incubating the culture at 37° for 5 days and supernatant (containing expressed protein) will be recovered by centrifugation. [Pro⁹]GLP-1-PSS-DOM7h-8 fusion will be purified from the periplasmic fraction using affinity capture on protein L-agarose. The resin will then be washed with 10 column volumes of PBS and bound protein eluted with 0.1 M glycine pH2. Protein will then be loaded in the glycine buffer, onto a cation exchange column (1 ml S-column, GE healthcare) that is pre-equilibrated with 20mM citrate buffer at pH 6.2. Elution will be with a 0 - 50% gradient of the same buffer supplemented with 1M NaCl. Protein of the correct size on an SDS-PAGE gel will then be concentrated using a 5K vivaspin column (Vivascience) and buffer exchanged into PBS for biological assay.

### Example 17. E. coli expression of Peptide YY fused to a Dom7h-8.

Peptide YY (3-36) (PYY: amino acid sequence ID No. 167 and nucleci acid sequence ID No. 168 IKPEAPGEDASPEELNRYYASLRHYLNLVTRQRY) inhibits food intake in humans and has a short half life in plasma (10-30min). It is released in response to a meal and acts via the Y2R in the arcuate nucleus to physiologically regulate food intake. PYY will be cloned into the pET GAS vector (WO05093074) abutting the DOM7h-8 (see Figures 20a and 20b which show the peptide C-terminal and N-terminal of the DOM7h-8 respectively.) Expression will be in BL21 DE3 Plys S cells at 25°C for 4 hours in TB media induced with 0.5mM IPTG at before recovery of the cell pellet by centrifugation. Secreted proteins will then be recovered by periplasmic preparation. PYY Dom7h-8 fusion will be purified from the periplasmic fraction using affinity capture on protein L-agarose. The resin will then be washed with 10 column volumes of PBS and bound protein eluted with 0.1 M glycine pH2 and purified further by ion exchange. Purified protein will then be buffer exchanged to PBS by dialysis, concentrated in a 5K vivaspin column (Vivascience) and subjected to biological assay to measure stimulation of cAMP release as described (Goumain et al. (2001) The Peptide YY-Preferring Receptor Mediating Inhibition of Small Intestinal Secretion Is a Peripheral Y2 Receptor: Pharmacological Evidence and Molecular Cloning: Molecular pharmacology: 60 124-134). Briefly, Isolated intestinal crypt cells at 200µg protein/ml will be incubated under continuous agitation for 45min at 15°C in 0.5ml of phosphate-buffered saline, pH 7.0, containing 1.4% (w/v) bovine serum albumin, 0.1% bacitracin, and 0.2mM 3-isobutyl-1-methylxanthine (IBMX) as described (Servin et al. (1989): Peptide-YY and neuropeptide-Y inhibit vasoactive intestinal peptide-stimulated adenosine 3',5'-monophosphate production in rat small intestine: structural requirements of peptides for interacting with PYY-preferring receptors. Endocrinology 124: 692-700). PYY alone or PYY-Dom7h-8 fusion will be added together with a potent physiological stimulant of cAMP production in enterocytes (e.g., VIP). The reaction will be initiated by adding cells and stopped after 45min by adding 50µl of 11M perchloric acid. After centrifugation for 10min at 4,000g, the cAMP present in the supernatant will be succinylated, and its concentration will be measured by radioimmunoassay as described (Laburthe et al., (1982) Alpha-adrenergic inhibition of cyclic AMP accumulation in epithelial cells isolated from rat small intestine. Biochim Biophys Acta 721: 101-108).

It is expected that the fusion be of the expected size and will show PYY activity equivalent to the non-fusion controls.

### Example 18. E. coli expression of a Dom7h-8 Peptide YY, GLP-1, fusion

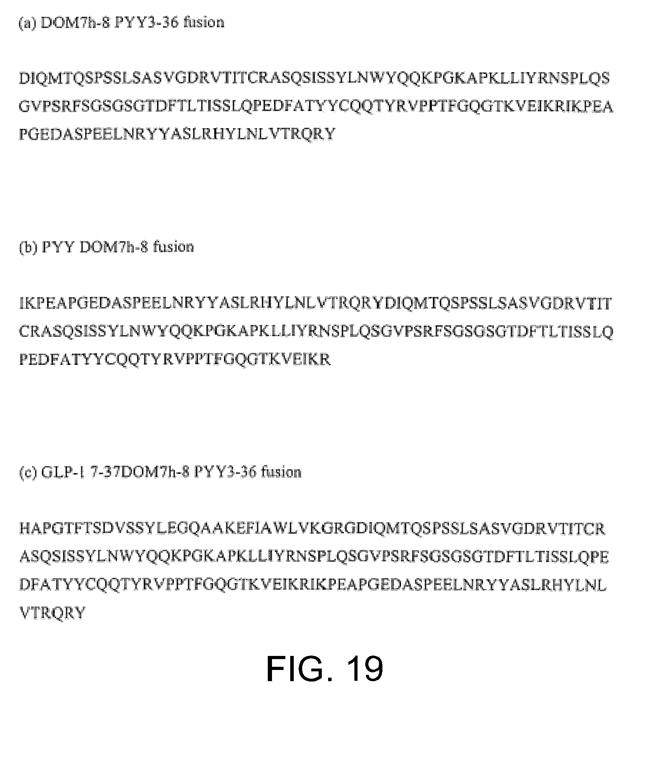

A [Pro⁹]GLP-1(7-37)-DOM7h-8-PYY (see Figure 19c) fusion will be cloned into the pET GAS vector and then expressed as described for the Dom7h-8 PYY described in Example 17. After purification, the fusion will be assayed for the biological activity of both PYY and GLP-1 following the assays described in Examples 17 and Example 12 respectively.

It is expected that the fusions will be of the expected size will show PYY and GLP-1 activity equivalent to the non-fusion controls.

While this invention has been particularly shown and described with references to preferred embodiments thereof, it will be understood by those skilled in the art that various changes in form and details may be made therein without departing from the scope of the invention encompassed by the appended claims.

SEQUENCE LISTING

GLP-1 (7-37)

Amino acid:

HAE GTFTSDVSSY LEGQAAKEFI AWLVKGRG - SEQ ID NO:157

Nucleotide sequence:

ca tgctgaaggg acctttacca gtgatgtaag ttcttatttg gaaggccaag ctgccaagga attcattgct
tggctggtga aaggccgagg a   SEQ ID NO:158

GLP-1 (7-36)

HAE GTFTSDVSSY LEGQAAKEFI AWLVKGR SEQ ID NO:159

ca tgctgaaggg acctttacca gtgatgtaag ttcttatttg gaaggccaag ctgccaagga attcattgct
tggctggtga aaggccga   - SEQ ID NO:160

Exendin-4

HGE GTFTSDLSKQ MEEEAVRLFI EWLKNGGPSS GAPPPS  - SEQ ID NO:161

catggt gaaggaacat ttaccagtga cttgtcaaaa cagatggaag aggaggcagt gcggttattt attgagtggc
ttaagaacgg aggaccaagt agcggggcac ctccgccatc g      - SEQ ID NO:162

Oxyntomodulin

HSQGTFTS DYSKYLDSRR AQDFVQWLMN TKRNRNNIA  - SEQ ID NO:163

cattcac agggcacatt caccagtgac tacagcaagt atctggactc caggcgtgcc caagattttg tgcagtggtt
gatgaatacc aagaggaaca ggaataacat tgcc  - SEQ ID NO:164

Exendin-3

HSDGTFTSDL SKQMEEEAVR LFIEWLKNGG PSSGAPPPS  - SEQ ID NO:165

catagtga tggaacattt accagtgact tgtcaaaaca gatggaagag gaggcagtgc ggttatttat tgagtggctt
aagaacggag gaccaagtag cggggcacct ccgccatcg   - SEQ ID NO:166

PYY (3-36)

IKPEAPGEDA SPEELNRYYA SLRHYLNLVT RQRY   - SEQ ID NO:167

Atcaaacccg aggctcccgg cgaagacgcc tcgccggagg agctgaaccg ctactacgcc tccctgcgcc
actacctcaa cctggtcacc cggcagcggt at - SEQ ID NO:168

Gastric inhibitory peptide

YAEGTFISD YSIAMDKIHQ QDFVNWLLAQ KGKKNDWKHN ITQ - SEQ ID
NO:169

tacgcggaa gggactttca tcagtgacta cagtattgcc atggacaaga ttcaccaaca agactttgtg aactggctgc
tggcccaaaa ggggaagaag aatgactgga aacacaacat cacccag - SEQ ID NO:170

## Claims

1. A drug conjugate or fusion comprising an insulinotropic agent (IA) or a PYY peptide and an antibody fragment that binds an antigen, wherein the antigen acts to increase the half-life of the drug conjugate or fusion *in vivo.*

2. The drug conjugate or fusion of claim 1, wherein the drug is a drug fusion protein comprising the insulinotropic agent peptide bonded to the antibody fragment.

3. The drug of claim 2, wherein the insulinotropic agent is fused to the antibody fragment via peptide linker moiety.

4. The drug of any one of claims 1-3, wherein the antigen is serum albumin.

5. The drug of any one of any one of the preceding claims, wherein the antibody fragment is an immunoglobulin single variable domain selected from a V_{H}, V_{L} or a V_{HH} domain

6. The drug of any one of any one of the preceding claims, wherein-the insulinotropic agent is selected from the group consisting of GLP-1, GLP-1 7-36, GLP-1 analogue, Exendin-3, an Exendin-3 analogue, Exendin-4 and an Exendin-4 analogue.

7. The drug of any one of any one of the preceding claims, comprising 2, 3 or 4 insulinotropic agent (IA) moieties.

8. The drug of claim 7, comprising IA-IA'-AF or IA-(AF)ₙ-IA', wherein IA and IA' are the same or different insulinotropic agents.

9. The drug of any one of the preceding claims, comprising an anti-satiety agent.

10. The drug of claim 9, wherein the anti-satiety agent is selected from the group consisting of PYY, a PYY analogue, PYY (3-36).

11. The drug of claim 9 or 10, comprising IA-(AF)ₙ-(IA')ₓ -[anti-satiety agent] or [anti-satiety agent]- (IA')ₓ-(AF)ₙ-IA, wherein n equals 1, 2, 3, 4, or 5. and x equals zero, 1, 2, 3, 4, or 5.

12. A drug fusion having the formula:
a-(X)ₙ₁-b-(Y)ₙ₂-c-(Z)ₙ₃-d or a-(Z)ₙ₃-b-(Y)ₙ₂-c-(X)ₙ₁-d,
wherein
X is an insulintropic agent or an analogue thereof;
Y is an immunoglobulin heavy chain variable domain (V_{H}) that has binding specificity for serum albumin, or an immunoglobulin light chain variable domain (V_{L}) that has binding specificity for serum albumin;
Z is a polypeptide drug that has binding specificity for a target;
a, b, c and d are independently a polypeptide comprising one to about 100 amino acid residues or absent;
n1 is one to about 10;
n2 is one to about 10; and
n3 is zero to about 10.

13. The drug of claim 1 or 12, further comprising an agent selected from the group consisting of insulin, Exendin-4, Exendin-3, PYY (3-36), Resistin, Leptin, Melanocortin 3 receptor/ Melanocortin 4 receptor (MC3R/MC4R) antagonist, Agouti related peptide (AgRP) antagonist, Apolipoprotein A-IV, Enterostatin, Gastrin-Releasing Peptide (GRP), Insulin-like growth factor -1 (IGF1), Bone morphogenetic protein 9 (BMP-9), IL-22, RegIV, interferon alfa, INGAP peptide, somatostatin, amylin, neurulin, interferon beta, interferon hybrids, adiponectin, endocannabinoids, C peptide, WNT10b, Orexin-A, adrenocorticotrophin, Enterostatin, Cholecystokinin, oxyntomodulin, Melanocyte Stimulating Hormones, melanocortin, Melanin concentrating hormone, BB-2, Neuropeptide y (NPY) Y2 agonists, NPY Y5/Y1 antagonists, OXM, galanin 1R (Gal-1R) antagonists, melanin concentrating hormone 1R (MCH-1R) antagonists, MC-3/4 agonists, bombesin like receptor 3 (BRS-3) agonists, pancreatic polypeptide, anti-Ghrelin antibody fragment, brain-derived neurotrophic factor, human growth hormone, parathyroid hormone, follicle stimulating hormone, Gastric inhibitory peptide or an analogue thereof.

14. A drug according to any one of the preceding claims, for use in treating and/or preventing a condition in a patient, , wherein the condition is selected from the group consisting of hyperglycemia, type 2 diabetes, impaired glucose tolerance, type 1 diabetes, obesity, hypertension, syndrome X, dyslipidemia, cognitive disorders, atheroschlerosis, myocardial infarction, coronary heart disease and other cardiovascular disorders, stroke, inflammatory bowel syndrome, dyspepsia and gastric ulcers.

15. A drug according to any_one of the preceding claims, for use in decreasing food intake by a patient, decreasing β-cell apoptosis, increasing β-cell function and β-cell mass and/or restoring glucose sensitivity of β-cells in a patient.
